# EUROPEAN PATENT SPECIFICATION

(11) **EP 3 128 975 B1**
(45) Date of publication and mention of the grant of the patent: **14.11.2018**
(21) Application number: 15714076.5
(22) Date of filing: 13.03.2015
(51) Int. Cl.: A61F 13/495, A61F 13/511, A61F 13/512, A61F 13/513

(54) **ABSORBENT ARTICLES HAVING SUBSTRATES HAVING ZONAL TREATMENTS**
ABSORBIERENDE ARTIKEL MIT SUBSTRATEN MIT ZONALEN BEHANDLUNGEN
ARTICLES ABSORBANTS COMPRENANT DES SUBSTRATS À TRAITEMENTS PAR ZONE

(30) Priority: 08.04.2014 US 201414247598
(43) Date of publication of application: 15.02.2017
(73) Proprietor: The Procter & Gamble Company, Cincinnati, OH 45202 (US)
(72) Inventor: ARIZTI, Blanca, 65824 Schwalbach am Taunus (DE); ROE, Donald, Carroll, Cincinnati, Ohio 45202 (US); GREENING, Nelson, Edward, II, Cincinnati, Ohio 45202 (US); FEIST, Barry, Robert, Cincinnati, Ohio 45202 (US); BICKING, Amanda, Margaret, Cincinnati, Ohio 45202 (US); O'REILLY, Marie, Brigid, Cincinnati, Ohio 45202 (US); WALTHER, Rachael, Eden, Cincinnati, Ohio 45202 (US)
(74) Representative: L'Huillier, Florent Charles
(86) International application number: PCT/US2015/020495
(87) International publication number: WO 2015/156953

(56) References cited:
- EP-A1- 0 674 499
- EP-A1- 1 124 522
- EP-A1- 2 656 826
- EP-A2- 0 585 904
- WO-A1-99/25287
- WO-A1-2006/071143
- WO-A1-2010/017362
- WO-A1-2013/051971

## Description

### FIELD

The present disclosure is generally directed to absorbent articles for personal hygiene. The absorbent articles may each comprises one or more substrates comprising zones having the same or different treatments.

### BACKGROUND

Absorbent articles for personal hygiene are designed to absorb and contain bodily exudates (e.g., urine, bowel movements "BM"). These absorbent articles may comprise several layers providing different functions, for example, a topsheet, a backsheet, and an absorbent core disposed between the topsheet and the backsheet, among other layers.

The function of the absorbent core is to absorb and retain the bodily exudates for a prolonged amount of time, for example, overnight for a diaper, minimize re-wet to keep the wearer dry, and avoid soiling of clothes or bed sheets. Some currently marketed absorbent articles comprise absorbent cores comprising an absorbent material which is a blend of comminuted wood pulp (i.e., airfelt) with superabsorbent polymers ("SAP") in particulate form, also known as absorbent gelling materials ("AGM"). Other absorbent articles have an absorbent core consisting essentially of SAP as the absorbent material and one or more hotmelt adhesives (so called "airfelt-free" cores).

Absorbent articles may also comprise a liquid management system ("LMS") that may have an acquisition layer and/or a distribution layer. Some absorbent articles may comprise leg cuffs and waist bands which provide improved containment of liquids and other bodily exudates. Usually, each leg cuff comprises one or more elastic strands or elements comprised in the chassis of the diaper, for example, between the topsheet and backsheet in the area of the leg openings to provide an effective seal while the absorbent article is in use. These elasticized elements which may be substantially planar with the chassis of the absorbent article will be referred to herein as gasketing cuffs. It is also usual for the leg cuffs to comprise raised elasticized flaps, herein referred to as barrier leg cuffs, which improve the containment of fluid in the leg-torso joint regions.

Absorbent articles, such as diapers (taped or pants) or adult incontinence products, appear to have a shortcoming in their topsheets and/or their LMS in that the front and back regions, or other regions, are not specifically designed for their intended use in either function or appearance. Most commercial diapers or adult incontinence products include topsheets and/or acquisition layers that have zero features (regular nonwoven material) or one feature, such as apertures or embossments, for example, throughout the entire topsheet or LMS. The embossments or apertures are typically the same size and shape (same appearance as well) in the front and the back regions and, therefore, do not provide specific configurations for urine management compared to BM management or do not provide specific configurations that give the appearance of urine management compared to the appearance of BM management. This can be problematic in that urine management should be treated much differently than BM management to achieve an improved diaper owing to the fact that BM and urine have significant differences in rheology and solids content. It is not a one-size-fits-all situation. In view of the foregoing, topsheets and/or LMSs of absorbent articles should be improved to provide more zonal treatments or features that handle, or give the appearance of handling, urine management and BM management differently.

In addition to the above, typical absorbent articles need improved systems of handling bodily exudates once the bodily exudates are received by the topsheet. In general, some absorbent articles acquire bodily exudates too quickly or too slowly and other absorbent articles may leak because of the bodily exudates not be absorbed, or fully or properly absorbed, into the core. As such, it may be beneficial for topsheets and/or LMSs to have features that direct or alter (e.g., slow, hasten, restrict, channel) the flow of bodily exudates into, over, and/or through the topsheets and/or LMSs. This may be desirable to achieve better bodily exudate (e.g., urine) distribution into an absorbent core, for example. As a further example, this may further be desirable to achieve reduced leakage by maintaining the bodily exudates over a portion of the absorbent core for a suitable period of time so that they can be fully and properly absorbed by the core. In view of the foregoing, topsheets or LMSs should be improved to provide for better bodily exudate management.

### SUMMARY

The present disclosure is directed to an absorbent article as defined in claim 1, comprising a liquid permeable topsheet, a liquid impermeable backsheet, and an absorbent core positioned at least partially intermediate the liquid permeable topsheet and the liquid impermeable backsheet. The absorbent article may comprise a substantially laterally-extending separation element defining a visual front portion and a visual back portion on a wearer-facing surface of the absorbent article. The liquid permeable topsheet may comprise a first zone in the visual front portion that comprises a first geometric treatment and a second zone in the visual back portion that comprises a second geometric treatment. The absorbent article comprises a lateral axis defining a front region and a back region. The liquid permeable topsheet comprises a first zone at least partially in the front region. The first zone comprises a first morphological treatment configured for urine handling. The liquid permeable topsheet comprises a second zone at least partially in the back region. The second zone comprises a second morphological treatment configured for BM handling. A pattern of the first morphological treatment in the first zone is nonsymmetrical to, or symmetrical to, a pattern of the second morphological treatment in the second zone about the lateral axis.

In one form, the present disclosure is directed, in part, to an absorbent article comprising
a liquid permeable topsheet, a liquid impermeable backsheet, a lateral axis, and an absorbent core positioned at least partially intermediate the liquid permeable topsheet and the liquid impermeable backsheet. The liquid permeable topsheet comprises a first zone at least partially positioned on a first side of the lateral axis and a second zone at least partially positioned on a second side of the lateral axis. The first zone may have apertures defined therein having an effective aperture area in the range of about 0.2mm² to about 15 mm² according to the Aperture Test. The first zone may have a % effective open area of about 15% to about 40% according to the Aperture Test. The second zone may have apertures defined therein having an effective aperture area in the range of about 0.05mm² to about 2mm² according to the Aperture Test. The second zone may have a % effective open area of about 2% to about 15% according to the Aperture Test. The apertures in the first zone may be at least about 25% larger than the apertures in the second zone.

In one form, the present disclosure is directed, in part, to absorbent article comprising a liquid permeable topsheet, a liquid impermeable backsheet, a lateral axis defining a front region on a first side of the lateral axis and a back region on a second side of the lateral axis, and an absorbent core positioned at least partially intermediate the liquid permeable topsheet and the liquid impermeable backsheet, The liquid permeable topsheet may comprise a first zone situated primarily in the front region and having a substantially transferrable chemical treatment and a second zone situated primarily in the back region and having the substantially transferrable chemical treatment. A basis weight of the substantially transferrable chemical treatment may be greater in the second zone than in the first zone.

In one form, the present disclosure is directed, in part, to an absorbent article comprising a liquid permeable topsheet, a liquid impermeable backsheet, a laterally-extending separation element defining a front region on a first side of the separation element and a back region on a second side of the separation element, and an absorbent core positioned at least partially intermediate the liquid permeable topsheet and the liquid impermeable backsheet. The liquid permeable topsheet may comprise a first zone situated primarily in the front region and having a first substantially transferrable chemical treatment that may be hydrophobic and a second zone situated primarily in the back region and having a second substantially transferrable chemical treatment that may have a different hydrophilicity as the first chemical treatment.

In one form, the present disclosure is directed, in part, to an absorbent article comprising
a liquid permeable topsheet, a liquid impermeable backsheet, a liquid management system, and an absorbent core positioned at least partially intermediate the liquid management system and the liquid impermeable backsheet. The liquid management system is positioned at least partially intermediate the liquid permeable topsheet and the absorbent core. The liquid permeable topsheet and the liquid management system may comprise a first zone on a first side of a lateral axis of the absorbent article and a second zone on a second side of the lateral axis. Portions of the liquid management system may extend into or through portions of the liquid impermeable topsheet or portions of the liquid impermeable topsheet may extend into or through portions of the liquid management system in the first zone. Portions of the liquid management system may extend into or through portions of the liquid impermeable topsheet or portions of the liquid impermeable topsheet may extend into or through portions of the liquid management system in the second zone. The first zone or the second zone may comprise a substantially transferrable chemical treatment.

In one form, the present disclosure is directed, in part, to an absorbent article comprising
a liquid permeable topsheet, a liquid impermeable backsheet, and an absorbent core positioned at least partially intermediate the liquid permeable topsheet and the liquid impermeable backsheet. The liquid permeable topsheet may comprise a flow control material. The flow control material may form an at least mostly enclosed perimeter over at least a portion of the absorbent core. The flow control material may extend less than 0.2mm outwardly from the liquid permeable topsheet, measured according to the Flow Control Material Outward Extension Method herein, and may penetrate a portion of the liquid permeable topsheet.

In one form, the present disclosure is directed, in part, to an absorbent article comprising a liquid permeable topsheet, a liquid impermeable backsheet, a lateral axis defining a front region of the absorbent article on a first side of the lateral axis and a back region of the absorbent article on a second side of the lateral axis, and an absorbent core positioned at least partially intermediate the liquid permeable topsheet and the liquid impermeable backsheet. The liquid permeable topsheet may comprise a first zone situated primarily in the front region and comprising a first flow control material and a second zone situated primarily in the back region and comprising a second flow control material. The first flow control material may be different than the second flow control material.

In one form, the present disclosure is directed, in part, to an absorbent article comprising
a liquid permeable topsheet, a liquid impermeable backsheet, a liquid management system, and an absorbent core positioned at least partially intermediate the liquid management system and the liquid impermeable backsheet. The liquid management system is positioned at least partially intermediate the liquid permeable topsheet and the absorbent core. The liquid permeable topsheet or the liquid management system may comprise a flow control material positioned in or surrounding a urine or feces insult zone.

### BRIEF DESCRIPTION OF THE DRAWINGS

The above-mentioned and other features and advantages of the present disclosure, and the manner of attaining them, will become more apparent and the disclosure itself will be better understood by reference to the following description of non-limiting examples of the disclosure taken in conjunction with the accompanying drawings, wherein:
Fig. 1 is a top view of an absorbent article with some layers partially removed in accordance with the present disclosure;
Fig. 2 is a cross-sectional view of the absorbent article taken about line 2-2 of Fig. 1 in accordance with the present disclosure;
Fig. 3 is a view of the absorbent article of Fig. 2 where the absorbent article has been at least partially loaded with fluid in accordance with the present disclosure;
Fig. 4 is a top view of another absorbent article with some layers partially removed in accordance with the present disclosure;
Fig. 5 is a cross-sectional view of the absorbent article taken about line 5-5 of Fig. 4 in accordance with the present disclosure;
Fig. 6 is a top view of an absorbent core of the absorbent article of Fig. 4 with some layers partially removed in accordance with the present disclosure;
Fig. 7 is a cross-sectional view of the absorbent core taken about line 7-7 of Fig. 6 in accordance with the present disclosure;
Fig. 8 is a cross-sectional view of the absorbent core taken about line 8-8 of Fig. 6 in accordance with the present disclosure;
Fig. 9 is a top view of a LMS of the absorbent article of Fig. 4 with some layers partially removed in accordance with the present disclosure;
Fig. 10 is a cross-sectional view of the liquid management system taken about line 10-10 of Fig. 9 in accordance with the present disclosure;
Figs. 11-14 are examples longitudinal cross-sectional views of a portion of an absorbent article having a channel in an absorbent core and an LMS and a substantially laterally-extending separation element extending from the topsheet in accordance with the present disclosure;
Figs. 15-17 illustrate examples topsheets (and LMSs if interpenetrating the topsheets) having two zones, each zone having one or more treatments or no treatments, in accordance with the present disclosure;
Figs. 18 and 19 illustrate examples topsheets (and LMSs if interpenetrating the topsheets) having four zones, each zone having one or more treatments or no treatments, in accordance with the present disclosure;
Figs. 20 to 21C illustrate example topsheets (and LMSs if interpenetrating the topsheets) having three or more zones, each zone having one or more treatments or no treatments, in accordance with the present disclosure;
Fig. 22 is an example of a topsheet (and LMS if interpenetrating the topsheet) having six zones, each zone having one or more treatments or no treatments, in accordance with the present disclosure;
Fig. 23 is a photograph of the example topsheet (and LMS) of Fig. 22, in accordance with the present disclosure;
Fig. 24 is an example of a topsheet (and LMS if interpenetrating the topsheet) having five zones, each zone having one or more treatments or no treatments, in accordance with the present disclosure;
Fig. 25 is a photograph of the example topsheet (and LMS) of Fig. 24, in accordance with the present disclosure;
Fig. 26 is an example of a topsheet (and LMS if interpenetrating the topsheet) having four zones, each zone having one or more treatments or no treatments, in accordance with the present disclosure;
Fig. 27 is an example of a topsheet (and LMS if interpenetrating the topsheet) having four zones, each zone having one or more treatments or no treatments, in accordance with the present disclosure;
Fig. 28 is a photograph of the example topsheet (and LMS) of Fig. 27, in accordance with the present disclosure;
Fig. 29 is an example of a topsheet (and LMS if interpenetrating the topsheet) having four zones, each zone having one or more treatments or no treatments, in accordance with the present disclosure;
Fig. 30 is a photograph of the example topsheet (and LMS) of Fig. 29, in accordance with the present disclosure;
Fig. 31 is an example of a topsheet (and LMS if interpenetrating the topsheet) having two zones, each zone having one or more treatments or no treatments, in accordance with the present disclosure;
Fig. 32 is a photograph of the example topsheet (and LMS) of Fig. 31, in accordance with the present disclosure;
Figs. 33-42 are example patterns of zonal topsheets in accordance with the present disclosure;
Fig. 43 is an example of a geometric treatment comprising apertures in accordance with the present disclosure;
Fig. 44A is an example geometric treatment comprising apertures and embossing in accordance with the present disclosure;
Fig. 44B illustrates an example substrate for use a portion of, or all of, a topsheet in accordance with the present disclosure;
Fig. 44C illustrates an example fabric substrate for use a portion of, or all of, a topsheet in accordance with the present disclosure;
Fig. 44D illustrates an example mesh for use a portion of, or all of, a topsheet in accordance with the present disclosure;
Fig. 44E illustrates an example film for use a portion of, or all of, a topsheet in accordance with the present disclosure;
Fig. 44F illustrates an example film for use a portion of, or all of, a topsheet in accordance with the present disclosure;
Fig. 45 is an example morphological treatment comprising embossing in accordance with the present disclosure;
Fig. 46A is an illustration of an example morphological treatment comprising embossing in accordance with the present disclosure;
Fig. 46B is a photograph of a topsheet having the morphological treatment of Fig. 46A in accordance with the present disclosure;
Figs. 47-49 are examples morphological treatments comprising embossing in accordance with the present disclosure;
Fig. 50 is an example of a morphological treatment comprising puckering in accordance with the present disclosure;
Fig. 51 is an example of a morphological treatment comprising folding in accordance with the present disclosure;
Fig. 52 is a perspective view of an example morphological treatment where portions of a liquid management system extend into or through a liquid permeable topsheet in accordance with the present disclosure;
Fig. 53 is an exploded perspective view taken from circle 43 of Fig. 52 in accordance with the present disclosure;
Fig. 54 is a perspective view of an example morphological treatment where portions of a liquid permeable topsheet extend into or through a liquid management system in accordance with the present disclosure;
Fig. 55 is an exploded perspective view taken from circle 55 of Fig. 54 in accordance with the present disclosure;
Fig. 56 is a perspective view a process used to make the morphological treatments of Figs. 52 and 44 in accordance with the present disclosure;
Fig. 57 is a front view of engagement of portions rolls from the process of Fig. 56 in accordance with the present disclosure;
Fig. 58 is a photograph of a morphological or chemical treatment in a topsheet in accordance with the present disclosure;
Fig. 59 is an illustration of a chemical treatment pattern in accordance with the present disclosure;
Fig. 60 is a photograph of the chemical treatment pattern of Fig. 59 on a topsheet in accordance with the present disclosure;
Fig. 61 is an illustration of a chemical treatment pattern in accordance with the present disclosure;
Fig. 62 is a photograph of the chemical treatment pattern of Fig. 61 on a topsheet in accordance with the present disclosure;
Figs. 63-68 are illustrations of example absorbent articles having various zones with various treatments in accordance with the present disclosure; and
Fig. 69 illustrates an example package of absorbent articles in accordance with the present disclosure.

### DETAILED DESCRIPTION

Various non-limiting embodiments of the present disclosure will now be described to provide an overall understanding of the principles of the structure, function, manufacture, and use of the absorbent articles comprising substrates comprising zonal treatments disclosed herein. One or more examples of these non-limiting embodiments are illustrated in the accompanying drawings, Those of ordinary skill in the art will understand that the absorbent articles comprising substrates comprising zonal treatments described herein and illustrated in the accompanying drawings are non-limiting example embodiments and that the scope of the various non-limiting embodiments of the present disclosure are defined solely by the claims. The features illustrated or described in connection with one non-limiting embodiment may be combined with the features of other non-limiting embodiments. Such modifications and variations are intended to be included within the scope of the present disclosure.

### Introduction

The term "absorbent article, as used herein, refers to disposable devices such as infant, child, or adult diapers, sanitary napkins, adult incontinence products, pant-style diapers, training pants, diaper inserts, and the like which are placed against or in proximity to the body of the wearer to absorb and contain the bodily exudates (e.g., urine and BM) discharged from the body, Typically, these articles comprise a topsheet, backsheet, an absorbent core, optionally a LMS, and typically other components, with the absorbent core normally placed at least partially between the backsheet and the LMS or between the topsheet and the backsheet, The absorbent articles of the present disclosure will be further illustrated in the below description and in the Figures in the form of a taped diaper. Nothing in this description should be, however, considered to limit the scope of the claims. As such the present disclosure applies to any suitable form of absorbent articles (e.g., training pants, taped diapers, adult incontinence products-in either taped or pant forms, sanitary napkins).

The term "nonwoven web", as used herein, means a manufactured sheet, web, or batt of directionally or randomly orientated fibers, bonded by friction, and/or cohesion, and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers may have diameters ranging from less than about 0.001 mm to more than about 0.2 mm and may come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electro spinning, carding, and airlaying. The basis weight of nonwoven webs is usually expressed in grams per square meter (g/m² or gsm).

The terms "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element.

The term "channel", as used herein, is a region or zone in a material layer that has a substantially lower basis weight (e.g., less than 50%, less than 70%, less than 90%) than the surrounding material in the material layer. The channel may be a region in a material layer that is substantially material-free (e.g., 90% material-free, 95% material-free, or 99% material-free, or completely material-free). A channel may extend through one or more material layers. The channels generally have a lower bending modulus than the surrounding regions of the material layer, enabling the material layer to bend more easily and/or contain more bodily exudates within the channels than in the surrounding areas of the material layer. Thus, a channel is not merely an indentation in the material layer that does not create a reduced basis weight in the material layer in the area of the channel.

The term "geometric treatment", as used herein, means at least a portion or region of a single or multi-layer substrate that comprises elements that are apertures of any suitable size and shape and/or elements that form a morphological treatment.

The term "morphological treatment", as used herein, means at least a portion or region of a single or multi-layer substrate that comprises elements having three-dimensional features, embossments, interpenetration of one layer into or through another layer (e.g., one or more layers of the LMS into the topsheet or the topsheet into one or more layer of the LMS), out-of-plane bumps, out-of-plane ridges, out-of-plane tufts, out-of-plane pleats, out-of-plane ripples, or fold lines. A morphological treatment causes a substantially uniform planar substrate to be transformed from a first morphological configuration (generally flat and planar) to another morphological configuration (generally not flat and planar). The morphological treatment is formed of a plurality of the elements. For the avoidance of doubt, a morphological treatment does not include apertures, but an apertured material may be subjected to a morphological treatment.

The term "chemical treatment", as used herein, means at least a portion or region of a single or multi-layer substrate that has a compound, composition, or substance applied to at least a portion thereof. Some examples are one or more skin care compositions, surfactants, inks, dyes, pigments, hydrophilic coatings, hydrophobic coatings, lotions, enzyme inhibitors, vitamins, and/or active ingredients. The chemical treatment may be sprayed on, printed on, slot coated, or otherwise applied to the at least a portion or region of the substrate.

The term "substantially durable", as used herein, means a chemical treatment where at least 94%, at least 95%, at least 96%, at least 97%, at least 98%, at least 99%, or more of the applied chemical treatment remains on the substrate from the time of manufacture throughout a typical period of intended use (e.g., from a point in time where an absorbent article is applied to a wearer to a point in time when the absorbent article is removed from the wearer and discarded).

The term "substantially transferrable", as used herein, means a chemical treatment where at least 10%, at least 20%, at least 30%, at least 40%, at least 50%, or even at least 60% or more of the applied chemical treatment transfers to the skin of a wearer during a typical period of intended use (e.g., from a point in time where an absorbent article is applied to a wearer to a point in time when the absorbent article is removed from the wearer and discarded).

The term "hydrophilic coating", as used herein, means a chemical treatment applied to a substrate to cause the substrate to become hydrophilic or more hydrophilic.

The term "hydrophilic", as used herein, refers to a substrate or composition having a contact angle less than or equal to 90° according to The American Chemical Society Publication "Contact Angle, Wettability, and Adhesion," edited by Robert F. Gould and copyrighted in 1964.

The term "hydrophobic coating", as used herein, means a chemical treatment applied to a substrate to cause the substrate to become hydrophobic or more hydrophobic.

The term "hydrophobic", as used herein, refers to a substrate or composition having a contact angle greater than or equal to 90° according to The American Chemical Society Publication "Contact Angle, Wettability, and Adhesion," edited by Robert F. Gould and copyrighted in 1964.

The term "flow control material", as used herein, may be a chemical treatment where a substance is applied to a substrate (such as a liquid permeable topsheet) that at least partially restricts, or fully restricts, the flow of bodily exudates therethrough. The flow control material may be a wax, an ink (having a pigment), a non-tack adhesive, a hot melt adhesive, a substantially durable component of a skin care composition, a polyolefin, a high molecular weight alcohol (one example of a component of a skin care composition), or other compositions substantially solid at 20 degrees C, for example. The flow control material may be substantially durable. The flow control material may also comprise when a material is applied to a substrate (e.g., a topsheet) and then the material and the substrate are run through two or more rolls to melt, join, bond, or attach the flow control material to the substrate.

The term "active ingredient", as used herein, means an ingredient that has a chemical, biochemical, and/or biological effect - i.e., causes, initiates, or affects a change in a chemical, biochemical, and/or biological reaction, system, process, or equilibrium. This is opposed to inactive ingredients which may typically be used as carrier media, viscosity modifiers, melt temperature mediators, or for purely physical reasons (i.e., fillers).

The term "enzyme inhibitor", as used herein, means a molecule, which binds to enzymes and decreases their activity.

### General Description of the Substrates Having Zonal Treatments

The absorbent articles of the present disclosure comprise one or more single or multi-layer substrates comprising one or more zones, alternatively two or more zones, alternatively three or more zones, and alternatively four or more zones, and so forth. Each of the zones in the substrates may have different treatments or the same treatments. One or more zones of the substrates may not have a treatment at all. Some of, none of, or all of the zones may comprise flow control materials (can be referred to herein as a "treatment" generally). Each of the zones may have different or the same treatments to better provide for urine management or BM management owing to the fact that the rheology and solids content of BM and urine may be quite different. Alternatively, each of the zones may have different or the same treatments to provide the appearance of better urine or BM management or to provide the caregiver or wearer with clues as to the correct orientation of the absorbent article when donned on the wearer.

The substrates may be, for example, a liquid permeable topsheet, a patch or layer positioned over the liquid permeable topsheet, one or more layers of a LMS, and/or other substrates within an absorbent article. The zones in the substrate or substrates (used interchangeably herein) may be formed at least partially in the front and/or back regions of the absorbent article, in the crotch region of the absorbent article, in regions of the absorbent article on a first and second side of either a lateral or longitudinal axis, in regions of the absorbent article on the same or different side of a substantially laterally-extending separation element, in regions of the absorbent article dispersed throughout other regions of the absorbent article, and/or otherwise dispersed throughout regions of the substrates of the absorbent articles. Each zone in the substrate may have one or more of the same or different geometric treatments, morphological treatments, and/or chemical treatments (together "treatments") as another zone in the substrate. By "the same", it is meant that the treatments are of the same type (e.g., both embossments) and have the same pattern, height, length, width, size, shape, frequency, and other dimension, for example. By "different", it is meant that the treatments may be the same (e.g., both embossments), but the pattern, height, length, width, size, shape, frequency, or other dimension is different. Alternatively, "different" can mean that the treatment is not the same as another treatment (e.g., apertures as one treatment and embossments as another treatment). Although the treatments in this scenario are "different" they may form symmetrical or asymmetrical patterns, or repeating or non-repeating patterns, about a lateral or longitudinal axis or a substantially laterally-extending separation element of an absorbent article. One or more zones in the substrate may overlap with, not overlap with, coincide with, or not coincide with other zones in the substrate. As such, the zones may be separate from each other or may overlap with each other. Any number of zones having the same or different treatments, or no treatments, may be provided in a particular substrate. A substrate may have one or more layers and the various treatments may be provided in one, all, or less than all of the layers.

Before the various zones and treatments and/or flow control material within the zones are discussed, a general discussion of absorbent articles will be presented to frame an example context of the zonal treatments and flow control materials of the present disclosure.

### General Description of an Example Absorbent Article

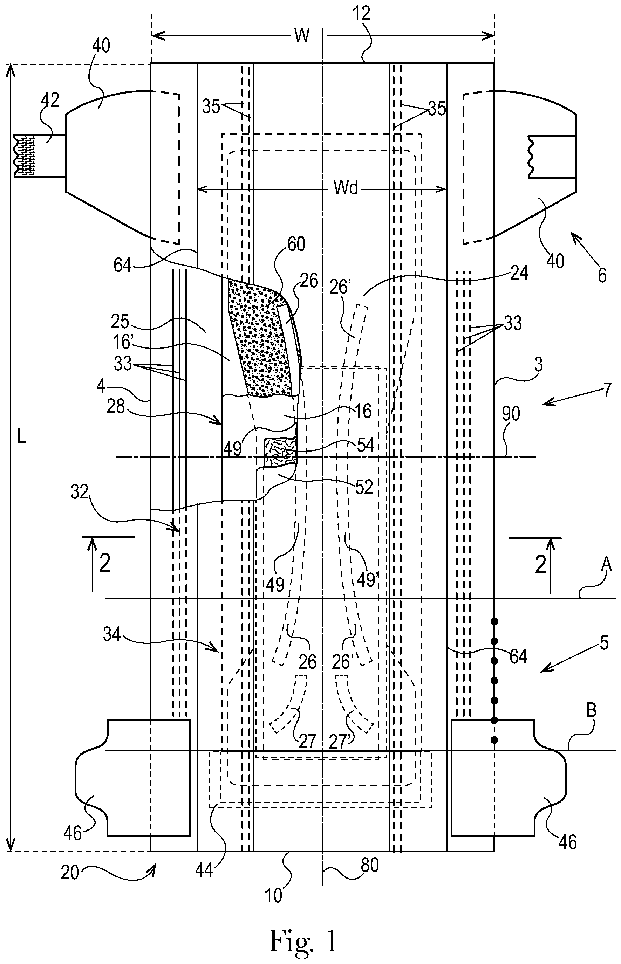

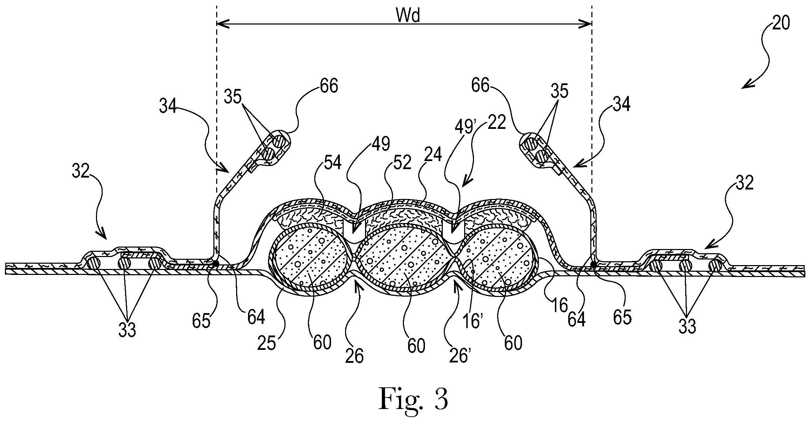

An example absorbent article 20 according to the present disclosure, shown in the form of a diaper, is represented in Figs. 1-3. Fig. 1 is a plan view of the diaper, in a flat-out state, wearer-facing surface toward the viewer, with portions of the structure being cut-away to more clearly show the construction of the diaper. This diaper is shown for illustration purpose only as the present disclosure may be used for making a wide variety of diapers and other absorbent articles.

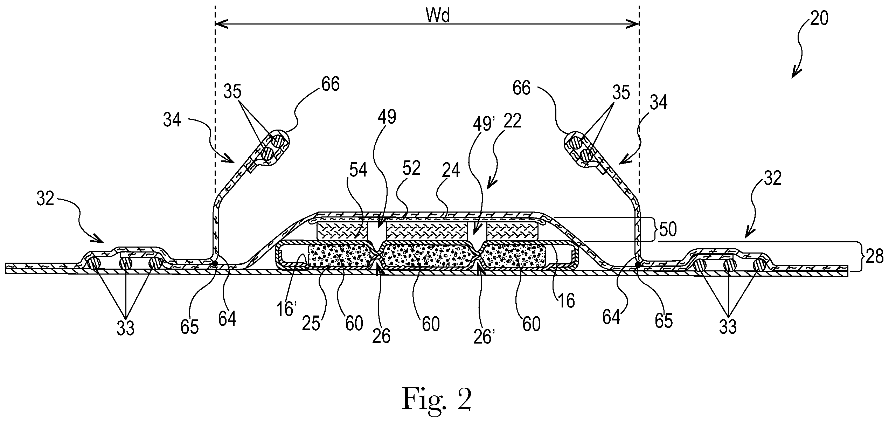

The absorbent article may comprise a liquid permeable topsheet 24, a liquid impermeable backsheet 25, an absorbent core 28 positioned at least partially intermediate the topsheet 24 and the backsheet 25, and barrier leg cuffs 34. The absorbent article also comprises a liquid management system ("LMS") 50 (shown in Fig. 2), which, in the example represented, may comprises a distribution layer 54 and an acquisition layer 52 that will both be further discussed below. In various embodiments, the acquisition layer 52 may instead distribute bodily exudates and the distribution layer 54 may instead acquire bodily exudates or both layers may distribute and/or acquire bodily exudates. The LMS 50 may also be provided as a single layer or two or more layers. The absorbent article may also comprise elasticized gasketing cuffs 32 joined to the chassis of the absorbent article, typically via the topsheet and/or backsheet, and substantially planar with the chassis of the diaper.

The Figures also show typical taped diaper components such as a fastening system comprising adhesive tabs 42 or other mechanical fasteners attached towards the rear edge of the absorbent article 20 and cooperating with a landing zone 44 on the front of the absorbent article 20. The absorbent article may also comprise other typical elements, which are not represented, such as a rear elastic waist feature and a front elastic waist feature, for example.

The absorbent article 20 may comprise a front waist edge 10, a rear waist edge 12 longitudinally opposing the front waist edge 10, a first side edge 3, and a second side edge 4 laterally opposing the first side edge 3. The front waist edge 10 is the edge of the absorbent article 20 which is intended to be placed towards the front of the user when worn, and the rear waist edge 12 is the opposite edge. Together the front waist edge 10 and the rear waist edge form waist opening when the absorbent article 20 is donned on a wearer. The absorbent article 20 may have a longitudinal axis 80 extending from the lateral midpoint of the front waist edge 10 to a lateral midpoint of the rear waist edge 12 of the absorbent article 20 and dividing the absorbent article 20 in two substantially symmetrical halves relative to the longitudinal axis 80, with article placed flat and viewed from the wearer-facing surface as illustrated Fig. 1. The absorbent article may also have a lateral axis 90 extending from the longitudinal midpoint of the first side edge 3 to the longitudinal midpoint of the second side edge 4. The length L of the absorbent article 20 may be measured along the longitudinal axis 80 from the front waist edge 10 to the rear waist edge 12. The crotch width of the absorbent article 20 may be measured along the lateral axis 90 from the first side edge 3 to the second side edge 4. The absorbent article 20 may comprise a front waist region 5, a rear waist region 6, and a crotch region 7. The front waist region, the rear waist region, and the crotch region each define 1/3 of the longitudinal length of the absorbent article. Front and back portions may also be defined on opposite sides of the lateral axis 90.

The topsheet 24, the backsheet 25, the absorbent core 28, and the other article components may be assembled in a variety of configurations, in particular by gluing or heat embossing, for example. Example diaper configurations are described generally in U.S. Pat. No. 3,860,003, U.S. Pat. No. 5,221,274, U.S. Pat. No. 5,554,145, U.S. Pat. No. 5,569,234, U.S. Pat. No. 5,580,411, and U.S. Pat. No. 6,004,306.

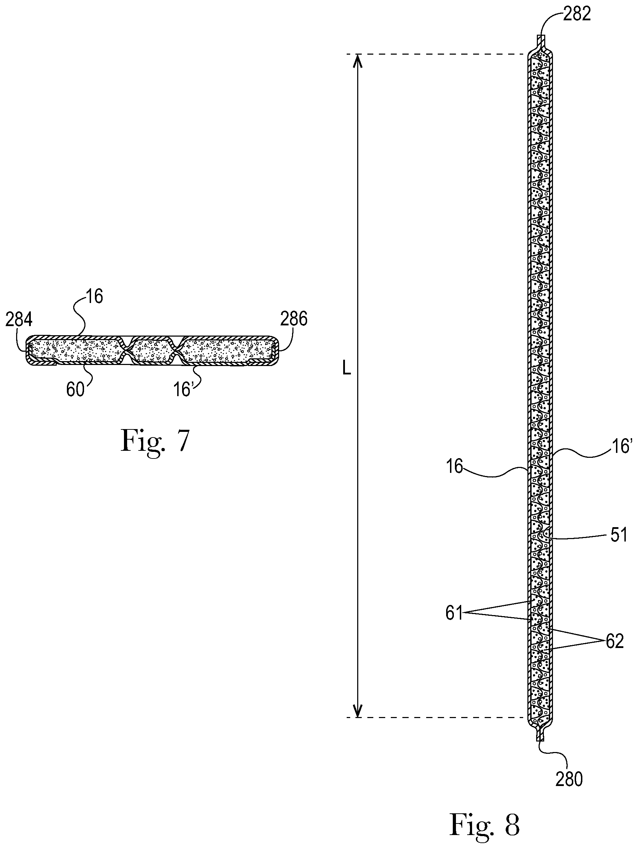

The absorbent core 28 may comprise an absorbent material comprising 75% to 100%, at least 80%, at least 85%, at least 90%, at least 95%, or at least 99%, all by weight, of the absorbent material, specifically reciting all 0.1% increments within the above-specified ranges and all ranges formed therein or thereby, and a core wrap enclosing the absorbent material. The core wrap may typically comprise two materials, substrates, or nonwoven materials 16 and 16' (see Fig. 8) for the top side and bottom side of the core.

The absorbent core 28 may comprises one or more channels, represented in Fig. 1 as the four channels 26, 26' and 27, 27'. Additionally or alternative, the LMS 50 may comprises one or more channels, represented in Figs. 1-3 as channels 49, 49'. In some embodiments, the channels of the LMS 50 may be positioned within the absorbent article 20 such they aligned with, substantially aligned with, overlap, or at least partially overlap, the channels of the absorbent core 28. These and other components of the absorbent articles will now be discussed in more details.

### Topsheet

The topsheet 24 is the part of the absorbent article that is directly in contact with the wearer's skin. The topsheet 24 may be joined to the backsheet 25, the core 28 and/or any other layers as is known to those of skill in the art. Usually, the topsheet 24 and the backsheet 25 are joined directly to each other in some locations (e.g., on or close to the periphery of the article) and are indirectly joined together in other locations by directly joining them to one or more other elements of the absorbent article 20.

The topsheet 24 may be compliant, soft-feeling, and non-irritating to the wearer's skin. Further, at least a portion of the topsheet 24 may be liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. If the topsheet 24 includes fibers, the fibers may be spunbond, carded, wet-laid, meltblown, hydroentangled, or otherwise processed as is known in the art, in particular spunbond PP nonwoven.

Typical absorbent article topsheets have a basis weight of from about 5 gsm to about 50 gsm, from about 10 to about 35 gsm or from about 12 to about 30 gsm, but other basis weights are within the scope of the present disclosure.

### Backsheet

The backsheet 25 is generally that portion of the absorbent article 20 positioned adjacent the garment-facing surface of the absorbent core 28 and which prevents, or at least inhibits, the bodily exudates absorbed and contained therein from soiling articles such as bedsheets and undergarments. The backsheet 25 is typically impermeable, or at least substantially impermeable, to liquids (e.g., urine, running BM), but permeable to vapors to allow the diaper to "breath". The backsheet may, for example, be or comprise a thin plastic film such as a thermoplastic film having a thickness of about 0.012 mm to about 0.051 mm. Example backsheet films include those manufactured by Tredegar Corporation, based in Richmond, VA, and sold under the trade name CPC2 film. Other suitable backsheet materials may include breathable materials which permit vapors to escape from the absorbent article 20 while still preventing, or at least inhibiting, bodily exudates from passing through the backsheet 25. Example breathable materials may include materials such as woven webs, nonwoven webs, composite materials such as film-coated nonwoven webs, microporous films, and monolithic films.

The backsheet 25 may be joined to the topsheet 24, the absorbent core 28, and/or any other element of the absorbent article 20 by any attachment methods known to those of skill in the art. Suitable attachment methods are described above with respect to methods for joining the topsheet 24 to other elements of the absorbent article 20.

### Absorbent Core

As used herein, the term "absorbent core" refers to the individual component of the absorbent article having the most absorbent capacity and that comprises an absorbent material. The absorbent core may comprise a core wrap or core bag (hereafter "core wrap") enclosing the absorbent material. The term "absorbent core" does not include the LMS or any other component of the absorbent article which is not either integral part of the core wrap or placed within the core wrap. The absorbent core may comprise, consist essentially of, or consist of, a core wrap, absorbent material as defined below, and glue enclosed within the core wrap. Pulp or air-felt may also be present within the core wrap and may form a portion of the absorbent material. The absorbent core periphery, which may be the periphery of the core wrap, may define any suitable shape, such as a "T," "Y," "hour-glass," or "dog-bone" shape, for example. An absorbent core periphery having a generally "dog bone" or "hour-glass" shape may taper along its width towards the middle or "crotch" region of the core. In this way, the absorbent core may have a relatively narrow width in an area of the absorbent core intended to be placed in the crotch region of an absorbent article.

The absorbent core 28 of the present disclosure may comprise an absorbent material with a high amount of superabsorbent polymers (herein abbreviated as "SAP") enclosed within a core wrap. The SAP content may represent 70% to 100% or at least 70%, 75%, 80%, 85%, 90%, 95%, 99%, or 100% by weight of the absorbent material contained in the core wrap. The SAP useful with the present disclosure may include a variety of water-insoluble, but water-swellable polymers capable of absorbing large quantities of fluids. The core wrap is not considered as absorbent material for the purpose of assessing the percentage of SAP in the absorbent core. The remainder of the absorbent material in the core 28 may be air-felt.

"Absorbent material" means a material which has some absorbency property or liquid retaining properties, such as SAP, cellulosic fibers as well as synthetic fibers. Typically, glues used in making absorbent cores have no absorbency properties and are not considered as absorbent material. The SAP content may be higher than 80%, for example at least 85%, at least 90%, at least 95%, at least 99%, and even up to and including 100% of the weight of the absorbent material contained within the core wrap, as stated above. This provides a relatively thin core compared to conventional cores typically comprising between 40-60% SAP, for example, and high content of cellulose fibers or airfelt. The absorbent material may comprise less than 15% or less than 10% weight percent of natural or synthetic fibers, less than 5% weight percent, less than 3% weight percent, less than 2% weight percent, less than 1% weight percent, or may even be substantially free of, or free of, natural and/or synthetic fibers, specifically reciting all 0.1% increments within the specified ranges and all ranges formed therein or thereby. The absorbent material may comprise little or no airfelt (cellulose) fibers, in particular the absorbent core may comprise less than 15%, 10%, 5%, 3%, 2%, 1% airfelt (cellulose) fibers by weight, or may even be substantially free of, or free of, cellulose fibers, specifically reciting all 0.1% increments within the specified ranges and all ranges formed therein or thereby.

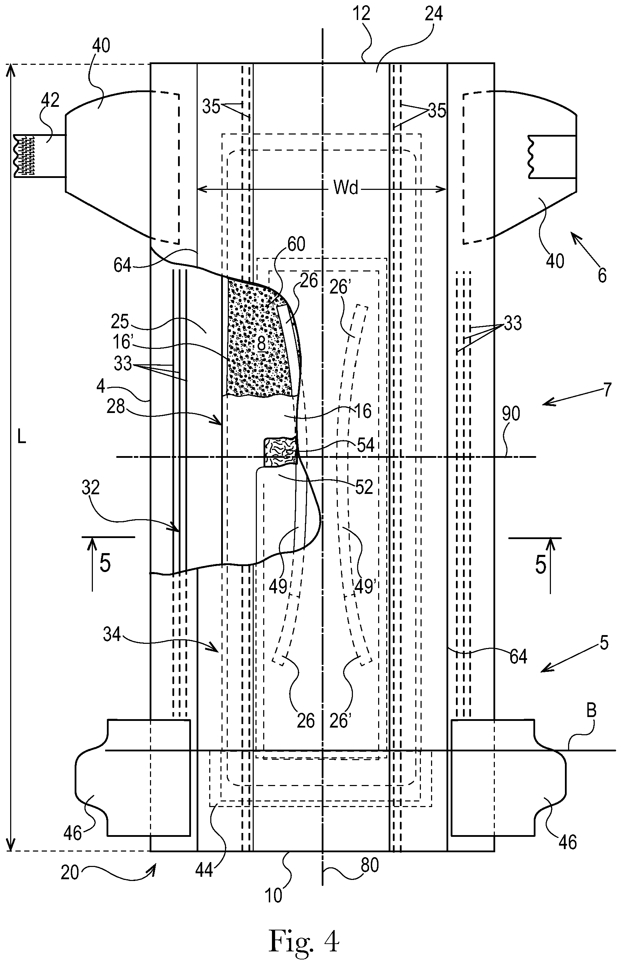

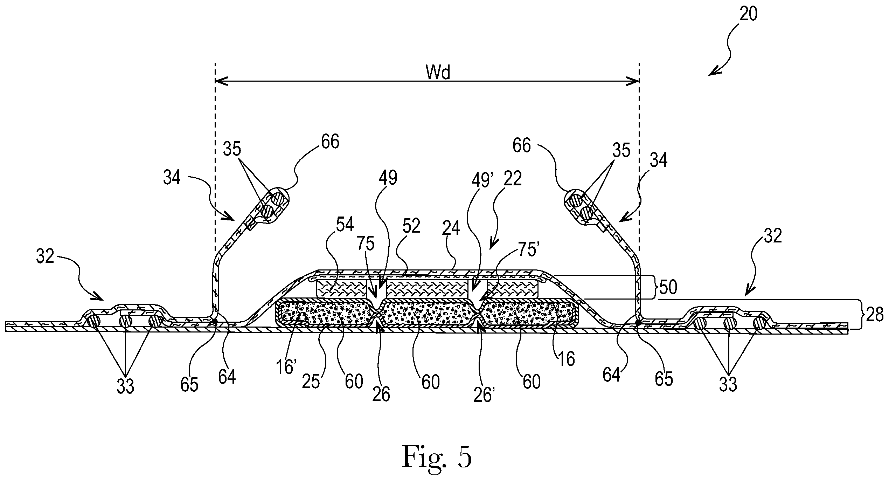

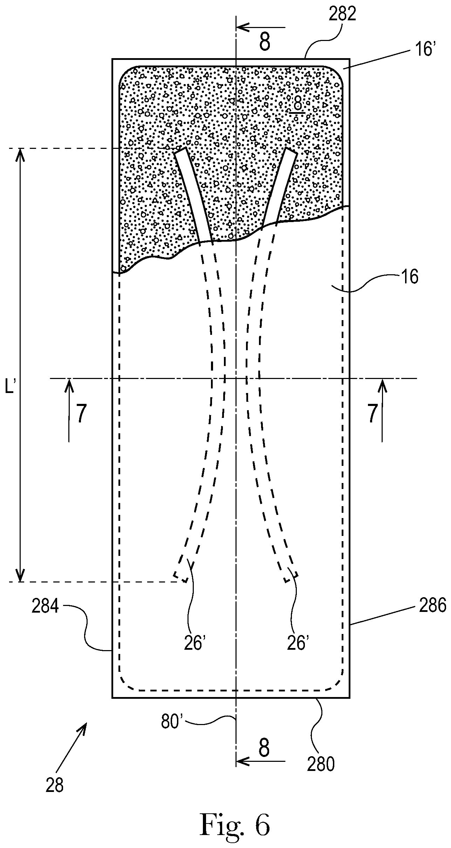

The example absorbent core 28 of the absorbent article of Figs. 4 and 5 is shown in isolation in Figs. 6-8. The absorbent core 28 may comprises a front side 280, a rear side 282, and two longitudinal sides 284, 286 joining the front side 280 and the rear side 282. The absorbent core 28 may also comprise a generally planar top side and a generally planar bottom side. The front side 280 of the core 28 is the side of the core 28 intended to be placed towards the front waist edge 10 of the absorbent article. The core 28 may have a longitudinal axis 80' corresponding substantially to the longitudinal axis 80 of the absorbent article, as seen from the top in a planar view as in Fig. 1. The absorbent material may be distributed in higher amount towards the front side than towards the rear side as more absorbency may be required at the front in particular articles. The absorbent material may have a non-uniform basis weight or a uniform basis weight across any portion of the core. The core wrap may be formed by two nonwoven materials, substrates, laminates, or other materials, 16, 16' which may be at least partially sealed along the sides of the absorbent core. The core wrap may be at least partially sealed along its front side 280, rear side 282, and two longitudinal sides 284, 286 so that substantially no absorbent material leaks out of the absorbent core wrap. The first material, substrate, or nonwoven 16 may at least partially surround the second material, substrate, or nonwoven 16' to form the core wrap, as illustrated in Fig. 7. The first material 16 may surround a portion of the second material 16' proximate to the first and second side edges 284 and 286.

Cores comprising relatively high amount of SAP with various core designs are disclosed in U.S. Pat. No. 5,599,335 (Goldman), EP 1,447,066 (Busam), WO 95/11652 (Tanzer), U.S. Pat. Publ. No. 2008/0312622A1 (Hundorf), and WO 2012/052172 (Van Malderen).

The absorbent material may be one or more continuous layers present within the core wrap. Alternatively, the absorbent material may be comprised of individual pockets or stripes of absorbent material enclosed within the core wrap. In the first case, the absorbent material may be, for example, obtained by the application of a single continuous layer of absorbent material. The continuous layer of absorbent material, in particular of SAP, may also be obtained by combining two or more absorbent layers having discontinuous absorbent material application pattern, wherein the resulting layer is substantially continuously distributed across the absorbent particulate polymer material area, as disclosed in U.S. Pat. Appl. Publ. No. 2008/0312622A1 (Hundorf), for example. The absorbent core 28 may comprise a first absorbent layer and a second absorbent layer. The first absorbent layer may comprise the first material 16 and a first layer 61 of absorbent material, which may be 100% or less of SAP. The second absorbent layer may comprise the second material 16' and a second layer 62 of absorbent material, which may also be 100% or less of SAP. The absorbent core 28 may also comprise a fibrous thermoplastic adhesive material 51 at least partially bonding each layer of absorbent material 61, 62 to its respective material 16 or 16'. This is illustrated in Figs. 7-8, as an example, where the first and second SAP layers have been applied as transversal stripes or "land areas" having the same width as the desired absorbent material deposition area on their respective substrate before being combined. The stripes may comprise different amount of absorbent material (SAP) to provide a profiled basis weight along the longitudinal axis of the core 80. The first material 16 and the second material 16' may form the core wrap.

The fibrous thermoplastic adhesive material 51 may be at least partially in contact with the absorbent material 61, 62 in the land areas and at least partially in contact with the materials 16 and 16' in the junction areas. This imparts an essentially three-dimensional structure to the fibrous layer of thermoplastic adhesive material 51, which in itself is essentially a two-dimensional structure of relatively small thickness, as compared to the dimension in length and width directions. Thereby, the fibrous thermoplastic adhesive material may provide cavities to cover the absorbent material in the land area, and thereby immobilizes this absorbent material, which may be 100% or less of SAP.

### Core Wrap

The core wrap may be made of a single substrate, material, or nonwoven folded around the absorbent material, or may comprise two (or more) substrates, materials, or nonwovens which are attached to another. Typical attachments are the so-called C-wrap and/or sandwich wrap. In a C-wrap, as illustrated, for example, in Figs. 2 and 7, the longitudinal and/or transversal edges of one of the substrates are folded over the other substrate to form flaps. These flaps are then bonded to the external surface of the other substrate, typically by gluing. Other techniques may be used to form a core wrap. For example, the longitudinal and/or transversal edges of the substrates may be bonded together and then folded underneath the absorbent core 28 and bonded in that position.

The core wrap may be at least partially sealed along all the sides of the absorbent core so that substantially no absorbent material leaks out of the core. By "substantially no absorbent material" it is meant that less than 5%, less than 2%, less than 1%, or about 0% by weight of absorbent material escape the core wrap. The term "seal" is to be understood in a broad sense. The seal does not need to be continuous along the whole periphery of the core wrap but may be discontinuous along part or the whole of it, such as formed by a series of seal points spaced on a line. A seal may be formed by gluing and/or thermal bonding.

The core wrap may also be formed by a single substrate which may enclose as in a parcel wrap the absorbent material and be sealed along the front side and rear side of the core and one longitudinal seal.

### SAP Deposition Area

The absorbent material deposition area 8 may be defined by the periphery of the layer formed by the absorbent material 60 within the core wrap, as seen from the top side of the absorbent core. The absorbent material deposition area 8 may have various shapes, in particular, a so-called "dog bone" or "hour-glass" shape, which shows a tapering along its width towards the middle or "crotch" region of the core. In this way, the absorbent material deposition area 8 may have a relatively narrow width in an area of the core intended to be placed in the crotch region of the absorbent article, as illustrated in Fig. 1. This may provide better wearing comfort. The absorbent material deposition area 8 may also be generally rectangular, for example as shown in Figs. 4-6, but other deposition areas, such as a "T," "Y," "hour-glass," or "dog-bone" shapes are also within the scope of the present disclosure.

### Channels in the Absorbent Core

The absorbent material deposition area 8 may comprise at least one channel 26, which is at least partially oriented in the longitudinal direction of the absorbent article 80 (i.e., has a longitudinal vector component). Other channels may be at least partially oriented in the lateral direction (i.e., has a lateral vector component) or in any other direction. In the following, the plural form "channels" will be used to mean "at least one channel". The channels may be circular, oblong, or be in the shape of a variety of other closed polygons. The channels may be formed in various ways. For example, the channels may be formed by zones within the absorbent material deposition area 8 which may be substantially free of, or free of, absorbent material, in particular, SAP. In addition or alternatively, the channels may also be formed by continuously or discontinuously bonding the top side of the core wrap to the bottom side of the core wrap through the absorbent material deposition area 8. The channels may be continuous or intermittent. The liquid management system 50, or another layer of the absorbent article, may also comprise channels, which may or not correspond to the channels of the absorbent core, as described in more detail below.

The absorbent core 28 may comprise more than two channels, for example, at least 3, at least 4, etc. Shorter channels may also be present, for example in the rear waist region 6 or the front waist region 5 of the core as represented by the pair of channels 27, 27' in Fig. 1 towards the front of the absorbent article 20. The channels may comprise one or more pairs of channels symmetrically arranged, or otherwise arranged relative to the longitudinal axis 80 or the lateral axis 90.

At least some or all of the channels may be permanent channels, meaning their integrity is at least partially maintained both in the dry state and in the wet state. Permanent channels may be obtained by provision of one or more adhesive materials, for example, the fibrous layer of adhesive material or construction glue that helps adhere a substrate with an absorbent material within the walls of the channel. Permanent channels may also be formed by bonding the upper side and lower side of the core wrap (e.g., the first substrate 16 and the second substrate 16') and/or the topsheet 24 to the backsheet 25 together through the channels. Typically, an adhesive may be used to bond both sides of the core wrap or the topsheet and the a backsheet through the channels, but it is possible to bond via other known processes, such as pressure bonding, ultrasonic bonding, heat bonding, or combination thereof. The core wrap or the topsheet 24 and the backsheet 25 may be continuously bonded or intermittently bonded along the channels. The channels may advantageously remain or become visible at least through the topsheet and/or backsheet when the absorbent article is fully loaded with a fluid. This may be obtained by making the channels substantially free of SAP, so they will not swell, and sufficiently large so that they will not close when wet. Furthermore, bonding the core wrap to itself or the topsheet to the backsheet through the channels may be advantageous.

Absorbent cores and/or LMSs without any channels are also within the scope of the present disclosure. These cores may include airfelt-free cores, SAP/pulp cores, pulp cores, or other cores known to those of skill in the art.

### Barrier Leg Cuffs

The absorbent article may comprise a pair of barrier leg cuffs 34. Each barrier leg cuff may be formed by a piece of material which is bonded to the absorbent article so it can extend upwards from the inner surface of the absorbent article and provide improved containment of liquids and other bodily exudates approximately at the junction of the torso and legs of the wearer. The barrier leg cuffs 34 are delimited by a proximal edge 64 joined directly or indirectly to the topsheet 24 and/or the backsheet 25 and a free terminal edge 66, which is intended to contact and form a seal with the wearer's skin. The barrier leg cuffs 34 extend at least partially between the front waist edge 10 and the rear waist edge 12 of the absorbent article on opposite sides of the longitudinal axis 80 and are at least present in the crotch region 7. The barrier leg cuffs 34 may be joined at the proximal edge 64 with the chassis of the absorbent article by a bond 65 which may be made by gluing, fusion bonding, or combination of other suitable bonding processes. The bond 65 at the proximal edge 64 may be continuous or intermittent. The bond 65 closest to the raised section of the leg cuffs 34 delimits the proximal edge 64 of the standing up section of the leg cuffs 34.

The barrier leg cuffs 34 may be integral with the topsheet 24 or the backsheet 25 or may be a separate material joined to the absorbent article's chassis. The material of the barrier leg cuffs 34 may extend through the whole length of the diapers but may be "tack bonded" to the topsheet 24 towards the front waist edge 10 and rear waist edge 12 of the absorbent article so that in these sections the barrier leg cuff material remains flush with the topsheet 24.

Each barrier leg cuff 34 may comprise one, two or more elastic strands or strips of film 35 close to this free terminal edge 66 to provide a better seal.

In addition to the barrier leg cuffs 34, the absorbent article may comprise gasketing cuffs 32, which are joined to the chassis of the absorbent article, in particular to the topsheet 24 and/or the backsheet 25 and are placed externally relative to the barrier leg cuffs 34. The gasketing cuffs 32 may provide a better seal around the thighs of the wearer. Each gasketing leg cuff may comprise one or more elastic strings or elastic elements in the chassis of the absorbent article between the topsheet 24 and backsheet 25 in the area of the leg openings. All or a portion of the barrier leg and/or gasketing cuffs may be treated with a lotion or skin care composition. The barrier leg cuffs may be constructed in a number of different configurations, including those described in U.S. Pat. App. Publ. No. 2012/0277713.

### Front and Rear Ears

In an embodiment, the absorbent article may comprise front ears 46 and rear ears 40. The ears may be an integral part of the chassis, such as formed from the topsheet 24 and/or backsheet 25 as side panel. Alternatively, as represented on Fig. 1, the ears (46, 40) may be separate elements attached by gluing, heat embossing, and/or pressure bonding. The rear ears 40 may be stretchable to facilitate the attachment of the tabs 42 to the landing zone 44 and maintain the taped diapers in place around the wearer's waist. The rear ears 40 may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

### Liquid Management System (LMS)

One function of the LMS 50 is to quickly acquire the fluid and distribute it to the absorbent core 28 in an efficient manner. The LMS 50 may comprise one or more layers, which may form a unitary layer or may remain as discrete layers which may be attached to each other. The LMS 50 may comprise two layers: a distribution layer 54 and an acquisition layer 52 disposed between the absorbent core and the topsheet, but the present disclosure is not limited to such a configuration.

The LMS 50 may comprise SAP as this may slow the acquisition and distribution of the fluid. In other embodiments, the LMS may be substantially free (e.g., 80%, 85%, 90%, 95%, or 99% free of) or completely free of SAP. The LMS may also comprise one or more of a variety of other suitable types of materials, such as opened-cell foam, air-laid fibers, or carded, resin bonded nonwoven materials, for example. Suitable example LMSs are described in WO 2000/59430 (Daley), WO 95/10996 (Richards), U.S. Pat. No. 5,700,254 (McDowall), and WO 02/067809 (Graef), for example.

### Distribution Layer

The LMS 50 may comprise a distribution layer 54. The distribution layer 54 may comprise at least 50% or more by weight of cross-linked cellulose fibers, for example. The cross-linked cellulosic fibers may be crimped, twisted, or curled, or a combination thereof including crimped, twisted, and curled. This type of material is disclosed in U.S. Pat. Publ. No. 2008/0312622 A1 (Hundorf).

### Acquisition Layer

The LMS 50 may alternatively or additionally comprise an acquisition layer 52. The acquisition layer 52 may be disposed, for example, between the distribution layer 54 and the topsheet 24. The acquisition layer 52 may be or may comprise a non-woven material, such as an SMS or SMMS material, comprising a spunbonded, a melt-blown and a further spunbonded layer or alternatively a carded chemical-bonded nonwoven. The acquisition layer 52 may comprise air or wet-laid cellulosic, cross-linked cellulosic, or synthetic fibers, or blends thereof. The acquisition layer 52 may comprise a roll-stock web of synthetic fibers (which may be processed to increase void space, such as by solid state formation), or a combination of synthetic and cellulosic fibers, bonded together to form a highloft material. Alternatively, the acquisition layer 52 may comprise absorbent open cell foam. The nonwoven material may be latex bonded.

### Channels in Liquid Management System

The LMS 50 of the absorbent article 20 may comprise channels that may generally enable better conformation of the absorbent article to the wearer's anatomy, leading to increased freedom-of-movement and reduced gapping. One or more of the channels of the LMS 50 may be configured to work in concert with various channels in the absorbent core 28, as discussed above. Furthermore, channels in the LMS 50 may also provide increased void space to hold and distribute urine, BM or other bodily exudates within the absorbent article, leading to reduced leakage and skin contact. Channels in the LMS 50 may also provide internal serviceable indicia, especially when highlighted via physical differences in texture, color, and/or pattern, to facilitate achieving the correct alignment of the absorbent article on a wearer. Thus, such physical differences may be, for example, visually and/or tactilely noticeable.

Similar to the channels in the absorbent core 28, a channel in the LMS 50 may be any region in a layer, or extending through more than one layer, that has a substantially lower basis weight or thickness than the surrounding material, as set forth in the definition of "channel" above. The channels in the LMS 50 may also serve to reduce the tension forces to enable controlled bending and maintain the LMS 50 in close proximity to the absorbent core 28. Thus, the presence of channels in the LMS 50, which may or may not be aligned with any channels in an underlying absorbent core 28, may generally function as hinges to allow for a more flexible composite structure. In some cases, for example, the channels of the LMS 50 allow for the LMS 50 to move toward the absorbent core 28 in a controlled bending arrangement, thereby limiting the separation between the LMS 50 and the absorbent core 28. Moreover, a channel in the LMS 50 may assist in the routing of fluid or other bodily exudates from one region of the absorbent article 20 to another region of the absorbent article 20. Such routing may desirably improve the overall distribution of fluid through the absorbent article 20 and may lead to increase in comfort, wearability, or longevity of the article.

For multi-layered LMSs, the channels may be present in one or more layers of the LMS 50 and may vary in their dimensions in all three planes of reference. The width of a given channel in the LMS 50 may vary in the longitudinal direction (i.e., in a direction substantially parallel to the longitudinal axis of the absorbent article). A channel may also have a different width, length, and/or volume in front of a lateral axis or lateral separation element of the absorbent article than behind the lateral axis or lateral separation element. The channels of the LMS 50 may have a range of widths, lengths, shapes, volumes, and patterns, similar to the channels described above with regard to the absorbent core 28.

One or more channels in the LMS 50 may at least partially overlap, or fully overlap, a channel in the absorbent core 28, creating a deeper recess in the overlapping regions. For embodiments where the LMS 50 includes more than one layer, the layer closest to the absorbent core 28 may include a channel. One or more layers in the structure, such as the topsheet 24, an acquisition layer 52, distribution layer 54, or other layers, may be bonded to an element of the absorbent core 28 in this region to increase the depth of the combined channel. In an embodiment, the channel in the acquisition layer 52 of the LMS 50 and the channel in the absorbent core 28 are coincident such that the channels are completely overlapping. In another embodiment, channels in the LMS and storage layers have no overlapping area. Other embodiments have a vertical overlap between the channels in the two layers that encompass the intervening range such that they partially overlap.

Referring again to Figs. 1-5, the LMS 50 in the illustrated example is shown defining two channels 49, 49'. The channels 49, 49' are at least partially oriented in the longitudinal direction of the absorbent article 80 (i.e., has a longitudinal vector component). Other channels in the LMS may be at least partially oriented in the lateral direction (i.e., has a lateral vector component), or in any other direction, and the channels in the LMS 50 may be continuous or intermittent. Some channels in the LMS may be round, oblong, square, rectangular, triangular or any other suitable shape. The channels may be formed in various ways. For example, the channels may be formed by zones within the LMS 50 which may be substantially free of, or free of, acquisition or distribution material.

The channels of the LMS 50 may be present at least at the same longitudinal level as the lateral axis 90 in the absorbent article, as represented in Fig. 1 with the two longitudinally extending channels 49, 49'. The channels may also extend from the crotch region 7 or may be present in the front waist region 5 and/or in the rear waist region 6 of the absorbent article. In Fig. 1, the channels 49, 49' are generally coincident with channels 26, 26', with channels 26, 26' having a longer length in the longitudinal direction towards the front waist edge 10 of the absorbent article 20 .

The LMS 50 may define any suitable number of channels, such as at least one or more than two channels. Shorter channels may also be present, for example in the rear waist region 6 or the front waist region 5 of the LMS 50. The channels of the LMS 50 may comprise one or more pairs of channels symmetrically arranged, or otherwise arranged relative to the longitudinal axis 80 and/or the lateral axis 90, or other transverse axis. The channels may extend substantially longitudinally or substantially laterally.

At least some or all of the channels in the LMS 50 may be permanent channels, meaning their integrity is at least partially maintained both in the dry state and in the wet state. Permanent channels may be obtained by provision of one or more adhesive materials, for example, the fibrous layer of adhesive material or construction glue that helps adhere a substrate with an absorbent material within the walls of the channel. Permanent channels may also be formed by bonding the topsheet 24 to the backsheet 25 together through a channel of the LMS 50. Typically, an adhesive may be used to bond the topsheet 24 and the backsheet 25 through the channels, but it is possible to bond via other known processes, such as pressure bonding, ultrasonic bonding, heat bonding, or combination thereof. The topsheet 24 and the backsheet 25 may be continuously bonded or intermittently bonded along or within portions of or all of the channels.

In an embodiment, referring to Fig. 1, the LMS 50 may comprise at least two channels (e.g., 49, 49'). These channels may be free of, or substantially free of (e.g., less than 10%, less than 5%, less than 3%, less than 2%, or less than 1%), non-woven material or cross-linked cellulose fibers and may be at least partially oriented in the longitudinal direction and/or may be at least partially oriented in the lateral direction.

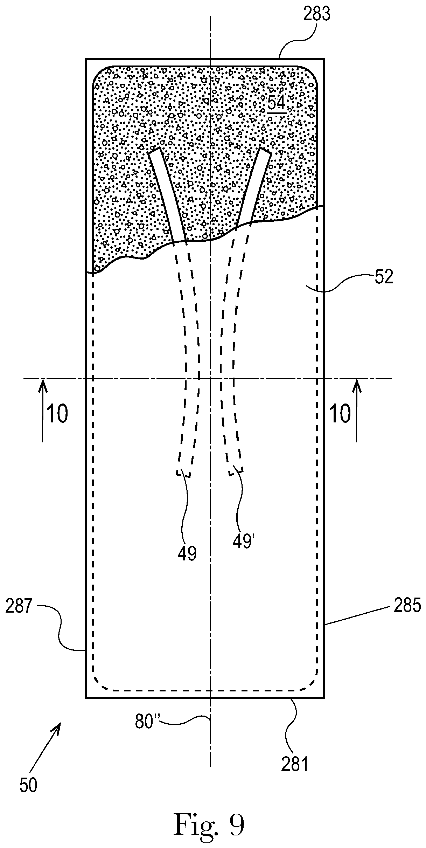

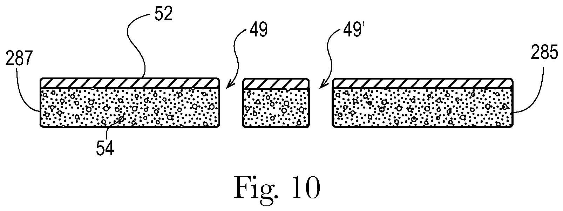

The example LMS 50 of the absorbent article of Figs. 4-5 is shown in isolation in Figs. 9-10 where Fig. 10 is a cross-sectional view of the LMS 50 taken about line 10-10 of Fig. 9. The LMS 50 may comprises a front side 281, a rear side 283, and two longitudinal sides 285, 287 joining the front side 281 and the rear side 283. The LMS 50 may also comprise a generally planar top side and a generally planar bottom side. The front side 281 of the LMS is the side of the LMS intended to be placed towards the front waist edge 10 of the absorbent article. The LMS 50 may have a longitudinal axis 80" corresponding substantially to the longitudinal axis 80 of the absorbent article, as seen from the top in a planar view as in Fig. 1. In the illustrated embodiment, the LMS 50 comprises a distribution layer 54 and an acquisition layer 52 which cooperate to define the channels 49, 49'. In other embodiments, less than all of the layers of the LMS 50 may define the channel such that at least one layer of the LMS 50 is continuous while another layer of the LMS 50 is discontinuous.

While portions of the channels 26, 26' of the absorbent core 28 and the channels 49, 49' of the LMS 50 shown in Figs. 1-10 are generally aligned, this disclosure is not so limited. In fact, as is to be appreciated, particular arrangements of the channels in an LMS 50 and/or an absorbent core 28 may vary.

### Substantially Laterally-Extending Separation Element

A wearer-facing surface, or topsheet, of an absorbent article may have a visual front portion and a visual back portion. The visual front portion and the visual back portion may be separated by a substantially laterally-extending separation element 100. The term "substantially laterally" means within +/- 15 degrees from a direction parallel to the lateral axis. The substantially laterally-extending separation element 100 may be, for example, a graphical indicia printed on the topsheet of the absorbent article, or other layer of the absorbent article (e.g., LMS 50), that is visible through the topsheet. The substantially laterally-extending separation element 100 may also be a portion of a tinted layer that is visible through the wearer-facing surface of the topsheet or the end of an underlying layer that has a different color than the topsheet. Alternatively or additionally, the visual front portion may be visually distinct from the visual back portion based on a color difference and/or a printed pattern difference. Such visual separation between the visual front portion and the visual back portion may help for proper alignment of the absorbent article during its application and help the appearance of separate zones configured for urine management and, separately, for BM management.

The substantially laterally-extending separation element 100, in various forms, may comprise a structural separator that is located in the region of the absorbent article generally corresponding to the perineal region of the wearer (i.e., disposed between the urethra and the anus). The structural separator may, for example, prevent, or at least somewhat inhibit, the surface migration of urine to the back of the absorbent article and BM to the front of the absorbent article. A structural separator may include any three-dimensional feature or component that functions as a transverse or laterally extending barrier ("TVB"), such as one or more projections above the wearer-facing surface of the absorbent article, recesses below the plane of the wearer-facing surface, and combinations thereof. One example includes a substantially laterally-oriented web or sheet that is attached to the wearer-facing surface of the absorbent article and that is attached on its ends to the barrier leg cuffs. Attachment to the barrier leg cuffs and the wearer-facing surface may provide a "seal" created by the TVB with respect to the front and back regions of the absorbent article to prevent, or at least inhibit, bodily exudates flow between the regions.

The structural separator may be rectangular or square when laid out flat in a relaxed, contracted state onto an even horizontal surface. The structural separator may also be trapezoidal when laid out flat in a relaxed, contracted state onto an even horizontal surface. The structural separator may be hydrophobic (e.g., it may be hydrophilic and made hydrophobic with a hydrophobic coating, for example a wax or a hydrophobic surface coating comprising one or more silicone polymers or fluorinated polymers.) The structural separator may have an elastic behavior such that it can be significantly elastically extensible in a lateral, transverse direction or other direction. The structural separator may have a certain tension during wear of the absorbent article to ensure that the structural separator forms an effective separator (barrier) with a Z-direction dimension, to avoid, or at least inhibit, migration of feces from the back to the front of the structural separator. Other structural separators may include raised or thicker portions of the topsheet, elements of the LMS or absorbent core, separately applied elements, or holes or depressions in one or more of the absorbent core elements or LMS.

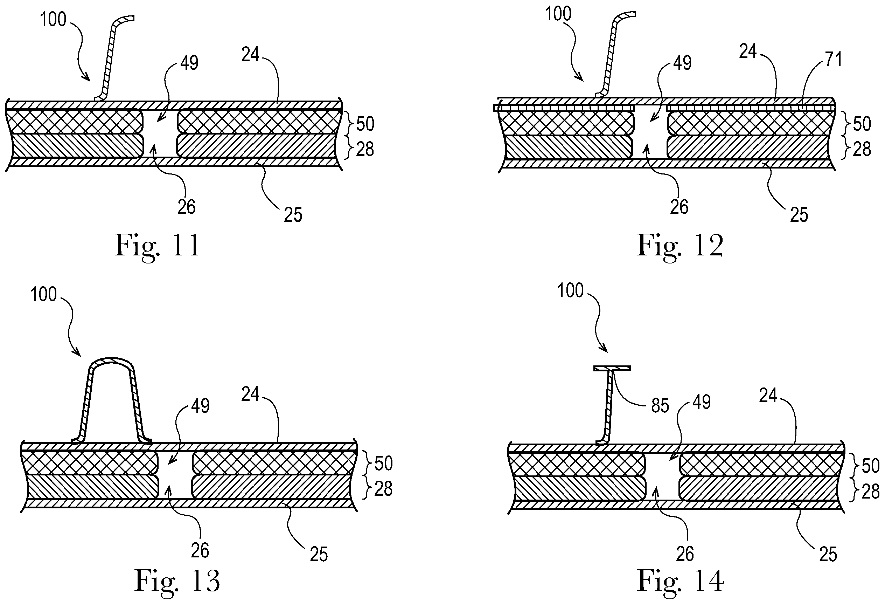

Further to the above, the structural separator may have any suitable structure and may be a ridge, bump, and/or flap, for example. Some example cross-sectional views of substantially laterally-extending separation elements 100 configurations in the form of structural separators are illustrated in Figs. 11-14. Any other suitable structural separators are within the scope of the present disclosure. The structural separator may be placed along a lateral axis of an absorbent article or may be positioned at an angle that is oblique to the lateral axis. The structural separator may also be placed in other locations that are not along the lateral axis (e.g., location in front of or behind the lateral axis). One or more structural separators may be incorporated into absorbent articles having a variety of configurations.

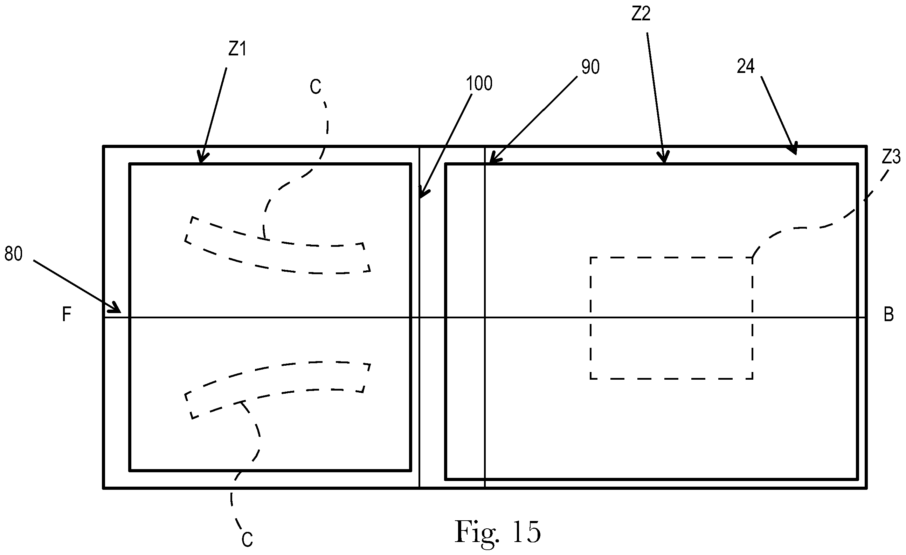

Substrates, such as topsheets and/or LMS, for example, may have one or more zones in different regions or areas of the substrates. Some example configurations of zones in a topsheet and/or an LMS are illustrated in Figs. 15-32. In various figures, the zones are labeled Z1-Z6, although it is within the scope of the present disclosure to have any number of zones having any number of configurations, shapes, and/or sizes. While the topsheet 24 is illustrated as rectangular in Figs. 15-19, it is within the scope of the present disclosure to have the topsheet be any suitable shape for absorbent articles, such as an hourglass shape, for example. The wearer-facing surface of the topsheet 24 is facing the viewer in Figs. 15-32. Throughout Figs. 15-32, if applicable, element 100 is the substantially laterally-extending separation element, element 90 is a lateral axis of the absorbent article, element 80 is a longitudinal axis of the absorbent article, F stands for the front of the absorbent article, and B stands for the back of the absorbent article. The lateral axis and the longitudinal axis will be apparent in all of Figs. 15-32, although not illustrated in each figure.

For all of the zones discussed below, it will be understood that although a particular treatment may be specified for a specific zone, the zone may also include one or more other treatments or flow control materials. For example, if a certain morphological treatment is specified in a certain zone, another morphological treatment and/or another chemical or geometric treatment may also be provided in the same zone, or a portion thereof, although not specifically stated for each zone in the examples below. The zones may also have any suitable size and/or shape and are not limited by the examples illustrated below.

Referring to Fig. 15, the topsheet 24 comprises a first zone Z1 in the front of the absorbent article and a second zone Z2 at least partially in the back of the absorbent article. Zone Z1 is positioned on a first side of the substantially laterally-extending separation element 100 (e.g., a recess, a raised portion, a structural separator, a printed line or graphic) and zone Z2 is positioned on a second side of the substantially laterally-extending separation element 100. Zones Z1 and Z2 may both overlap the longitudinal axis 80 and only zone Z2 may overlap the lateral axis 90. In other instances, only zone Z1 may overlap the lateral axis 90. In the example of Fig. 15, the substantially laterally-extending separation element 100 may be optional. The first and second zones Z1 and Z2 may each comprise any number of geometric, morphological, chemical treatments and/or flow control materials, or one of the zones Z1 or Z2, or portions thereof, may not comprise a treatment or a flow control material at all. Either of, or both of, zones Z1 or Z2 may also comprise apertures.

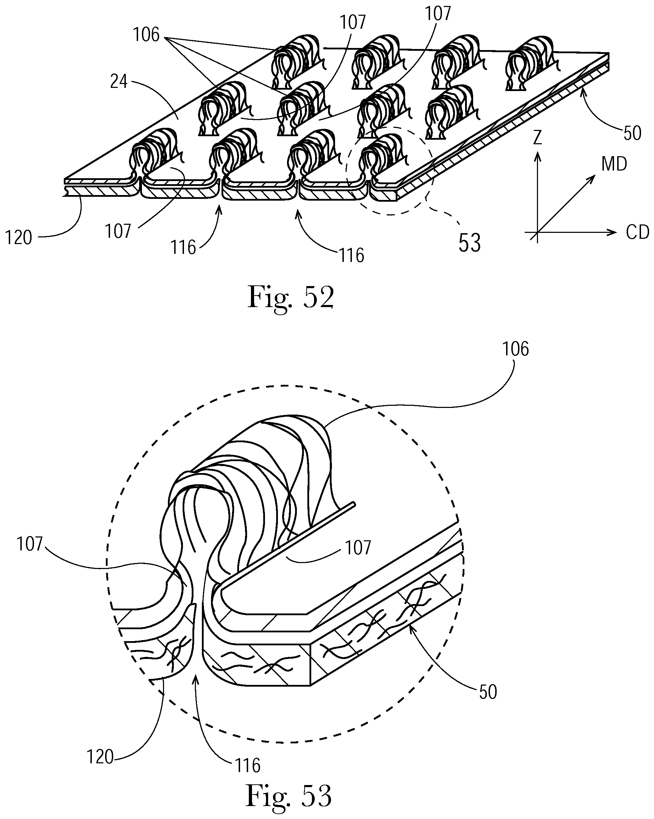

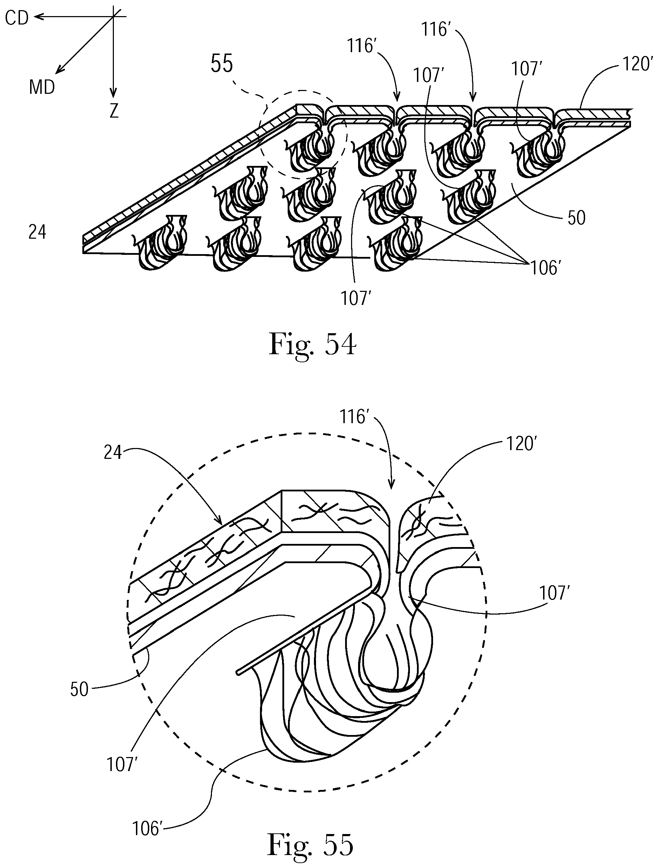

Referring again to Fig. 15, the substantially laterally-extending separation element 100 may define a visual front portion and a visual back portion on either side thereof. The visual front portion comprises zone Z1 and the visual back portion comprises zone Z2. Zone Z1 may comprise a first geometric treatment and zone Z2 may comprise a second geometric treatment. The first and second geometric treatments may be the same or different. The first and second geometric treatments may comprise elements that differ in depth, length, frequency, size, shape, pattern, dimensions, and/or structure. Either of the first and second geometric treatments may comprise apertures or morphological treatments (see e.g., Figs. 52 and 54). The apertures may be different or the same in either of the treatments. The morphological treatments may be the same or different. The topsheet 24 may comprise a third geometric treatment Z3 (illustrated in dash) in either of the zones Z1 or Z2. A portion of an absorbent core and/or a liquid management system may have one or more channels, C, defined therein. A portion of the zones Z1 and/or Z2 may or may not overlap at least a portion of, or all of the channel(s).

Referring again to Fig. 15, the topsheet 24 may comprise zone Z1 at least partially in the front region (i.e., first side of the lateral axis 90) and a zone Z2 at least partially in the back region (i.e., second side of the lateral axis 90). Zone Z1 comprises a first morphological treatment configured for urine handling and zone Z2 comprises a second morphological treatment configured for BM handling. A pattern of the first morphological treatment in zone Z1 is nonsymmetrical to a pattern of the second morphological treatment in zone Z2 about the lateral axis 90. Either of the zones Z1 and Z2 may have apertures defined therein. The apertures may have any suitable effective aperture areas and the topsheet 24 may have any suitable % open areas in the various zones. The first and second morphological treatments may be those illustrated in Figs. 52 or 54 hereof, for example. Either or both of the zones Z1 or Z2 may comprise a chemical treatment or a third morphological treatment. An area of zone Z1 may be smaller or larger than an area of zone Z2. Zone Z1 may have a dimension of at least 30mm or at least 40mm measured in a direction parallel to the lateral axis 90 and zone Z2 may have a dimension of at least 30mm or at least 40mm measured in the direction parallel to the lateral axis 90. Although not illustrated in Fig. 15, the absorbent article may comprise barrier leg cuffs and a waist edge.

Still referring to Fig. 15, zone Z1 may comprise a substantially transferrable chemical treatment and zone Z2 may comprise a substantially transferrable chemical treatment. The substantially transferrable chemical treatments may be the same or different. A basis weight of the substantially transferrable chemical treatment in zone Z2 may be greater than, less than, substantially the same as, or the same as, the substantially transferrable chemical treatment in zone Z1. The substantially transferrable chemical treatment may comprise a skin care composition or a BM anti-stick lotion. At least one of the zones Z1 or Z2 may also comprise a substantially durable chemical treatment. The substantially durable chemical treatment may comprise a pigment or an ink, for example. The zones Z1 and Z2 (or Z3) also comprise one or more morphologic and may comprise one or more geometric treatments. The chemical treatments may or may not overlap with the morphological or geometric treatments. Zone Z1 or Z2 may comprise at least a third chemical treatment (e.g., Z3 in Fig. 15) that may either be substantially transferrable or substantially durable. Any of the chemical treatments may overlap at least a portion of, or all of, one or more channels C. In other instances, the various chemical treatments may not overlap any of the channels C. The substantially transferrable chemical treatment in zone Z1 may be hydrophobic and the substantially transferrable chemical treatment in zone Z2 may have a different hydrophilicity. The substantially transferrable chemical treatment in zone Z1 may be more hydrophilic or more hydrophobic than the substantially transferrable chemical treatment in zone Z2. In other instance, the substantially transferrable chemical treatment in zone Z2 may be hydrophobic or hydrophilic. The substantially transferrable chemical treatments may overlap or not overlap with one or more morphological treatments (see e.g., Figs. 52 and 54 hereof) or one or more geometrical treatments.

Again referring to Fig. 15, zone Z1 may comprise a substantially durable chemical treatment and zone Z2 may comprise a substantially durable chemical treatment. The substantially durable chemical treatments may be the same or different. A basis weight of the substantially durable chemical treatment in zone Z2 may be greater than, less than, or the same as, the substantially durable chemical treatment in zone Z1. One both of the zones Z1 or Z2 may also comprise a substantially transferrable chemical treatment.

In other instances, one of the zones Z1 or Z2 may comprise a substantially durable chemical treatment and the other of the zones may comprise a substantially transferrable chemical treatment.

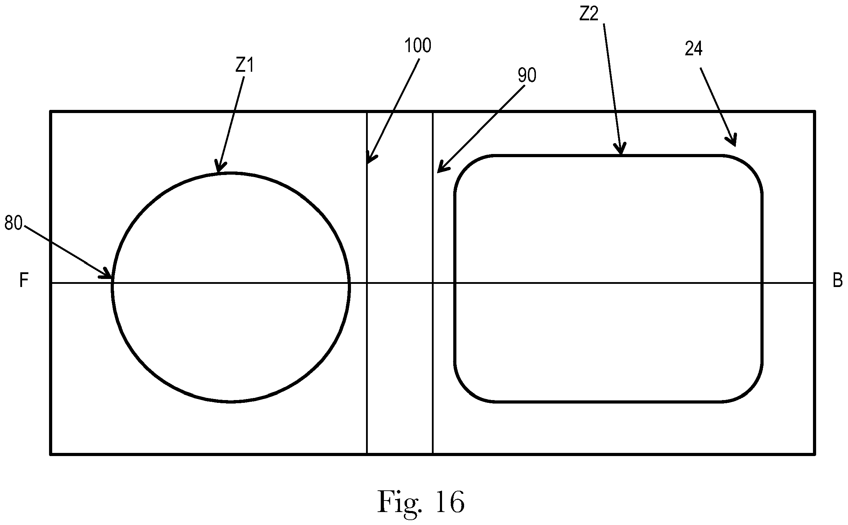

Referring to Fig. 16, the topsheet 24 comprises a first zone Z1 in the front of the absorbent article and a second zone Z2 in the back of the absorbent article. Zone Z1 is positioned on a first side of the substantially laterally-extending separation element 100 and zone Z2 is positioned on a second side of the substantially laterally-extending separation element 100, In the example of Fig. 16, the substantially laterally-extending separation element 100 may be optional. Zones Z1 and Z2 may both overlap the longitudinal axis 80 and neither of the zones Z1 and Z2 may overlap the lateral axis 90. In other instances, only zone Z1 or only zone Z2 may overlap the lateral axis 90. The first and second zones Z1 and Z2 may comprise any number of geometric, morphological, chemical treatments and/or flow control materials, or one of the zones Z1 or Z2, or portions thereof, may not comprise a treatment or a flow control material at all. Either of zones Z1 or Z2 may also comprise apertures.

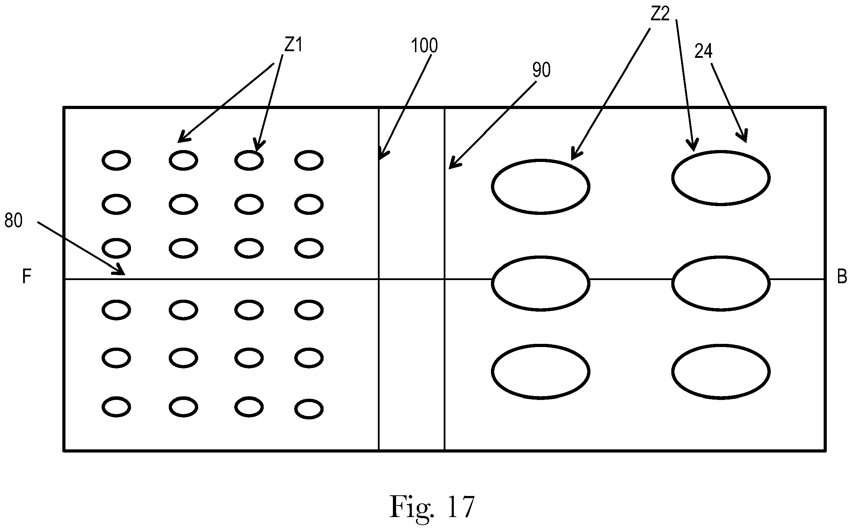

Referring to Fig. 17, the topsheet 24 comprises a first zone Z1 comprising a plurality of elements in the front portion of the absorbent article and a second zone Z2 comprising a plurality of elements in the back portion of the absorbent article. Zone Z1 is positioned on a first side of the substantially laterally-extending separation element 100 and zone Z2 is positioned on a second side of the substantially laterally-extending separation element 100. In the example of Fig. 17, the substantially laterally-extending separation element 100 may be optional. The elements of the first zone Z1 do not overlap the lateral axis 90 or the longitudinal axis 80 and some of the elements of the second zone Z2 overlap only the longitudinal axis. It is within the scope of the present disclosure to have at least some of the elements of either zone overlap or not overlap one of the axes 80 or 90. The elements that make up zones Z1 and Z2 may comprise a plurality of apertures. The apertures in zone Z1 may be smaller than the apertures in zone Z2 or *vice versa.* The smaller apertures in zone Z1 may be configured for urine management, while the larger apertures in zone Z2 may be configured for BM management. Example effective aperture areas and example % effective open areas are described herein. While the apertures of the first and second zones Z1 and Z2 are illustrated as generally ovate, they may have any suitable size, shape, and/or pattern. In other instances, the elements that make up zones Z1 and/or Z2 may not form apertures and instead they may comprise one or more other treatments and/or flow control materials.

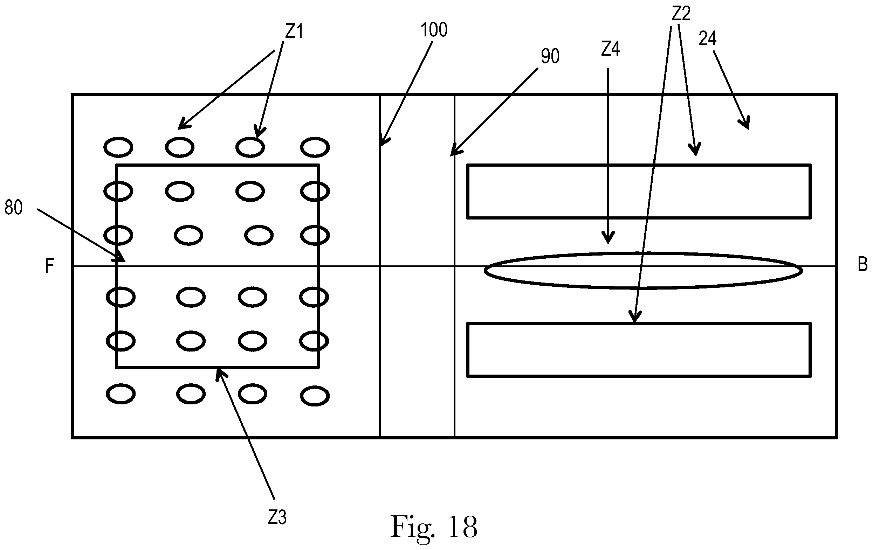

Referring to Fig. 18, the topsheet 24 has a first zone Z1 comprising a plurality of elements in the front of the absorbent article and a second zone Z2 comprising a plurality of elements in the back portion of the absorbent article. The topsheet 24 also has a third zone Z3 in the front of the absorbent article and a fourth zone Z4 in the back of the absorbent article. Zones Z1 and Z3 are positioned on a first side of the substantially laterally-extending separation element 100 and a first side of the lateral axis 90 and zones Z2 and Z4 are positioned on a second side of the substantially laterally-extending separation element 100 and on a second side of the lateral axis 90. The elements of the first and second zones Z1 and Z2 do not overlap the lateral axis 90 or the longitudinal axis 80 and the third and fourth zones Z3 and Z4 do not overlap the lateral axis 90, but do overlap the longitudinal axis. It is within the scope of the present disclosure to have any of the zones (or elements forming the zones) overlap or not overlap one of the axes 80 or 90. Zone Z1 may comprise a plurality of elements that are apertures or that comprise another treatment or flow control material. Zone Z4 may comprise one large aperture, embossment, or other treatment, such as a chemical treatment or a flow control material, for example. The apertures in zone Z1 may be smaller than the aperture of zone Z4. The smaller apertures in zone Z1 may be configured for urine management, while the large aperture in zone Z4 may be configured for BM management. Zone Z3 overlaps at a least a portion of zone Z1 while zones Z2 and Z4 do not overlap with any other zone. It is within the scope of the present disclosure to have any of the various zones in any of the zonal examples to overlap or not overlap each other.

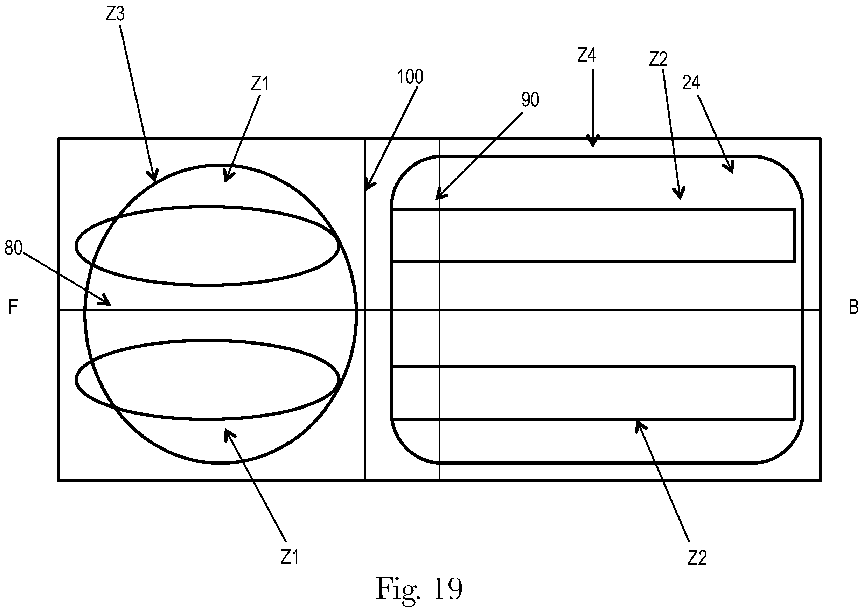

Referring to Fig. 19, the topsheet 24 comprises a first zone Z1 comprising a plurality of elements in the front of the absorbent article and a second zone Z2 comprising a plurality of elements at least partially in the back of the absorbent article. The topsheet 24 comprises a third zone Z3 in the front of the absorbent article and a fourth zone Z4 at least partially in the back of the absorbent article. Zones Z1 and Z3 are positioned on a first side of the substantially laterally-extending separation element 100 and zones Z2 and Z4 are positioned on a second side of the substantially laterally-extending separation element 100. The first and second zones Z1 and Z2 do not overlap the longitudinal axis 80 and the second and fourth zones Z2 and Z4 overlap the lateral axis 90. The fourth zone Z4 overlaps the lateral axis 90 and the longitudinal axis 80. It is within the scope of the present disclosure to have any of the zones overlap or not overlap one of the axes 80 or 90. The elements of zone Z1 may be apertures, or not. Zone Z3 overlaps at a least a portion of zone Z1 and zone Z4 overlaps at least a portion of zone Z2.

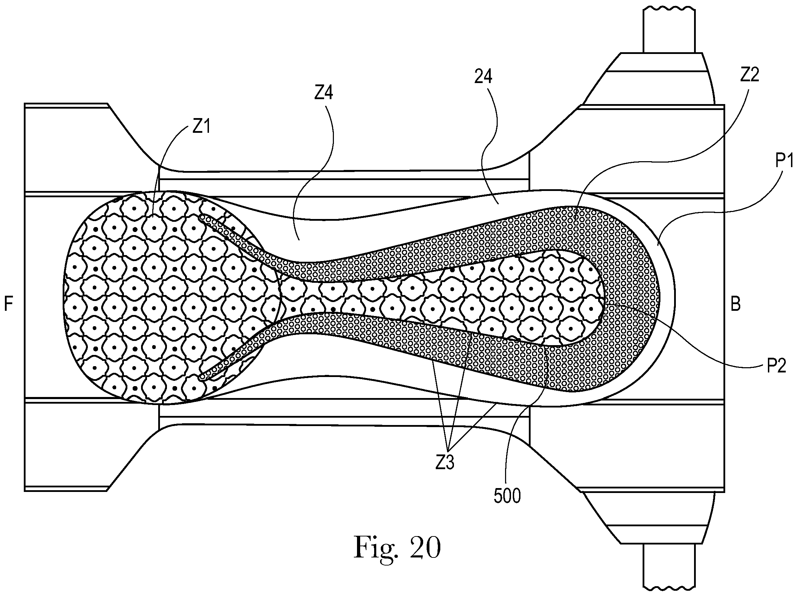

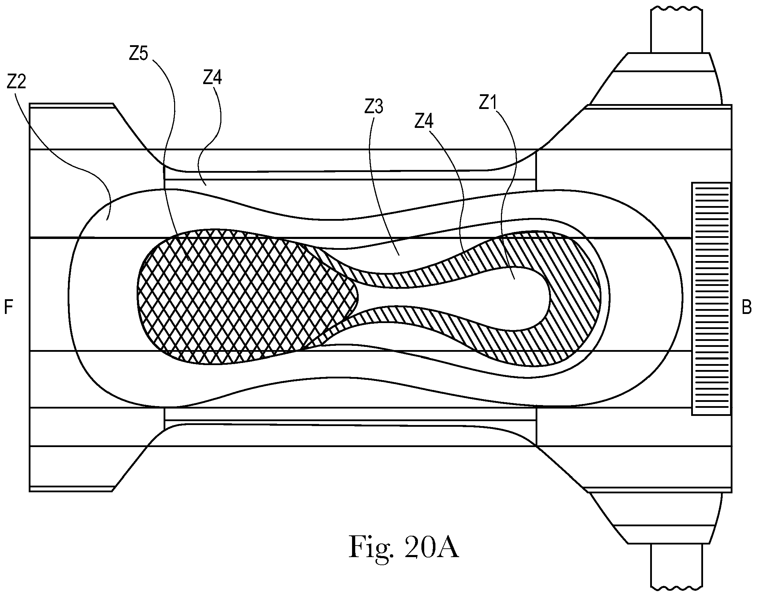

Referring to Fig. 20, a topsheet 24 of an absorbent article is illustrated with four zones, Z1-Z4. The zones Z1-Z4 may comprise any number of geometric, morphological, and/or chemical treatments or flow control materials, or one or more of the zones, or portions thereof, may not comprise a treatment or a flow control material at all. Zone Z3 may comprise deep emboss lines. Zone Z1 may comprise a morphological treatment (e.g., see Figs. 52 or 54) and/or a printed pattern. Zone Z2 may comprise a morphological treatment(e.g., see Figs. 52 or 54). Zone 4 may not comprise a treatment or may comprise a flow control material. Zone Z3 may also comprise a flow control material at least partially forming an enclosed perimeter over at least a portion of an absorbent core of the absorbent article.

Referring to Fig. 20A, a topsheet 24 of an absorbent article is illustrated with five zones, Z1-Z5. Zones Z1 and Z5 may comprise a morphological treatment (e.g., see Figs. 52 or 54). Zone Z2 may comprise a flow control material for urine, for example. The flow control material may form a fully enclosed perimeter within the topsheet 24. Zone Z3 may comprise a morphological treatment (e.g., Figs. 52 or 54), a printed pattern, and/or a flow control material. Zone Z4 may comprise a flow control material for BM, for example that may form a partially enclosed perimeter within the topsheet 24. Any of the zones may also comprise one or more additional treatments and/or flow control materials.

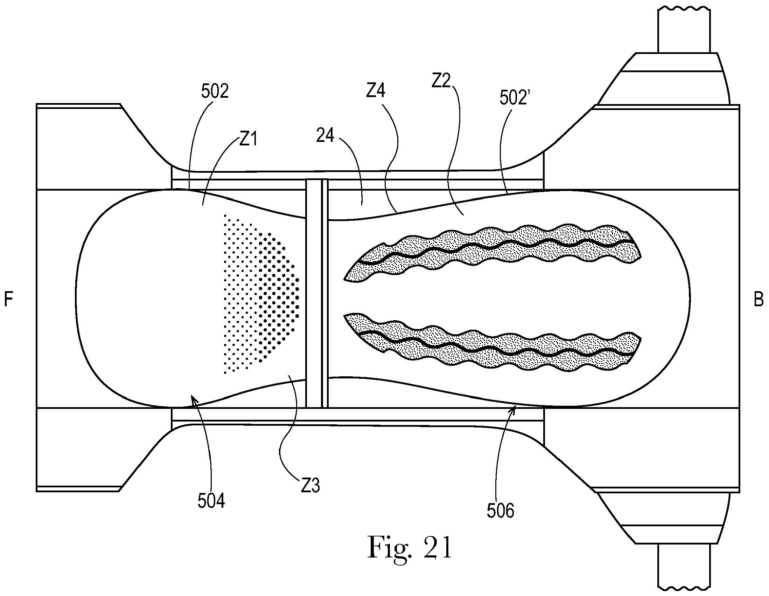

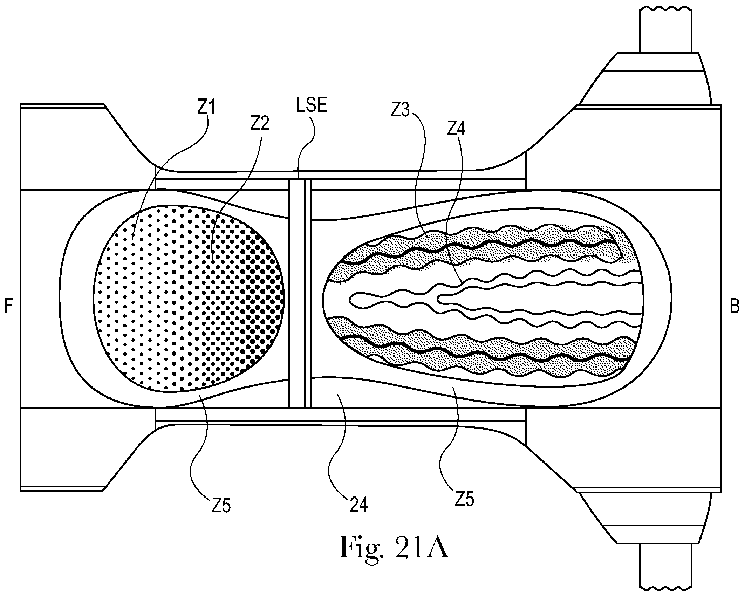

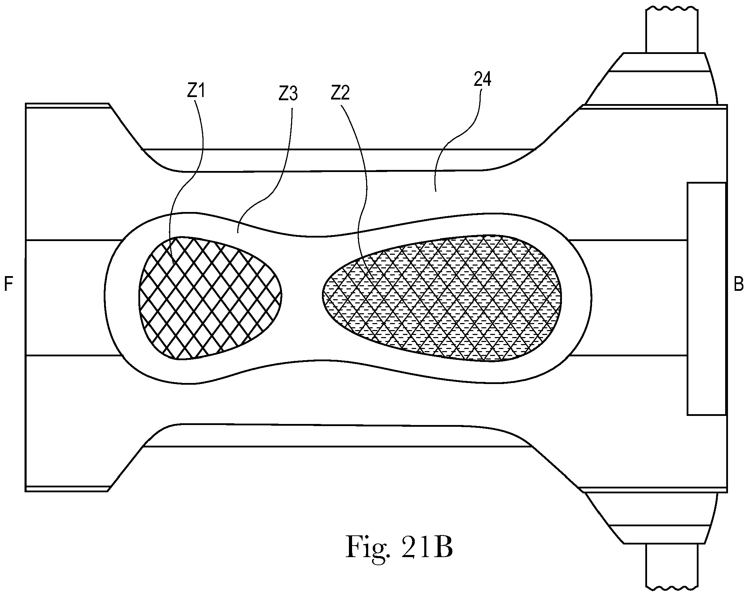

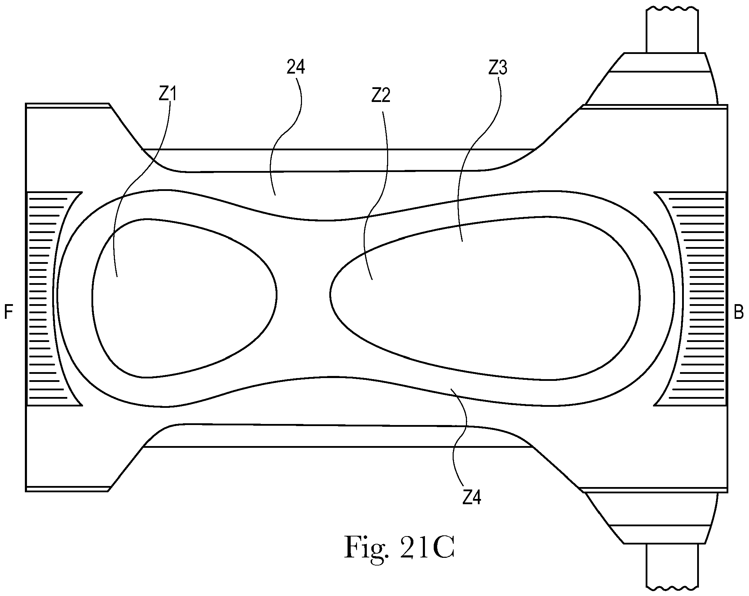

Referring to Fig. 21, an absorbent article comprising a topsheet 24 is illustrated with four zones Z1-Z4. Zone Z1 may comprise a chemical treatment of printed dots and/or a geometric treatment of apertures. Zone Z2 may comprise a chemical treatment of printed diamonds and/or a morphological treatment of puckering. Zone Z2 may also comprise apertures. Zone Z3 may or may not have a treatment. Zone Z4 may comprise a flow control material forming a fully enclosed, continuous perimeter in the topsheet and over a portion of the absorbent core. Although not illustrated, the enclosed perimeter of Zone Z4 may also be discontinuous or may only form an at least mostly enclosed perimeter over a portion of the absorbent core.

Referring to Fig. 21A, an absorbent article comprising a topsheet 24 is illustrated with five zones Z1-Z5. Zone Z1 may comprise a hydrophobic skin care composition. Zone Z2 may comprise a morphological treatment (see e.g., Figs. 52 or 54). Zone Z3 may comprise a morphological treatment (see e.g., Figs. 52 or 54). Zone Z4 may comprise a skin care composition that is more or less hydrophobic than the skin care composition of Zone Z1. Zone Z5 may comprise an embossed figure 8-like shape and/or a flow control material forming an enclosed, continuous perimeter. The continuous perimeter may surround one or more urine and/or BM insult zones (e.g., Zones Z1 and Z3). Although not illustrated, the perimeter may be discontinuous and at least mostly enclosed. Any of the zones may also comprise one or more additional treatments and/or flow control materials. The absorbent article may optionally comprise a lateral separation element LSE.

Referring to Fig. 21B , an absorbent article comprising a topsheet 24 is illustrated with three zones Z1-Z3. Zone Z1 may comprise a morphological treatment (see e.g., Figs. 52 or 54) to provide absorbency and dryness during and after a urination event. Zone Z2 may comprise a morphological treatment (see e.g., Figs. 52 or 54) to at least inhibit BM spreading. Zone Z3 may comprise a flow control material. The flow control material may form an enclosed perimeter around two bodily exudate receiving zones. The flow control material may be continuous or discontinuous. Any of the zones may also comprise one or more additional treatments and/or flow control materials. The absorbent article may optionally comprise a lateral separation element (not illustrated).

Referring to Fig. 21C , an absorbent article comprising a topsheet 24 is illustrated with four zones Z1-Z4. Zone Z1 may comprise a geometric treatment comprising apertures or may comprise a morphological treatment. The % effective open area of Zone Z1 may be about 5% to about 20% or about 10%. Zone Z2 may comprise a geometric treatment comprising apertures or may comprise a morphological treatment. The % effective open area of Zone Z2 may be about 15% to about 50% or about 30%. Zone Z3 may comprise an anti-stick lotion. Zone Z4 may comprise a morphological treatment and/or may comprise a flow control material. Any of the zones may also comprise one or more additional treatments and/or flow control materials. The absorbent article may optionally comprise a lateral separation element (not illustrated).

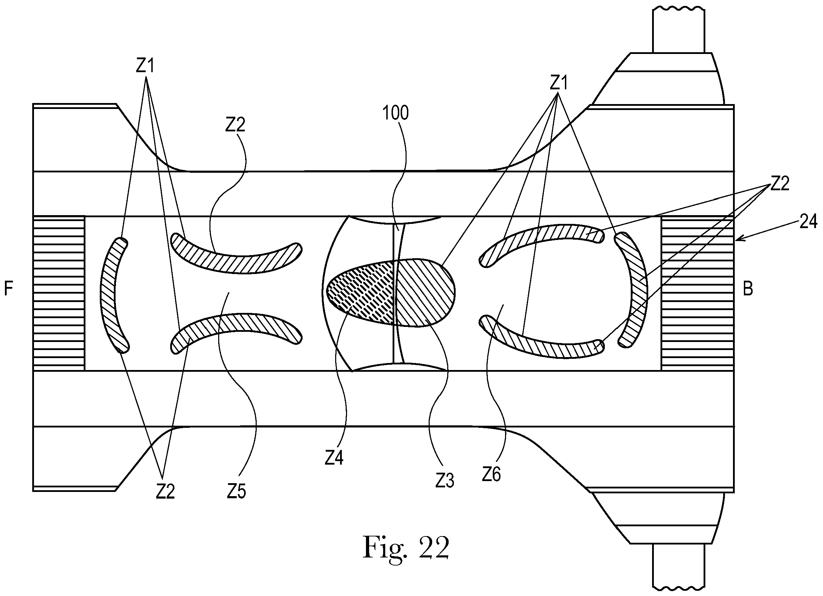

Referring to Fig. 22, an absorbent article comprising a topsheet 24 is illustrated with six zones. Zone Z1 may comprise deep embossed lines, printed lines, flow control materials, and/or other treatments. Zones Z2 to Z5 may each comprise any number of chemical, geometric, and/or morphological treatments or no treatments at all. Some of the zones may form arcuate shapes.

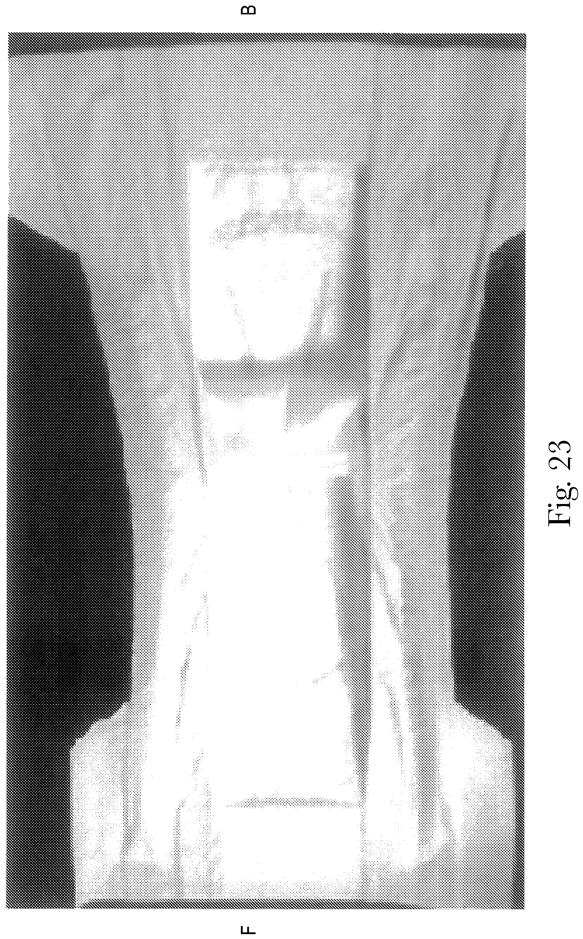

Fig. 23 is a photograph of an actual absorbent article comprising a topsheet similar to the example topsheet of Fig. 22.

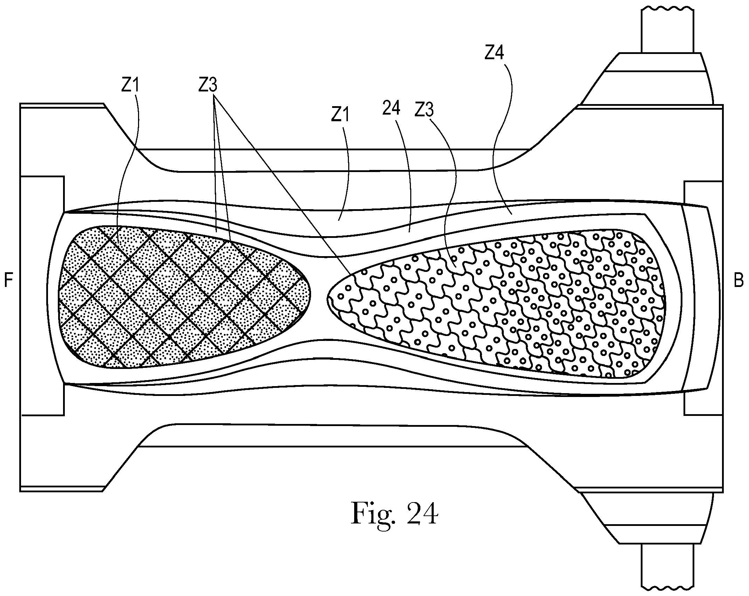

Referring to Fig. 24, an absorbent article comprising a topsheet 24 is illustrated with four zones. Zone Z1 may comprise a flow control material. Zone Z2 may comprise an embossing pattern or a printed pattern and/or one or more geometrical treatments. Zone Z3 may comprise embossing and/or a flow control material. Zone Z4 may have one or more treatments and/or flow control materials, or not. Any of zones Z1-Z4 may have one or more chemical treatments or other treatments.

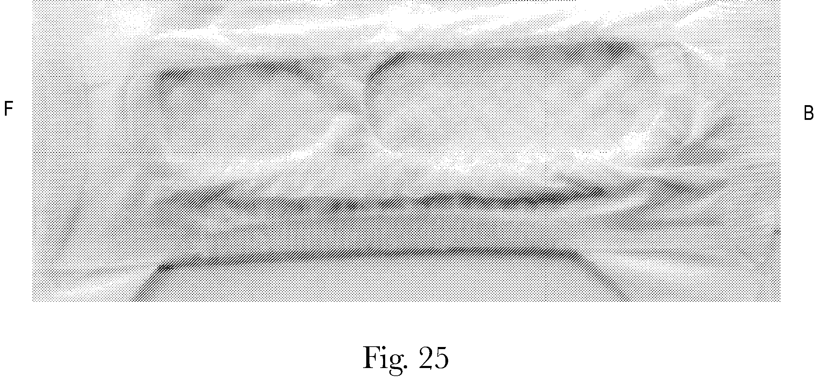

Fig. 25 is a photograph of an actual absorbent article comprising a zoned topsheet. The topsheet has at least two zones of embossing and may have other treatments.

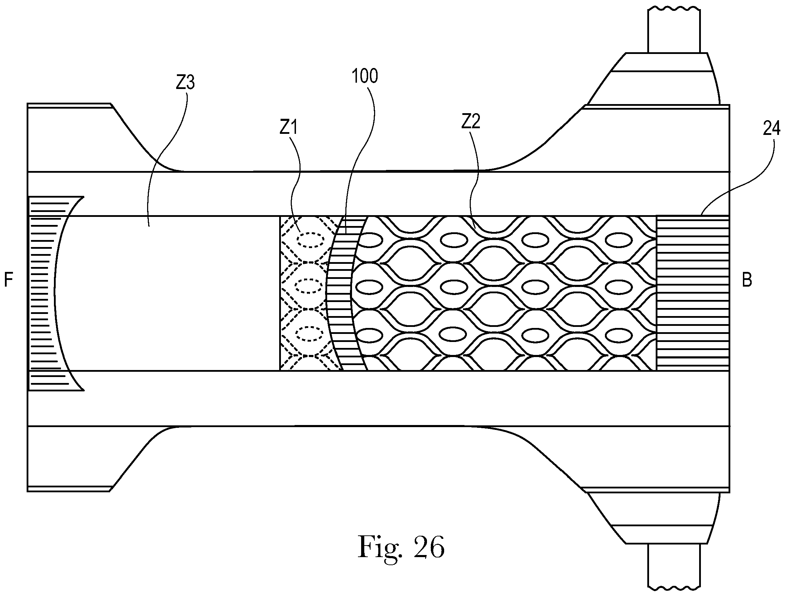

Referring to Fig. 26, an absorbent article comprising a topsheet 24 is illustrated with three zones. Zone Z1 may comprise embossing or other treatment having a first pattern, zone Z2 may comprise embossing or other treatment having a different pattern, and zone Z3 may comprise one or more treatments or may not. The article may also comprise a substantially laterally extending separation element.

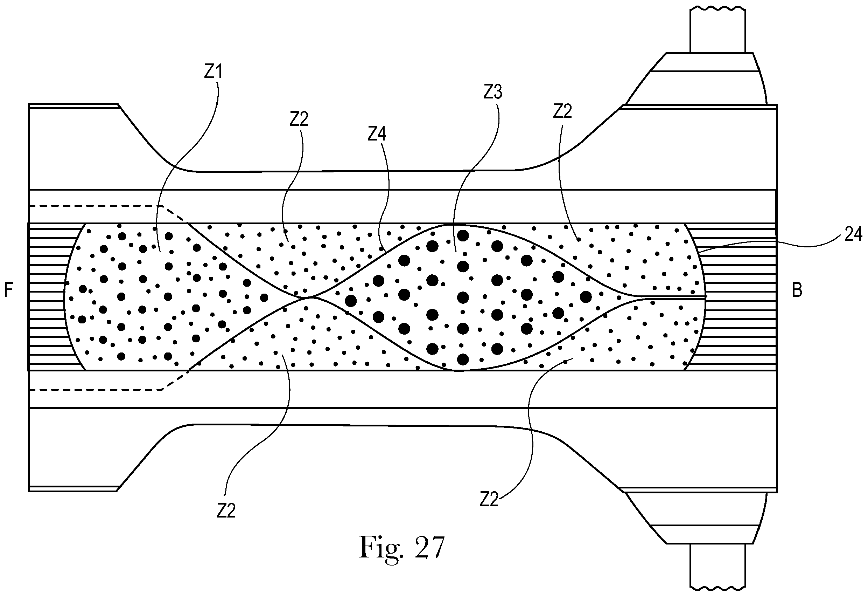

Referring to Fig. 27, an absorbent article comprising a topsheet 24 is illustrated with four zones. Zones Z1 and Z2 may each comprise a chemical treatment of small printed dots or may each comprise apertures. The dots and/or the apertures may be the same size or different sizes. The treatments in zones Z1 and Z2 may be same or different. Zone Z3 may comprise a chemical treatment of larger printed dots or apertures than the dots or apertures in zones Z1 and Z2. Zone Z4 may comprise fold lines, embossed lines, printed lines, a flow control material, or another treatment. The fold lines may be formed from portions of the topsheet or may be separate materials attached to the topsheet.

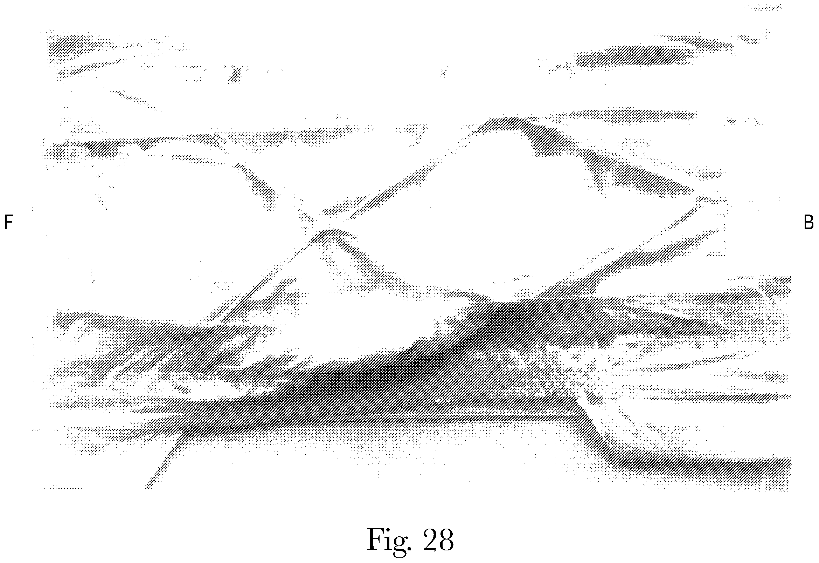

Fig. 28 is a photograph of an actual absorbent article comprising a topsheet somewhat similar to the example topsheet of Fig. 27.

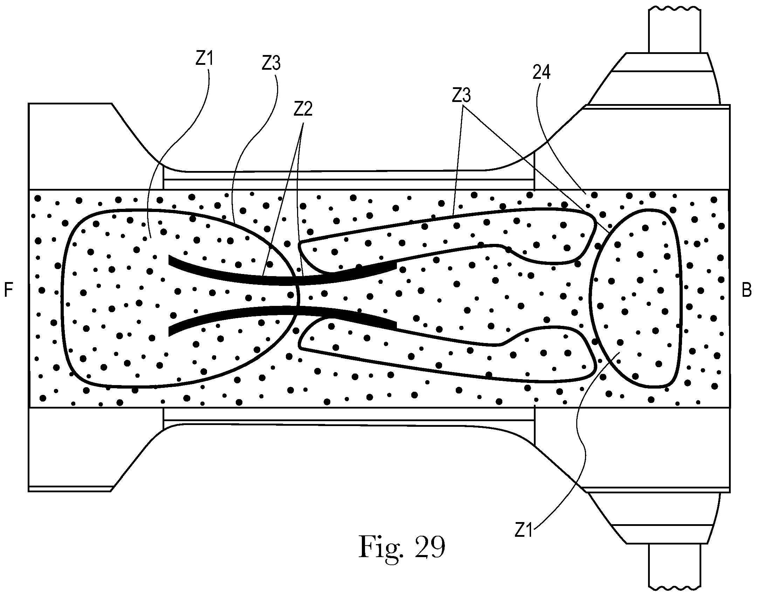

Referring to Fig. 29, an absorbent article comprising a topsheet 24 is illustrated with three zones. Zone Z1 may comprise a chemical treatment of small printed dots or may comprise apertures. Zone Z2 may comprise embossed lines, printed, lines or another treatment. Zone Z3 may comprise embossed lines, printed lines, or another treatment. Zone Z4 may comprise small printed dots or apertures.

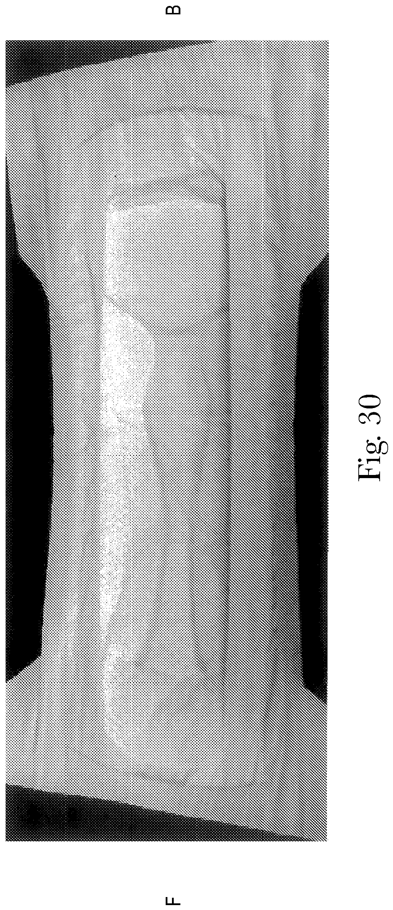

Fig 30 is a photograph of an actual absorbent article comprising a topsheet similar to the example topsheet of Fig. 29.

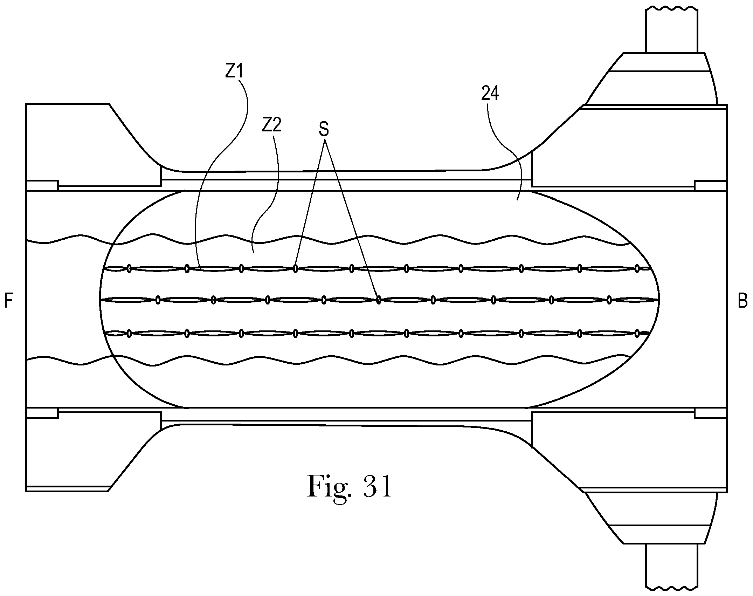

Referring to Fig. 31, an absorbent article comprising a topsheet 24 is illustrated with two zones. Zone Z1 may comprise slits. Zone Z2 may comprise folds, embossed lines, printed lines or no treatments. Either or both of the zones may also comprise a chemical treatment, such as a print. The pleats may be formed in a two layer topsheet. A first layer may be slit using a rotary die before or after the first layer is attached to a second layer. The first layer may be high pressure bonded to the second layer incrementally along the slits. The bond sites, S, may encompass a portion of the first layer on either side of the slits and attach the portions to the second layer.

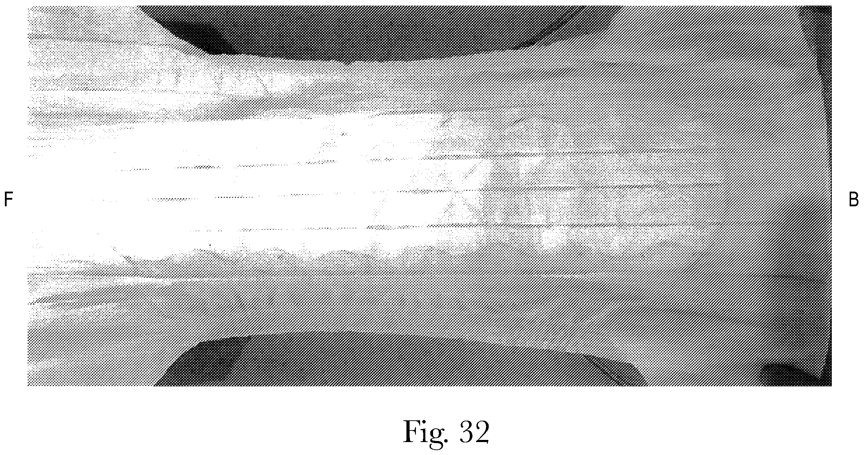

Fig. 32 is a photograph of an actual absorbent article comprising a topsheet similar to the example topsheet of Fig. 31.

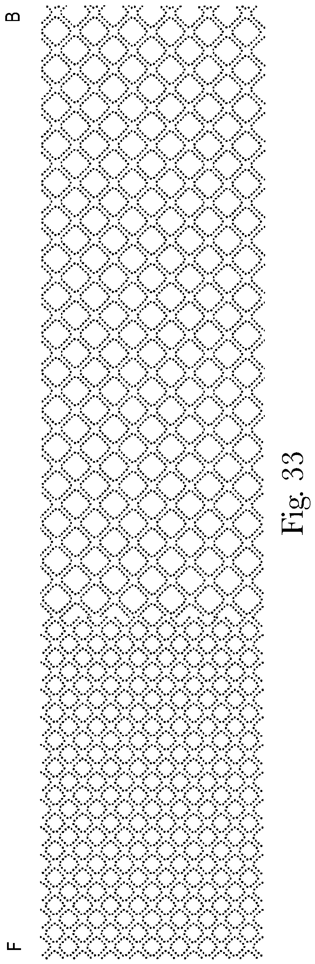

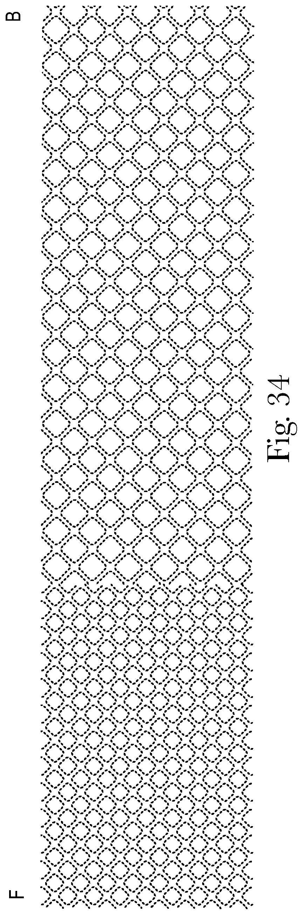

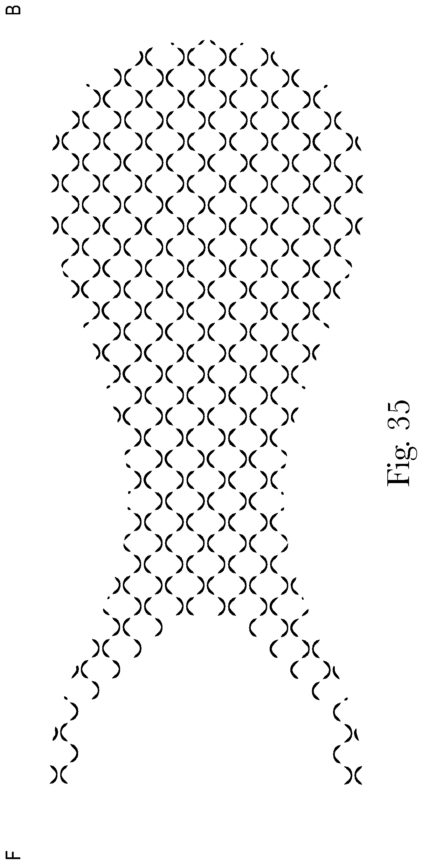

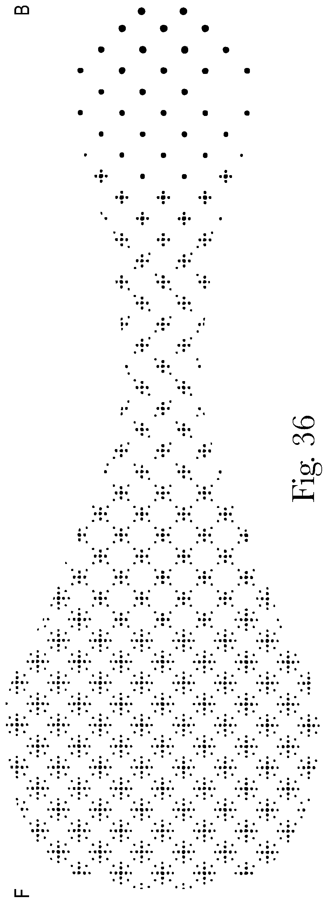

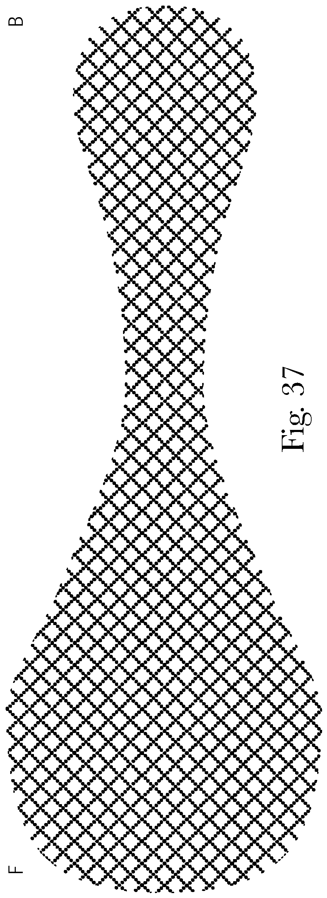

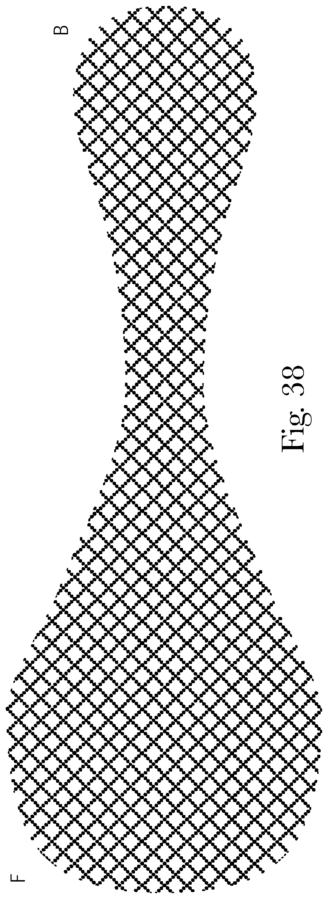

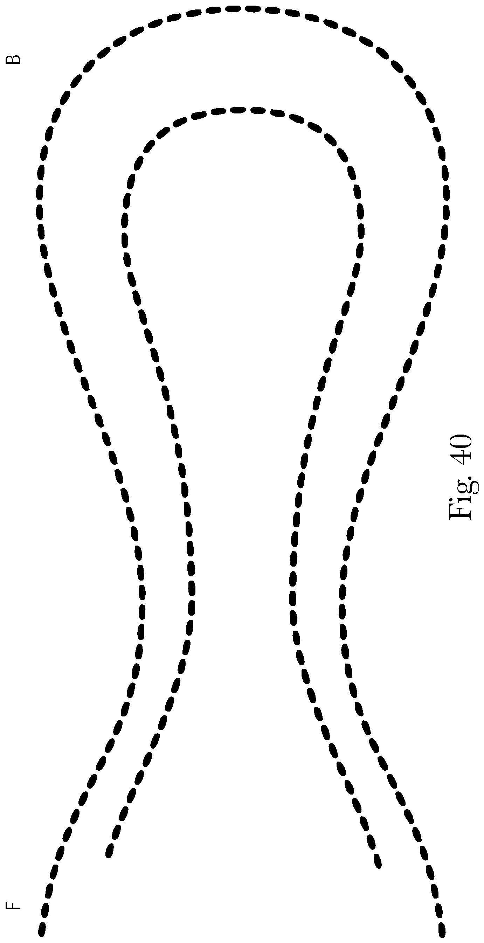

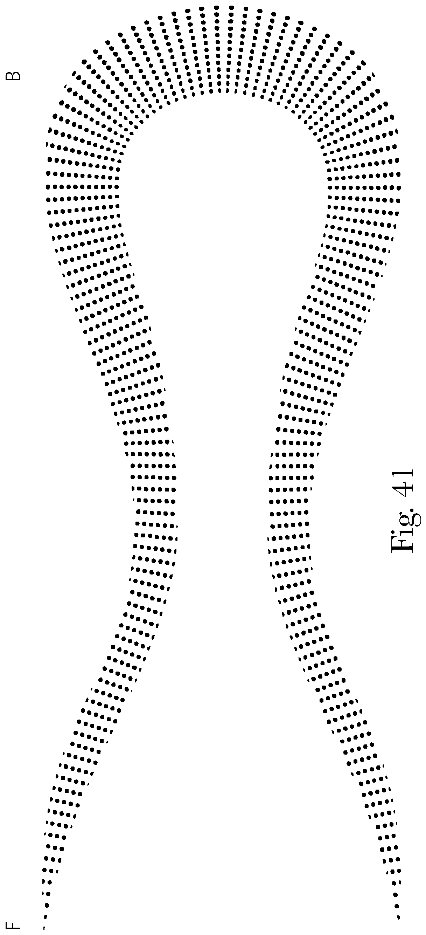

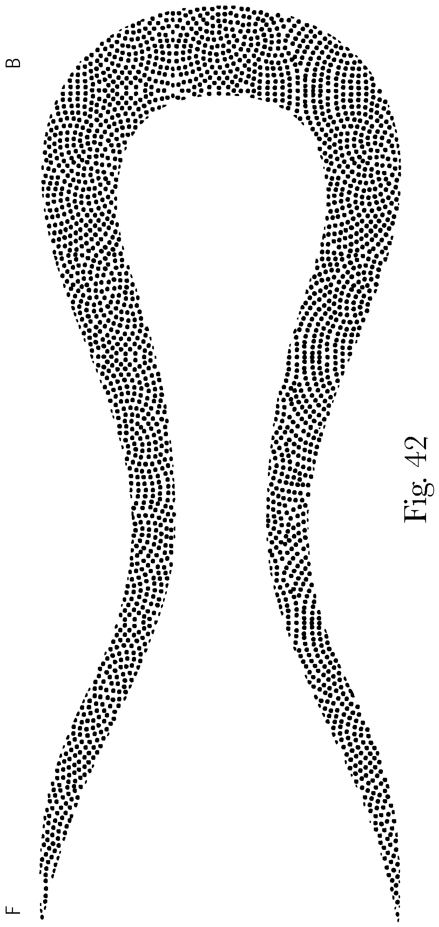

Figs. 33-42 are examples of designs for topsheets, or portions thereof, for absorbent articles. The designs may comprise geometric, morphological, and/or chemical treatments in one or more patterns. The designs may also comprise flow control materials that may or may not for an at least mostly enclosed perimeter. The patterns may vary throughout the length, or a portion of the length, of the topsheet. The front portion of the topsheet is labeled F and the back portion of the topsheet is labeled B. The topsheets may be rectangular or any other suitable shape and have the designs thereon or therein. The treatments may be embossments, printed graphics, flow control materials, and/or apertures, or combinations thereof, for example. Figs. 40-42 illustrate designs that comprise one or more flow control materials that form an at least mostly enclosed perimeter and are discontinuous. Fig. 40 illustrates two flow control materials that each form an at least mostly enclosed perimeter and that are both discontinuous, although one or both of them could be continuous.

As is illustrated in the various substrates (e.g., topsheets) with zones illustrated in Figs. 15-42, the zones may take on a variety of configurations, sizes, and shapes, and the zones, or portions thereof, may comprise chemical, geometric, and/or morphological treatments or, a particular zone may not comprise treatments at all. The zones or portions thereof may comprise flow control materials. The various treatments will be discussed below in greater detail below. Apertures may be created via punching, slitting, hydroforming, or overbonding followed by ringrolling. 3D structures may be formed with various solid state formation technologies, such as SELFing, IPS, or rIPS.

### Geometric Treatments

Some example geometric treatments are now discussed with reference to the figures. Because the phrase "geometric treatments" includes morphological treatments and apertures, only apertures will be discussed here, although it will be understood that the morphological treatments discussed below are also within the scope of the phrase "geometric treatments" as per the definition of geometric treatment.

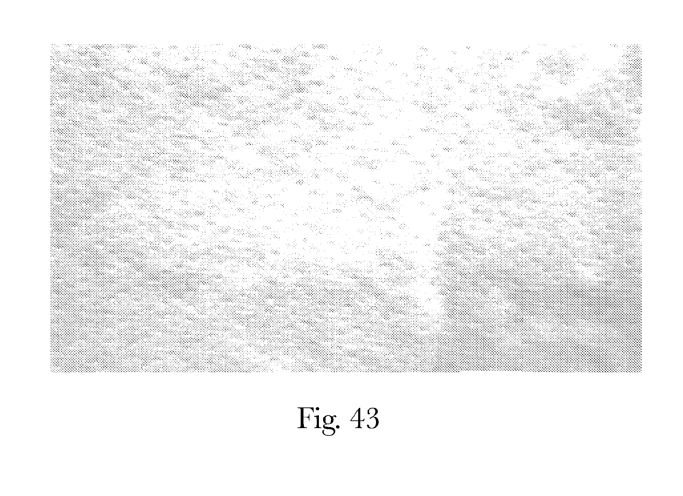

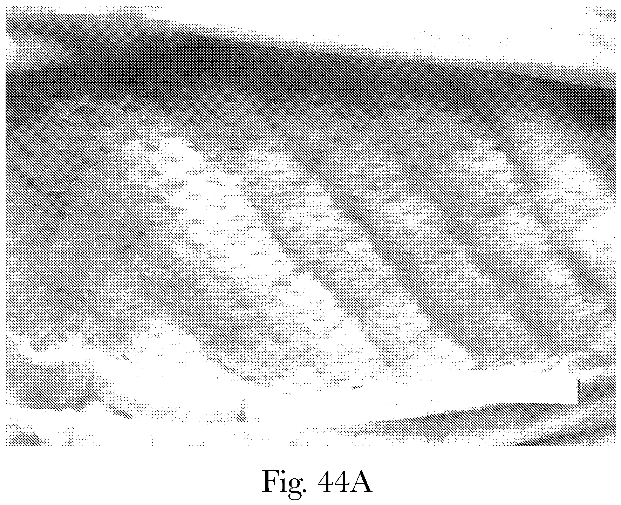

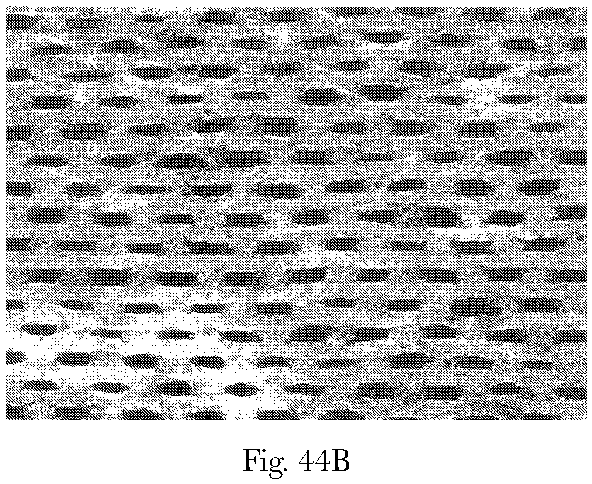

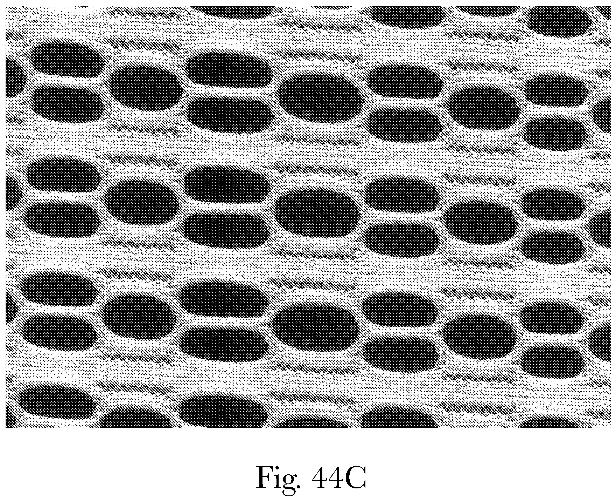

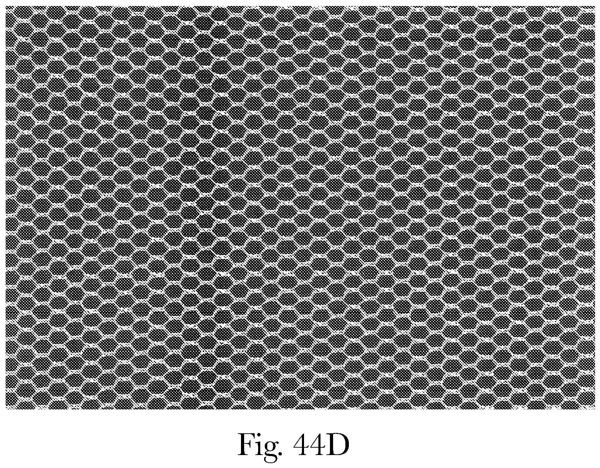

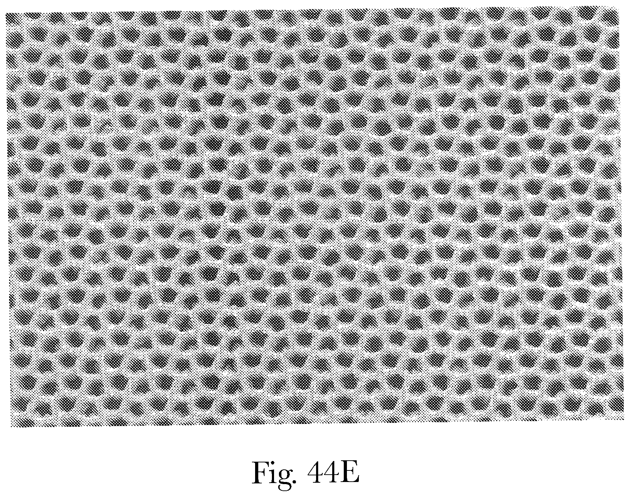

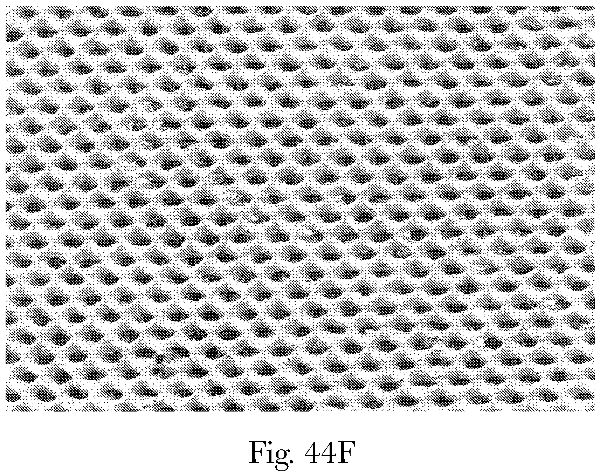

Figs. 43 through 44F illustrate example substrates (e.g., topsheets or portions thereof) that comprise geometric treatments comprising apertures. Figs. 43 illustrates a 27 gsm nonwoven web having apertures defined therein, while Figs. 44A illustrates an 18 gsm bicomponent nonwoven web having apertures defined therein. The apertures of a geometric treatment may have any suitable size, shape, configuration, and/or pattern. The apertures may be formed by any aperturing process generally known in the art, such as overbonding and ring rolling to rupture the overbonds, and pinholing, for example. The apertures may be uniformly spaced or non-uniformly spaced relative to each other. Furthermore, the apertures in each zone may be the same size or different sizes in the same zone or in different zones. Figs. 17-19, 21, 21A, and 27-30, among other figures, show various non-limiting examples of geometric treatments comprising apertures in a topsheet or other substrate. Fig. 44B illustrates an example substrate for use as a portion of, or all of, a topsheet. Fig. 44C illustrates an example fabric substrate for use as a portion of, or all of, a topsheet. Fig. 44D illustrates an example mesh substrate for use as a portion of, or all of, a topsheet. Fig. 44E illustrates an example film for use as a portion of, or all of, a topsheet. Fig. 44F illustrates an example film for use as a portion of, or all of, a topsheet.

The example substrate of Fig. 44B may have an effective aperture area (according to the Aperture Test below) in the range of about 0.5 mm² to about 10 mm², about 1 mm² to about 8 mm², about 1 mm² to about 6 mm², about 1 mm² to about 5 mm², about 1 mm² to about 3 mm², about 1.5 mm² to about 2.5 mm², about 1.9 mm², about 2.0 mm², about 2.1 mm², or about 2.011 mm², specifically reciting all 0.1 mm² increments within the specified ranges and all ranges formed therein or thereby. The example substrate of Fig. 44B may have a % effective open area (according to the Aperture Test below) in the range of about 5% to about 40%, about 10% to about 30%, about 15% to about 25%, about 16% to about 20%, about 17%, about 18%, or about 19%, specifically reciting all 0.1 % increments within the specified ranges and all ranges formed therein or thereby.

The example fabric substrate of Fig. 44C may have an effective aperture area (according to the Aperture Test below) in the range of about 3 mm² to about 30 mm², about 6 mm² to about 20 mm², about 8 mm² to about 14 mm², about 9 mm² to about 13 mm², about 10 mm² to about 12 mm², about 11 mm², about 12 mm², about 11.1 mm², about 11.2 mm², or about 11.11 mm², specifically reciting all 0.1 mm² increments within the specified ranges and all ranges formed therein or thereby. The example substrate of Fig. 44C may have a % effective open area (according to the Aperture Test below) in the range of about 15% to about 55%, about 20% to about 45%, about 25% to about 45%, about 30% to about 40%, about 35%, about 34%, or about 34.7%, specifically reciting all 0.1 % increments within the specified ranges and all ranges formed therein or thereby.

The example mesh substrate of Fig. 44D may have an effective aperture area (according to the Aperture Test below) in the range of about 0.2 mm² to about 4 mm², about 0.5 mm² to about 3 mm², about 0.5 mm² to about 2 mm², about 0.5 mm² to about 1.5 mm², about 0.8 mm² to about 1.3 mm², about 0.9 mm², about 1 mm², about 1.1 mm², about 1.2 mm², or about 1.018 mm², specifically reciting all 0.1 mm² increments within the specified ranges and all ranges formed therein or thereby. The example substrate of Fig. 44D may have a % effective open area (according to the Aperture Test below) in the range of about 20% to about 90%, about 30% to about 80%, about 50% to about 80%, about 50% to about 70%, about 55% to about 70%, about 60%, about 63.9%, or about 65%, specifically reciting all 0.1 % increments within the specified ranges and all ranges formed therein or thereby.

The example film of Fig. 44E may have an effective aperture area (according to the Aperture Test below) in the range of about 0.1 mm² to about 2 mm², about 0.1 mm² to about 1.5 mm², about 0.3 mm² to about 1 mm², about 0.4 mm² to about 0.9 mm², about 0.4 mm² to about 0.75 mm², about 0.5 mm² , about 0.508 mm², about 0.51 mm², or about 0.52 mm², specifically reciting all 0.1 mm² increments within the specified ranges and all ranges formed therein or thereby. The example film of Fig. 44E may have a % effective open area (according to the Aperture Test below) in the range of about 5% to about 50%, about 10% to about 35%, about 10% to about 30%, about 12% to about 25%, about 15%, about 18%, about 18.2%, or about 19%, specifically reciting all 0.1 % increments within the specified ranges and all ranges formed therein or thereby.

The example film of Fig. 44F may have an effective aperture area (according to the Aperture Test below) in the range of about 0.1 mm² to about 2 mm², about 0.1 mm² to about 1.5 mm², about 0.3 mm² to about 1 mm², about 0.4 mm² to about 0.9 mm², about 0.4 mm² to about 0.75 mm², about 0.4 mm², about 0.486 mm², or about 0.5 mm², specifically reciting all 0.1 mm² increments within the specified ranges and all ranges formed therein or thereby. The example film of Fig. 44F may have a % effective open area (according to the Aperture Test below) in the range of about 3% to about 35%, about 3% to about 20%, about 3% to about 15%, about 5% to about 13%, about 6%, about 11%, about 8%, about 8.7%, or about 9%, specifically reciting all 0.1 % increments within the specified ranges and all ranges formed therein or thereby.

A topsheet of an absorbent article may have two or more zones with at least a first zone being positioned on a first side of a lateral axis or a first side of a substantially laterally-extending separation element and with a second zone being positioned on a second side of the lateral axis or a second side of a substantially laterally-extending separation element. The first zone may have apertures defined therein which may have an effective aperture area in the range of about 0.2mm² to about 15mm², specifically reciting all 0.1 mm² increments within the specified range, according to the Aperture Test described below. The first zone may have an effective open area of about 15% to about 40%, specifically reciting all 0.1% increments within the specified range, according to the Aperture Test described below. The second zone may have apertures defined therein which may have an effective aperture area in the range of about 0.1mm² to about 2.0mm², about 0.05mm² to about 2mm², about 0.5mm² to about 2mm², or about 1.0mm², according to the Aperture Test described below. The second zone may have a % effective open area of about 2% to about 15%, specifically reciting all 0.1% increments within the specified range, according to the Aperture Test described below. The apertures in the first zone may be larger than the apertures in the second zone (e.g., 15-35% larger or 25% larger) such that the first zone is configured for BM management and such that the second zone is configured for urine management. Either of the first and second zones may comprise one or more chemical treatments and/or one or more morphological treatments. The morphological treatment may be present in a portion of, or all of, the first zone and/or the second zone. The absorbent article may also comprise barrier leg cuffs and a waist edge.

Any of the apertures of the geometric treatments may overlap or not overlap portions of, and/or all of, the channels (e.g., 49, 49') in the liquid management system 50 or the channels (e.g., 26, 26', 27, 27') in the absorbent core 28, if either set of channels is provided.

### Morphological Treatments

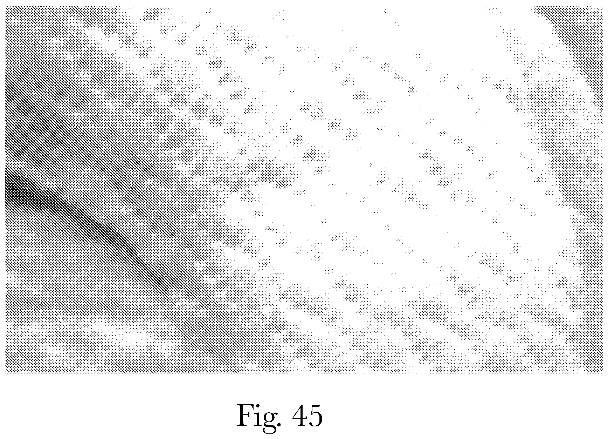

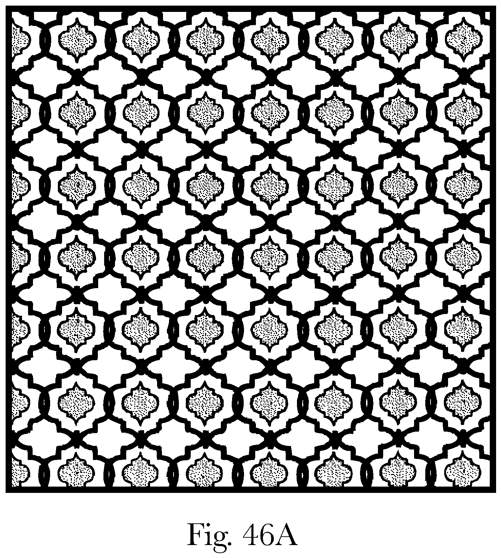

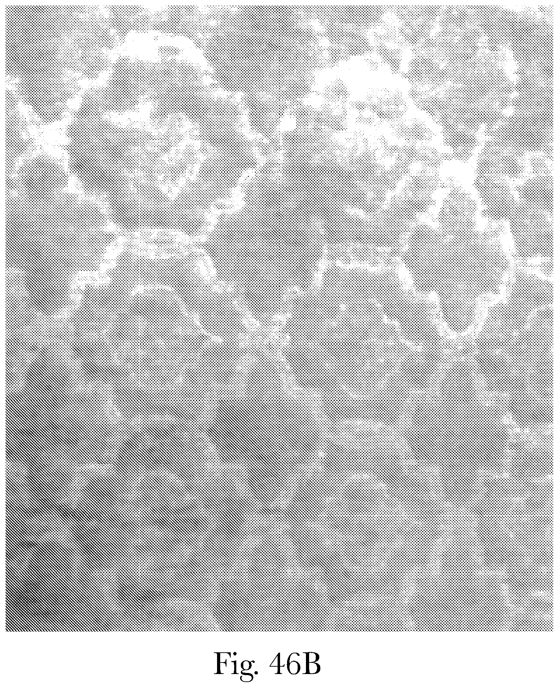

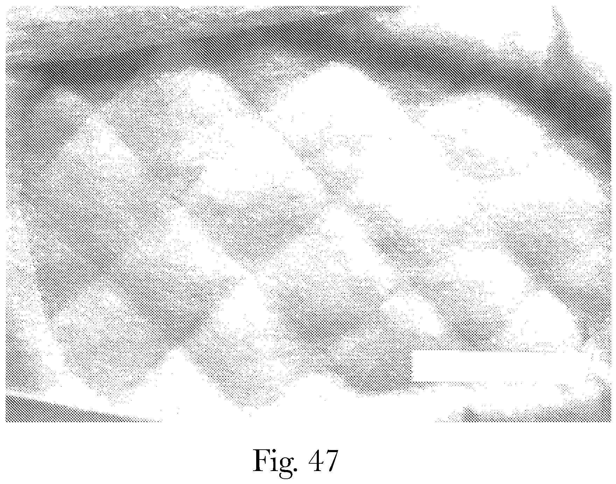

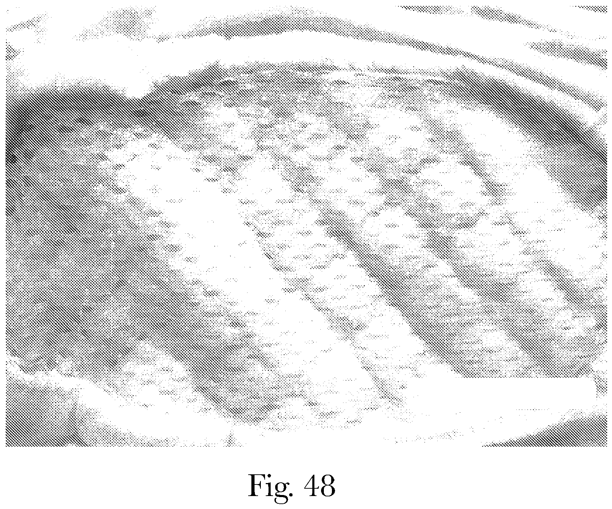

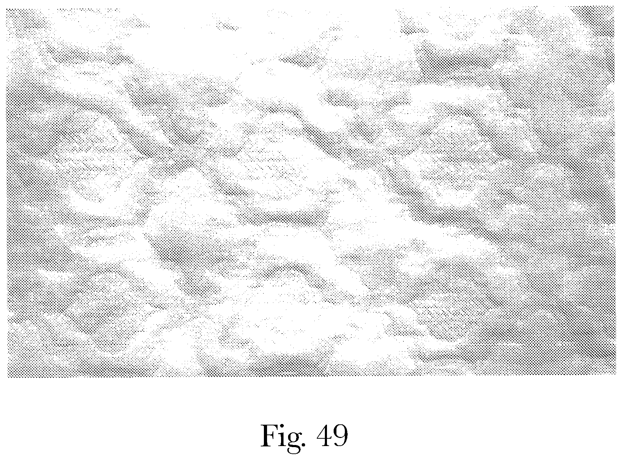

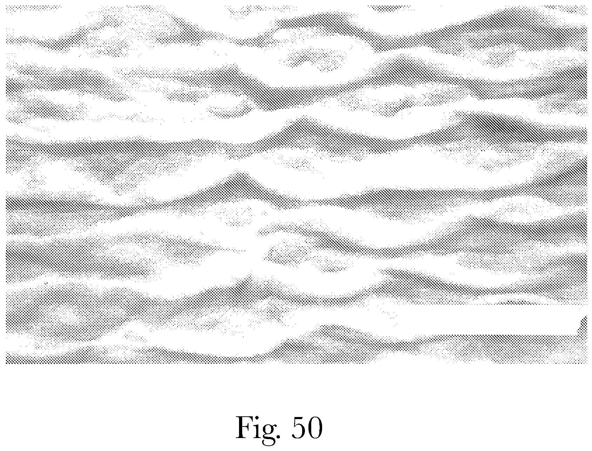

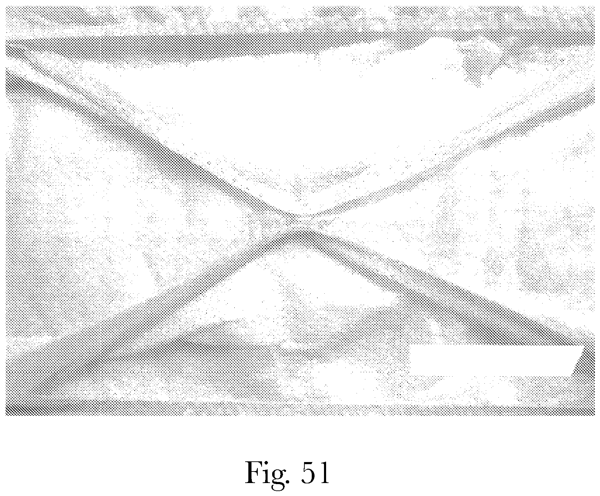

Various morphological treatments may be present in various zones of the topsheet 24, the LMS 50, or other substrate . Figs. 45-50 illustrate various morphological treatments. Fig. 45 illustrates an embossed pattern. Fig. 46A is a graphical illustration of the pattern of embossments shown in Fig. 46B. Fig. 47 illustrates another embossed pattern. Fig. 48 illustrated another embossed pattern with the substrate being apertured. Fig. 49 illustrates another embossed pattern. Fig. 50 illustrates an example morphological treatment that comprises puckered areas. The puckered areas may be formed in a two layer topsheet. Adhesive, or patterned adhesive, may be present intermediate the first and second layers. The first layer may be embossed to achieve the shape of the puckered areas and then attached to the second layer using the patterned adhesive. Fig. 51 illustrates an example morphological treatment that comprises fold lines. The fold lines may be formed by substrates attached to the topsheet. Other morphological treatments are illustrated in at least some of Figs. 20-32 in a topsheet. The elements of a morphological treatment may have any suitable sizes, shapes, dimensions, frequencies, configurations, and/or patterns. The morphological treatments may be formed by any processes known to those of skill in the art. The elements of each morphological treatment may be uniformly spaced or non-uniformly spaced relative to each other. Furthermore, the elements of a morphological treatment in each zone may be the same or different. The elements of a morphological treatment in a zone, or in more than one zone, may differ in pattern, depth, width, length, and/or frequency, or may be the same. One zone having a morphological treatment may be symmetrical to, or asymmetrical to, another zone having a morphological treatment with respect to a lateral axis, a longitudinal axis, or a substantially laterally-extending separation element of an absorbent article. Any of the zones having a morphological treatment may overlap or not overlap portions of, or all of, the channels (e.g., 49, 49') in the liquid management system 50 and/or the channels (e.g., 26, 26', 27, 27') in the absorbent core 28, if present.

An example morphological treatment is illustrated in Figs. 52 and 53. Fig. 53 is an exploded view from circle 53 of Fig. 52. In this example morphological treatment, portions 106 of the liquid management system 50 (one or more layers, or all layers, of the LMS 50) extend into or through the liquid permeable topsheet 24. Discontinuities 116 may be formed in a garment-facing surface 120 of the liquid management system 50. Flaps 107 may be formed in the liquid permeable topsheet 24 at locations where the portions 106 extend into and/or through the liquid permeable topsheet 24. Such a structure may aid in liquid absorption in that the liquid management system 50 may quickly wick liquid through the topsheet 24 owing to the projections 106. This structure may also help reduce the amount of time liquid, such as urine, remains on the topsheet 24.

Another example morphological treatment is illustrated in Figs. 54 and 55. Fig. 55 is an exploded view from circle 55 of Fig. 54. In this example morphological treatment, portions 106' of the liquid permeable topsheet 24 extend into or though one or more layers, or all layers, of the liquid management system 50. Discontinuities 116' may be formed in a wearer-facing surface 120' of the liquid permeable topsheet 24. Flaps 107' may be formed in the liquid management system 50 at locations where the portions 106' extend into and/or through the liquid management system 50. Such a structure may aid in liquid adsorption in that an absorbent core positioned under the LMS 50 may quickly absorb liquid through the topsheet 24 and the LMS 50 owing to the projections 106'. Such a structure may also reduce the amount of time liquid, such as urine, is present on the topsheet 24. This morphological treatment is essentially the reverse of the morphological treatment illustrated in Figs. 52 and 53.

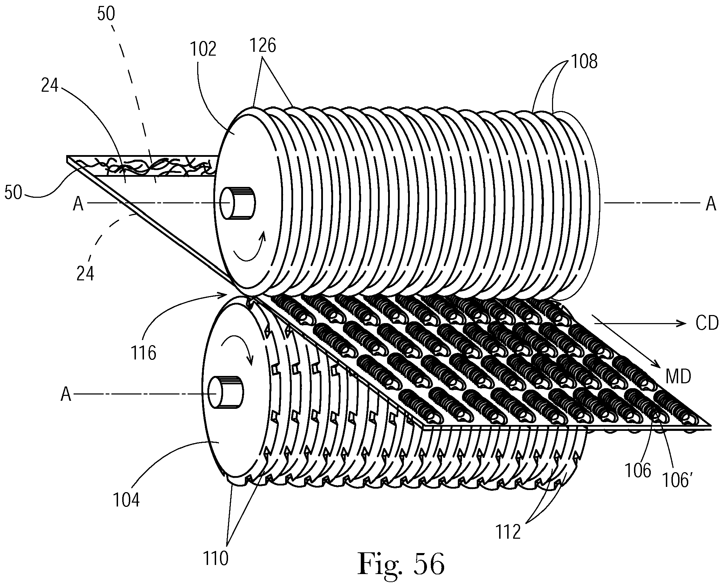

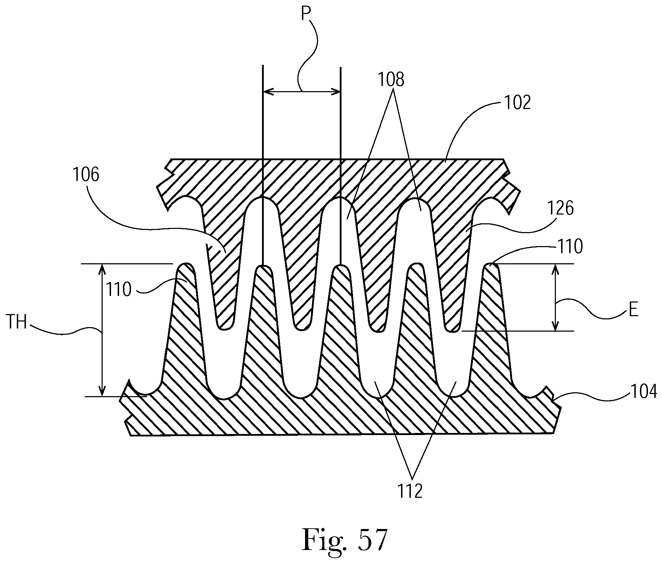

Both of the two above-described morphological treatments may be formed in a similar fashion. The portions 106 or 106' may be formed using the process illustrated in Fig. 56. The process may comprise advancing a web comprising the liquid permeable topsheet 24 superimposed over at least a portion of the liquid management system 50 or advancing a web comprising the liquid management system 50 superimposed over at least a portion of the liquid permeable topsheet 24 (as indicated in dashed reference numbers 24 and 50) through a nip 116 formed by two rolls 102 and 104. The two rolls rotate above their respective longitudinal axes A in the direction shown by the arrows on the rolls. The roll 102 may comprise a groove roll comprising a plurality of outwardly extending ridges 126 forming a plurality of grooves 108 therebetween. The roll 104 may comprise a plurality of radially outwardly extending teeth 110 and a plurality of grooves 112 formed therebetween. The ridges 126 of the roll 102 may be configured to extend into the grooves 112 of the roll 104 and the teeth 110 of the roll 104 may be configured to extend into the grooves 108 of the roll 102 to form the portions 106 or 106'. As can be seen in Fig. 56, this arrangement forces portions of the liquid management system 50 through or into the liquid permeable topsheet 24 to form the portions 106 or forces portions of the liquid permeable topsheet 24 though or into the liquid management system 50 to form the portions 106'. The intermeshing of the rolls 102 and 104 is illustrated in more detail in Fig. 57, without the liquid permeable topsheet 24 and the liquid management system 50 being present. "P" is the pitch between the teeth 110, "E" is the depth of engagement of the ridges 126 into the grooves 112, and "TH" is the tooth height. All of these measurements may be varied to achieve differently shaped and/or sized portions 106 or 106', for example. Further details regarding such a structure, and the method of producing it, is disclosed in U.S. Patent No. 7,648,752 to Hoying et al.

The teeth 110 on the roll 104 may vary about the circumference of the roll 104, in the direction of rotation of the roll 104, such that the formed projections 106 may differ in pattern, depth, length, width, and/or frequency of the extension of the liquid management system 50 into or through the liquid permeable topsheet 24 or the liquid permeable topsheet 24 into or through the liquid management system 50. The teeth on about 50% of the roll 104, in the direction of rotation of the roll 104, may have a first configuration, size, length, width, pattern, frequency etc. while the teeth on the remainder of the roll may have a second configuration, size, length, width, pattern, frequency etc. In other instances, teeth having certain configurations may be formed in more than two zones on the roll 104. The varying of the teeth may allow the rolls to create zones having different portions 106 or 106' and thereby different morphological treatments, in, for example, a first zone of the topsheet 24 and a second zone of the topsheet 24.

### Chemical Treatments

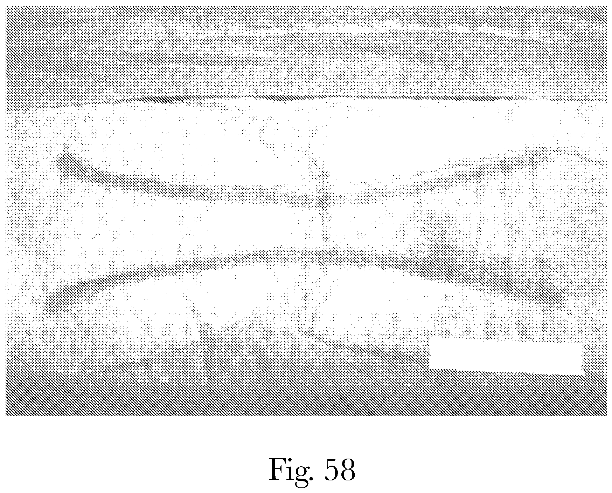

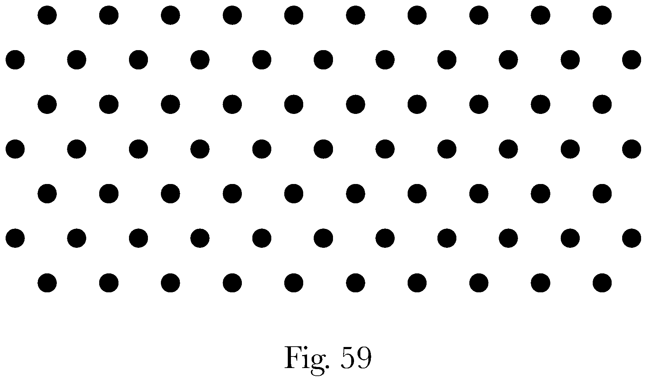

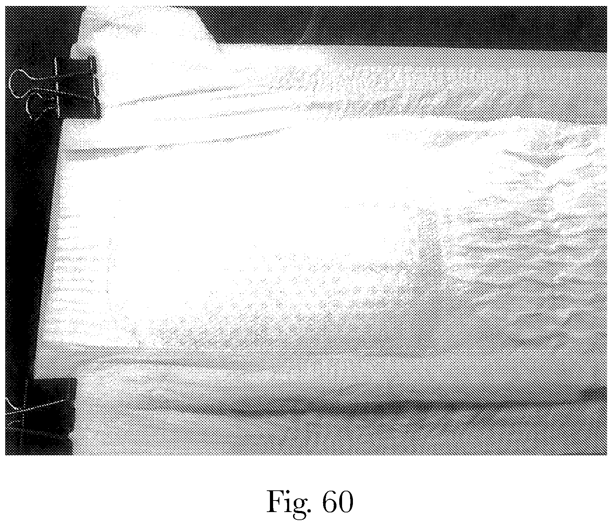

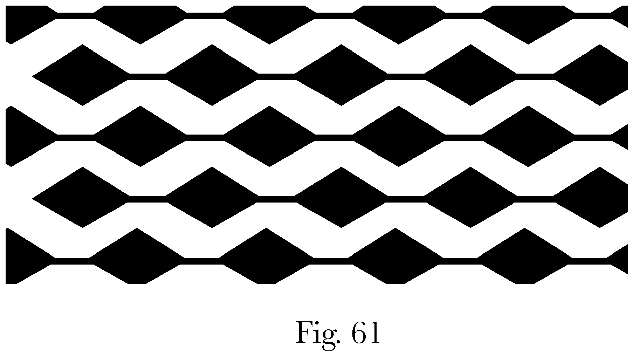

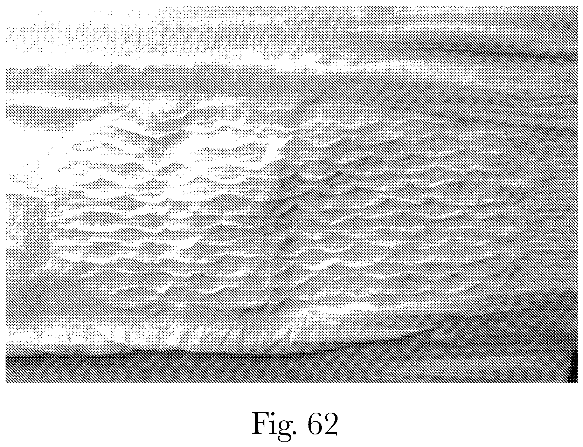

One or more of the various zones of the topsheet 24 and/or the liquid management system 50 may comprise one or more chemical treatments. The chemical treatments may have any suitable color, size, shape, thickness, and/or pattern. The various zones may have the same chemical treatments or different chemical treatments. More than one chemical treatment may be provided in a certain zone or in multiple zones. Some chemical treatments may surround, or at least partially surround, other zones of chemical treatments, or may surround, or at least partially surround, other geometric or morphological treatments. In other instances, some chemical treatments may at least partially overlap other chemical treatments and/or be positioned on or overlap geometric and/or morphological treatments. Some example chemical treatments are illustrated in Figs. 58-65. Various other figures, as described above, also illustrate various chemical treatments. Fig. 58 illustrates an example chemical treatment of a pattern of printed or dyed channels on a topsheet. Figs. 59 and 60 illustrate an example chemical treatment of a pattern of printed or dyed dots on a topsheet. Figs. 61 and 62 illustrate a chemical treatment of a pattern of printed or dyed diamonds on a topsheet.

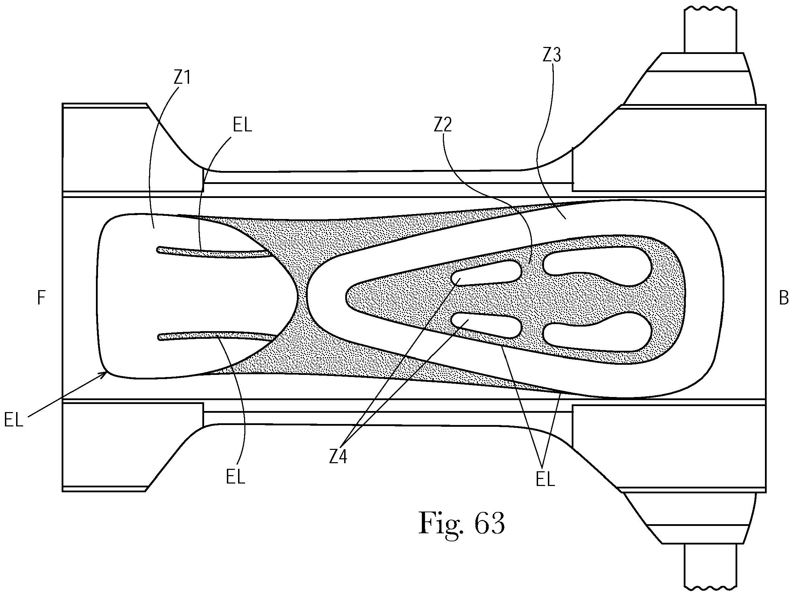

Fig. 63 illustrates an example of an absorbent article having a plurality of zones. One or more of the zones may comprise one or more chemical treatments and/or one or more other treatments. The first zone, Z1, may comprise a chemical treatment comprising a skin care composition, the second zone, Z2, may comprise a chemical treatment comprising an anti-stick lotion or a composition configured to aid in preventing BM from sticking to the skin of the wearer, and the third zone, Z3, may comprise a chemical treatment comprising a composition comprising an ink or a pigment. The various chemical treatments may be in patterns (e.g., stripes, dots) in the various zones. The patterns may be the same or different in various zones and/or within a certain zone. The absorbent article may also comprise deep embossed lines, EL, on either side of the third zone, Z3 and/or surrounding the first zone, Z1. The absorbent article may further comprise fourth and fifth zones, Z4 and Z5, respectively. These fourth and fifth zones, Z4 and Z5, may comprise any number of chemical, geometric, and/or morphological treatments, or may not comprise any treatments. The topsheet may also comprise flow control materials. The flow control materials may form a perimeter of, or be positioned around, the first zone Z1, the second zone Z2, and/or the third zone Z3. The flow control materials may be continuous or discontinuous.

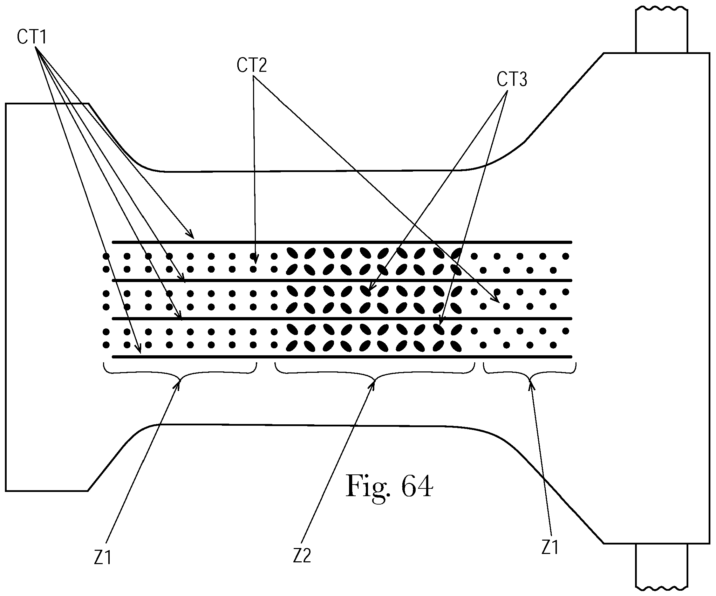

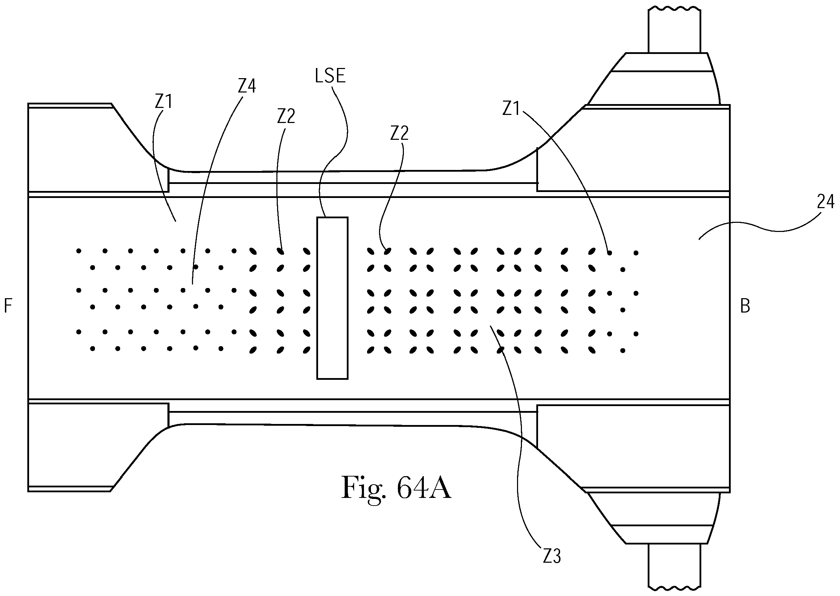

Fig. 64 illustrates an example of an absorbent article having at least two zones, Z1, and Z2. Zone Z1 may exist at opposite ends of zone Z2. Zones Z1 and Z2 may each comprise a geometric treatment and/or a morphological treatment. One or both of the morphological treatments may comprise portions of a liquid management system extending into or at least partially through the topsheet, as explained above in reference to Figs. 52-57. The morphological treatment in zone Z1 may have a different pattern (e.g., height, width, shape, frequency, length, spacing, color, material etc.) than the morphological treatment in zone Z2. Zones, Z1 and Z2, may also comprise a first chemical treatment, CT1 formed in dots, for example. The first chemical treatment, CT1 may comprise an anti-stick lotion or composition configured to aid in preventing BM from sticking to the skin of a wearer. Zone Z1 may also comprise a second chemical treatment, CT2, comprising an active ingredient, such as zinc oxide, or a vitamin, such as Vitamin E, for example. Zone Z2 may comprise a third chemical treatment, CT3, comprising an enzyme inhibitor, such as hexamidine, for example. The third chemical treatment, CT3, may also be a BM anti-stick lotion.

Fig. 64A illustrates an example of an absorbent article having four zones. Zone Z1 may comprise a morphological treatment (see e.g., Figs. 52 and 54). Zone Z2 may comprise a geometric treatment comprising apertures. Zone Z3 may comprise a chemical treatment comprising a BM anti-stick lotion that may be hydrophobic or hydrophilic. Zone Z4 may comprise a chemical treatment comprising a lotion with active ingredients. The lotion of Zone Z4 may be hydrophobic or hydrophilic. Zone 4 may at least partially overlap zone 1. Zone 3 may at least partially overlap zones 1 and 2. The absorbent article may also comprise a lateral separation element LSE.

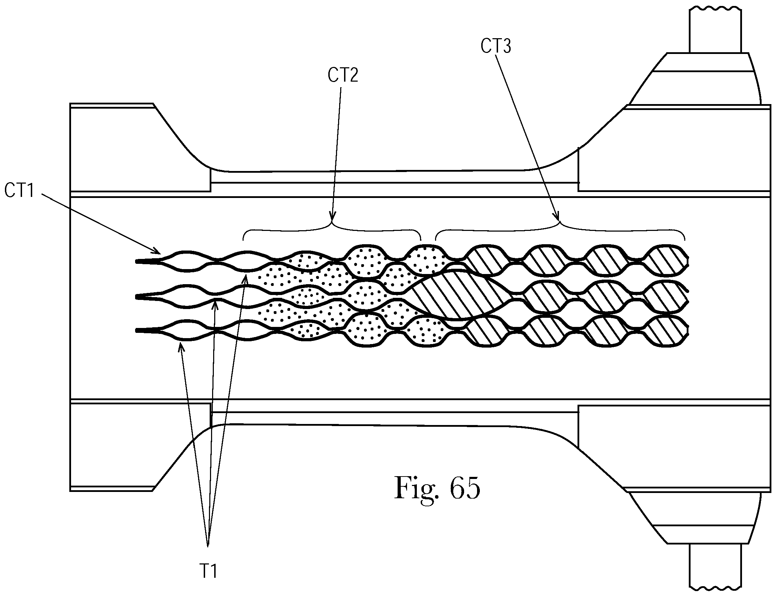

Fig. 65 illustrates an example of an absorbent article having a plurality of treatments. The first treatment, T1, may comprise a morphological treatment of deep embossing and a first chemical treatment, CT1, comprising compositions comprising inks or pigments. A second chemical treatment, CT2, may comprise an active ingredient, such as such as zinc oxide, or a vitamin, such as Vitamin E, for example. The active ingredient may be formed by a pattern of dots, for example. A third chemical treatment, CT3, may comprise one or more flow control materials. The flow control material may be formed by a pattern of stripes, for example. Any of the areas where the various treatments are present may also comprise additional treatments.

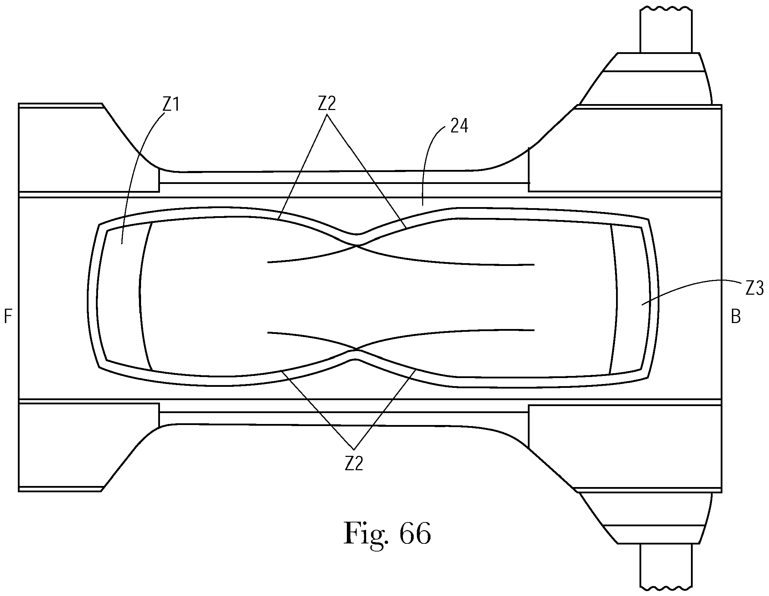

Fig. 66 illustrates an example of an absorbent article having a plurality of flow control materials in various zones. Zone Z1 may comprise a flow control material for urine. Zone Z2 may comprise a flow control material for BM. Zone Z3 may comprise a flow control material for either urine or BM. The absorbent article may also comprise one or more other treatments.

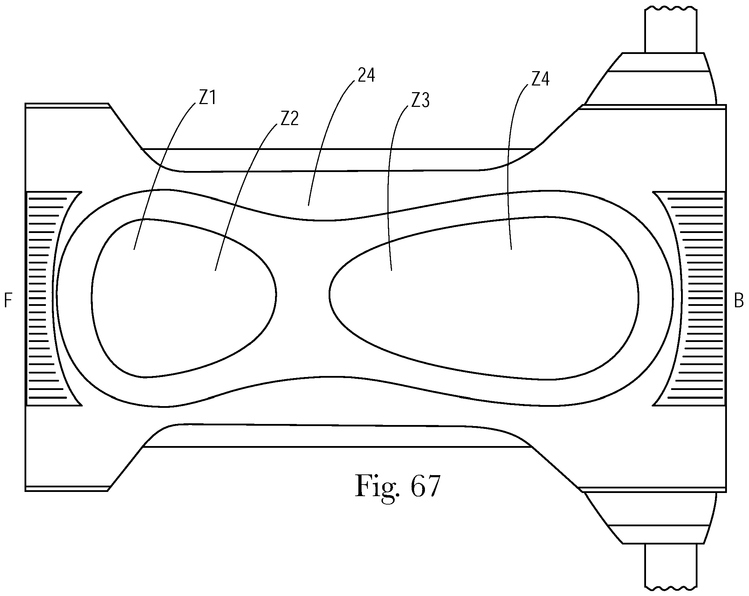

Fig. 67 illustrates an example of an absorbent article having a plurality of zones. Zone Z1 may comprise a morphological treatment (see e.g., Figs. 52 and 54). Zone Z2 may comprise a chemical treatment. Zone Z2 may at least partially overlap zone Z1. Zone Z3 may comprise a morphological treatment (see e.g., Figs. 52 and 54). Zone Z4 may comprise a BM anti-stick lotion. Zone Z4 may at least partially overlap Zone Z3. The absorbent article may also comprise one or more other treatments.

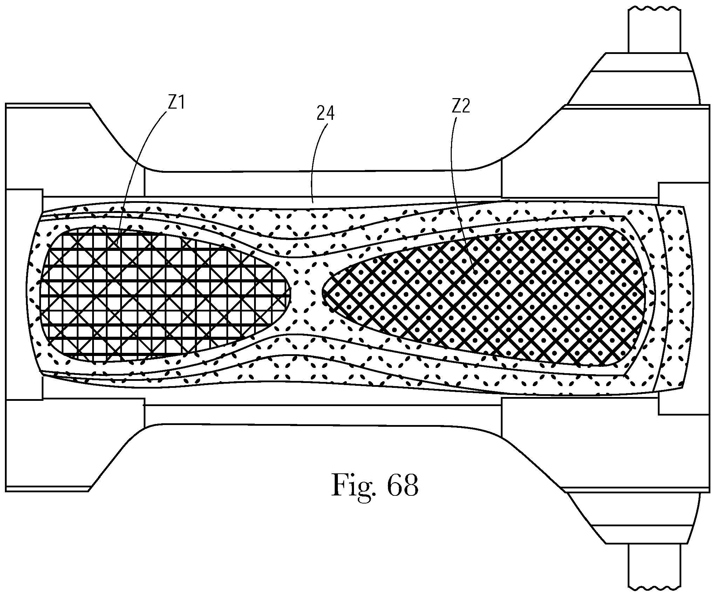

Fig. 68 illustrates an example of an absorbent article having a plurality of zones. Zone Z1 may comprise a geometric treatment comprising apertures. Zone Z2 may comprise a geometric treatment comprising apertures. The aperture may create a pattern. Zone Z1 may comprise a skin care composition and Zone Z2 may comprise a BM anti-stick lotion. The absorbent article may also comprise one or more other treatments.

Any of the zones having one or more chemical treatments or other treatments may overlap or not overlap portions of, or all of, the channels (e.g., 49, 49') in the liquid management system 50 and/or the channels (e.g., 26, 26', 27, 27') in the absorbent core 28.

### Hydrophobic/Hydrophilic

As discussed above, various zones of the topsheet may comprise chemical treatments that render those zones hydrophobic or hydrophilic or more hydrophobic or hydrophilic. Some additional details on these zones are provided below. A topsheet of an absorbent article may comprise a first chemical treatment comprising a hydrophilic coating and/or a second chemical treatment comprising a hydrophobic coating. In other instances, only one hydrophilic coating or hydrophobic coating may be provided on a topsheet. In still other instances, the first chemical treatment may by hydrophobic and the second chemical treatment may have a different hydrophilicity (i.e., more hydrophobic, less hydrophobic, more hydrophilic, or less hydrophilic). The hydrophilic coating and the hydrophobic coating may be positioned in any suitable areas or zones of the topsheet. The topsheet may comprise a first zone comprising the hydrophilic coating and a second zone comprising the hydrophilic coating. In other instances, one or more zones in the topsheet may comprise chemical treatments comprising either hydrophilic or hydrophobic coatings. The first and second zones of the topsheet may be positioned in the front and/or the back or the front and the back, respectively, of the absorbent article. In other instances, the first and seconds zones of the topsheet may be positioned on opposite sides of a substantially laterally-extending separation element, lateral axis, or longitudinal axis.

Chemical treatments that are hydrophilic may facilitate penetration of the liquid permeable topsheet, the LMS, and/or the absorbent core by urine or runny BM or may promote adherence of the BM to the liquid permeable topsheet vs. the wearer's skin. Hydrophobic treatments may hold a skin care composition in place to facilitate bodily contact, reduce rewet, improve skin dryness, and/or may prevent, or at least inhibit, runny BM from re-soiling the skin after having passed through a portion of the liquid permeable topsheet or the LMS.

### Active Ingredients/Enzyme Inhibitors/Vitamins

As mentioned above, any of various zone discussed herein or portions of the topsheets of the present disclosure may also comprise one or more active ingredients, enzyme inhibitors, and/or vitamins. An example of an active ingredient is zinc oxide, which may function as an antimicrobial agent to reduce the pathogen count on skin to help prevent opportunistic infection of the skin or urinary tract. An example of an enzyme inhibitor is hexamidine, which is a protease inhibitor which may help prevent attack by proteolytic fecal enzymes, such as trypsin, on the skin barrier, thereby reducing irritation and dermatitis. An example of a vitamin is Vitamin E, which may help stabilize the skin barrier function.

### Durable/Transferrable

As discussed above, some of the zones of the topsheets may comprise a substantially durable chemical treatment and/or a substantially transferrable chemical treatment. Those treatments are discussed further below. A topsheet of an absorbent article may comprise a first chemical treatment that is substantially transferrable or that is substantially durable and/or a second chemical treatment that is substantially durable or that is substantially transferrable. In one instance, only one substantially transferrable or substantially durable chemical treatment may be provided on a topsheet. The substantially transferrable and substantially durable chemical treatments may be positioned in any suitable areas or zones of the topsheet. The topsheet may comprise a first zone comprising the first chemical treatment that is substantially transferrable or substantially durable and a second zone comprising the second chemical treatment that is substantially durable or substantially transferrable. In other instances, one or more zones in the topsheet may comprise chemical treatments that are either substantially transferrable or substantially durable. In some instances, two substantially durable or substantially transferrable chemical treatments may be provided in various zone of a topsheet. In such an instance, one of the treatments may be applied to the topsheet in a different pattern, thickness, basis weight as the other treatment. The first and second zones of the topsheet may be positioned in the front and/or the back or the front and the back, respectively, of the absorbent article. In other instances, the first and second zones of the topsheet may be positioned on opposite sides of a substantially laterally-extending separation element, lateral axis, or longitudinal axis.

Some of the benefits of having a substantially durable chemical treatment are that a chemical treatment, such as a soil capture polymer composition, may remain on the topsheet to hold the BM on the topsheet more tightly than the BM adheres to the skin to facilitate more removal of BM from the skin with the absorbent article. Other examples of substantially durable chemical treatments may include: a) a hydrophilic treatment to help maintain urine penetration with subsequent urine events (i.e., after the first urination event), and/or b) a flow control material to preferentially restrict urine penetration in select regions of the topsheet (as described in greater detail below).

Some of the benefits of having a substantially transferrable chemical treatment are that a chemical treatment, such as a skin care composition, may be at least partially transferred to the skin of the wearer for skin barrier enhancement vs. irritants or other skin health benefits. Another example of substantially transferrable chemical treatments is BM anti-stick lotion which would prevent, or at least inhibit, BM adhesion to skin.

### Flow Control Materials

As discussed above, some of the zones of the topsheets may comprise chemical treatments comprising flow control material. Those flow control material are discussed further below.

The flow control materials may hasten, slow, or restrict the movement and/or penetration of bodily exudates on, into, or through a topsheet of an absorbent article.

A topsheet may comprise a first chemical treatment comprising a first flow control material (see Fig. 21C, Z5) and/or a second chemical treatment comprising a second flow control material (see Fig. 21C, Z5). These flow control materials may be the same or different. In one instance, only one flow control material may be provided on a topsheet. The first and second flow control materials may be positioned in any suitable areas, zones, and/or portions of the topsheet. The topsheet may comprise a first zone (see Fig. 15 Z1) comprising the first flow control material and a second zone (see Fig. 15, Z2) comprising the second flow control material. The first and second zones of the topsheet may be positioned in the front and/or the back or the front and the back, respectively, of the absorbent article. In other instances, the first and second zones of the topsheet may be at last partially positioned on opposite sides of a substantially laterally-extending separation element (see Fig. 15), lateral axis, or longitudinal axis.

The first and second flow control materials may be the same or different. The first flow control material may have a different permeability, basis weight, surface energy, and/or thickness compared to the second flow control material. The first flow control material and/or the second flow control material may comprise a pigment, a colorant, a printed ink, a dye, and/or a skin care composition. The pigments, colorants, printed inks, dyes, and/or skin care compositions of the first and second flow control materials may be the same or different.

The first and second flow control materials may have different patterns in different zones (see e.g. Fig. 21A, Z2 and Z4), for example, a first pattern in a first zone and a second pattern in a second zone, or may have the same patterns in different zones.

Flow control materials may at least partially, or fully, restrict or slow penetration of liquid bodily exudates in certain regions of the absorbent article and direct the exudates to regions where they may be preferentially stored (i.e., to reduce bulkiness, to improve fit, and/or to improve (reduce) rewet to the skin). For example, if the flow control material is located in a region where urine is expected to insult the product from the wearer and is directed in either or both longitudinal directions, this may reduce the wet bulk in the crotch region. Such a feature may also provide appearance and/or comfort/freedom of motion benefits for the absorbent article. Alternatively, the flow control material may prevent, or at least inhibit, rewet of bodily exudates (i.e., that have travelled through the topsheet) back through certain regions of the topsheet to avoid soiling otherwise uncontaminated skin.

In other instances, the flow control materials may hasten the flow of bodily exudates either across a topsheet or into or through a topsheet to allow the absorbent article to more effectively absorb the bodily exudates.

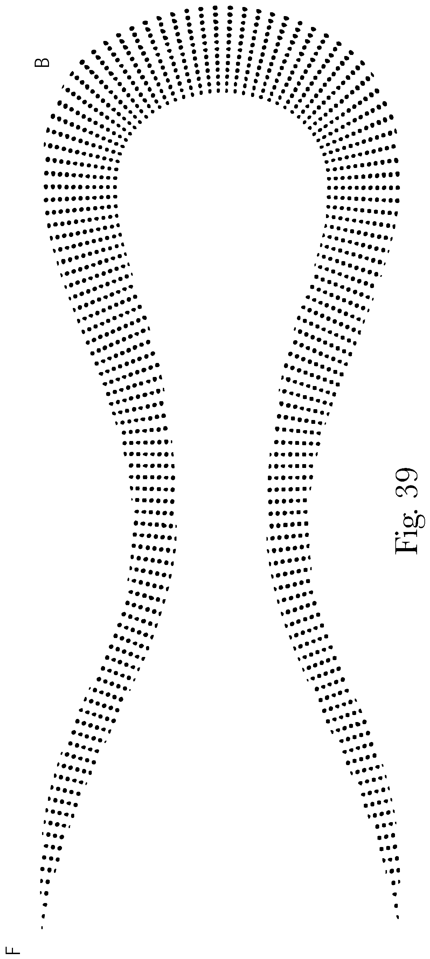

As an example of a flow control material, an absorbent article may comprise a liquid permeable topsheet, a liquid impermeable backsheet, and an absorbent core positioned at least partially intermediate the liquid permeable topsheet and the liquid impermeable backsheet. The liquid permeable topsheet may comprise a flow control material that may form an at least mostly, or fully, enclosed perimeter over at least a portion of the absorbent core (see e.g., Fig. 20, Z2-Z4, Fig. 21A, Z5, Fig. 21B, Z3, Fig. 21C, Z4, Fig. 24, Z1, Z3, and Z4, Fig. 29, Z3, and Figs. 39-42, for example. The term "at least mostly enclosed perimeter" means a perimeter that is at least 60% enclosed (i.e., 60% of a full enclosed shape is formed). The at least mostly enclosed perimeter, or fully enclosed perimeter, may be continuous (see Figs. 20, Z3 and Z4, 21A, Z5, 21B, Z3, 21C, Z4) or discontinuous (see Figs. 39-42). Discontinuous flow control material may be provided in a pattern of elements, such as stripes, dots, dashed lines, etc. The flow control material may extend less than 0.2mm, or more than 0.2mm, outwardly from the liquid permeable topsheet (measured according to the Flow Control Material Outward Extension Method herein) and may penetrate into or through a portion of the liquid permeable topsheet. The flow control material may also be applied to the liquid management system.

The topsheet may also comprise one or more geometric treatments, morphological treatments, and/or chemical treatments (chemical treatments other than flow control materials). Referring to Fig. 20, the geometric treatment Z2 may be fully positioned within, or partially positioned within, on, or outside of, or at least partially outside of, the at least mostly enclosed perimeter PI, or the fully enclosed perimeter formed by the first flow control material Z4. The topsheet 24 may also comprise a second flow control 500 material forming a second at least mostly, or fully, enclosed perimeter P2 in the topsheet 24. The second perimeter P2 may be at least partially, or fully, positioned within the at least mostly enclosed perimeter P1. A second geometric treatment, a second morphological treatment, or a second chemical treatment Z1 may be at least partially, or fully, positioned within the at least mostly enclosed second perimeter P2. The topsheet may also comprise one or more additional chemical treatments. The chemical treatments may be positioned on, inside, or outside of the perimeter P1 or the second perimeter P2.

The flow control material may penetrate through at least 5%, at least 10%, at least 15%, at least 20%, at least 25%, at least 50%, or more of a thickness of the liquid permeable topsheet. The flow control material may also penetrate onto and/or at least partially through the liquid management system of the absorbent article. In some instances, the flow control material may only be present on a surface of the topsheet or the liquid management system and/or the absorbent core. In other instances the flow control material may penetrate fully through the topsheet and/or the liquid management system. In still other instances, the flow control material may penetrate the topsheet or the liquid management system in the range of 1% to 75%, 5% to 50%, 5% to 50%, and 5% to 25% specifically reciting all 0.1% increments within the specified ranges and all ranges formed therein or thereby. The flow control materials may be applied to the topsheet or the liquid management system by any suitable methods known to those of skill in the art, such as spraying, melting, and rolling.

The flow control material may penetrate through at a depth of at least about 50 micrometers, at least about 75 micrometers, at least about 100 micrometers, at least about 150 micrometers, at least about 200 micrometers, at least about 250 micrometers, at least about 300 micrometers, or at least about 350 micrometers, below a wearer-facing surface of the topsheet, measured according to the Penetration Depth Test Method herein. The flow control material may also penetrate through at a depth in the range of about 25 micrometers to about 500 micrometers, about 50 micrometers to about 500 micrometers, about 100 micrometers to about 400 micrometers, about 100 micrometers to about 300 micrometers, about 150 micrometers to about 300 micrometers, or about 100 micrometers to about 250 micrometers, specifically reciting all 0.5 micrometer increments within the above-specified ranges and all ranges formed therein or thereby, below a wearer-facing surface of the topsheet, measured according to the Penetration Depth Test Method herein.

As another example, an absorbent article may comprise a liquid permeable topsheet, a liquid impermeable backsheet, a lateral axis defining a front region of the absorbent article on a first side of the lateral axis and a back region of the absorbent article on a second side of the lateral axis, and an absorbent core positioned at least partially intermediate the liquid permeable topsheet and the liquid impermeable backsheet. Referring generally to Fig. 15, the liquid permeable topsheet 24 may comprise a first zone Z1 situated primarily (e.g., at least 60%, or at least 75%) in the front region and comprising a first flow control material and a second zone Z2 situated primarily (e.g., at least 60%, or at least 75%) in the back region and comprising a second flow control material. The first flow control material may be different than, or the same as, the second flow control material. The first and second flow control material may be applied at the same or different basis weights and/or thicknesses. At least a portion of the first and second flow control materials may be deposited on or adjacent to a longitudinal axis 80 of the absorbent article. The first flow control material may comprise a different (or the same) pattern, shape, and/or coverage area than the second flow control material. The first zone Z1 may also comprise a first geometric treatment and the second zone Z2 may also comprise a second geometric treatment. The first flow control material may be associated with (e.g., positioned on, under, within, over, working in combination with) the first geometric treatment and the second flow control material may be associated with the second geometric treatment.

The flow control material(s) may also be positioned at: a) areas of a topsheet or liquid management system of an absorbent article which, in use, will be subjected to "high pressure" from the body (e.g., under the ischia or buttocks cheeks); b) at or proximate to a perimeter of the absorbent core; c) at or proximate to a location where the bodily exudates are expected to insult the absorbent article (i.e., to direct the bodily exudates to either the front or back portions (or other portions) of the absorbent article); d) at or proximate to perimeters of various zones, e) about or proximate to the lateral axis; or f) about a or proximate to, or on, a laterally extending separation element.

The absorbent article may comprise a liquid management system positioned at least partially intermediate the liquid permeable topsheet and the absorbent core. The liquid management system may comprise a third flow control material applied thereto and positioned to form an at least mostly, or fully, enclosed perimeter over a portion of the absorbent core.

As another example, an absorbent article may comprise a liquid permeable topsheet, a liquid impermeable backsheet, a liquid management system, and an absorbent core positioned at least partially intermediate the liquid management system and the liquid impermeable backsheet. The liquid management system may be positioned at least partially intermediate the liquid permeable topsheet and the absorbent core. Referring to Fig. 21, the liquid permeable topsheet 24 or the liquid management system may comprise a flow control material 502 positioned in or surrounding, or at least partially surrounding, a urine insult zone 504 and/or a feces insult zone 506. In other instances, the liquid permeable topsheet or the liquid management system may comprise a first flow control material 502 positioned in or surrounding, or at least partially surrounding a urine insult zone 504 and may comprise a second flow control material 502' positioned in or surrounding, or at least partially surrounding a feces insult zone 506.

Any of the various zones, or one or more portions thereof, either in addition to or in lieu of the various treatments discussed herein, may have one or more colors, shades of the same color, different colors, and/or different delta E values. For example, a first zone, or one or more portions thereof, may have a first color and a second zone (or third or fourth zone etc.), or one or more portions thereof, may have a second color. The first and second colors may be the same or different. In certain instances, all or some of zones, or one or more portions thereof, in a front region of an absorbent article may have the same or different colors as all or some of the zones, or portions thereof, in a rear region of the absorbent article. In some instances, the various treatments may have colors that are the same or different. The color may be provided by or on any of the materials, treatments (e.g., chemical treatments), and/or layers (e.g., acquisition layer) within the various zones.

### Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 100 mm, less than about 95 mm, less than about 90 mm, less than about 85 mm, less than about 85 mm, but greater than about 75 mm, less than about 80 mm, less than about 78 mm, less than about 76 mm, or less than about 74 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 100 mm, from about 70 mm to about 95 mm, from about 72 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

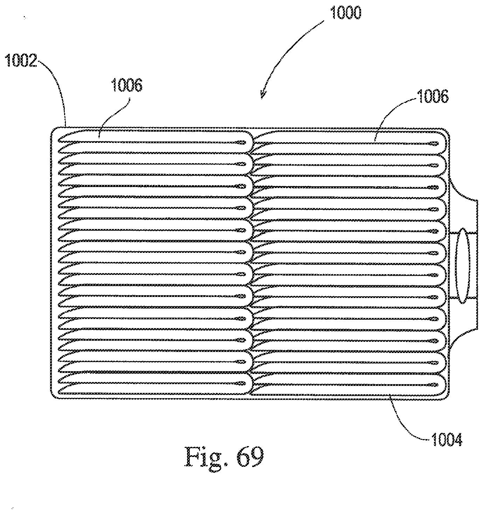

Fig. 69 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

### TEST METHODS

Condition all samples at about 23 °C ± 2 C° and about 50% ± 2% relative humidity for 2 hours prior to testing.

### Aperture Test

Aperture dimensions, effective aperture area, and % effective open area measurements are performed on images generated using a flat bed scanner capable of scanning in reflectance mode at a resolution of 6400 dpi and 8 bit grayscale (a suitable scanner is the Epson Perfection V750 Pro, Epson, USA). Analyses are performed using ImageJ software (v.s 1.46, National Institute of Health, USA) and calibrated against a ruler certified by NIST. A steel frame (100 mm square, 1.5 mm thick with an opening 60 mm square) is used to mount the specimen and a black glass tile (P/N 11-0050-30, available from HunterLab, Reston, VA) is used as the background for the scanned images.

Take the steel frame and place double-sided adhesive tape on the bottom surface surrounding the interior opening. To obtain a specimen, lay the absorbent article flat on a lab bench with the wearer-facing surface directed upward. Remove the release paper of the tape, and adhere the steel frame to the topsheet of the absorbent article. Using a razor blade, excise the top sheet from the underling layers of the absorbent article around the outer perimeter of the frame. Carefully remove the specimen such that its longitudinal and lateral extension is maintained. A cryogenic spray (such as Cyto-Freeze, Control Company, Houston TX) can be used to remove the topsheet specimen from the underling layers, if necessary. Five replicates obtained from five substantially similar absorbent articles are prepared for analysis.

Place the ruler on the scanner bed, close the lid and acquire a 50mm by 50mm calibration image of the ruler in reflectance mode at a resolution of 6400 dpi and 8 bit grayscale. Save the image as an uncompressed TIFF format file. Lift the lid and remove the ruler. After obtaining the calibration image, all specimens are scanned under the same conditions and measured based on the same calibration file. Next, place the framed specimen onto the center of the scanner bed with the wearer-facing surface of the specimen facing the scanner's glass surface. Place the black glass tile on top of the frame covering the specimen, close the lid and acquire a scanned image. In like fashion scan the remaining four replicates.

Open the calibration file in ImageJ and perform a linear calibration using the imaged ruler, with the scale set to Global so that the calibration will be applied to subsequent specimens. Open a specimen image in ImageJ. View the histogram and identify the gray level value for the minimum population located between the dark pixel peak of the holes and the lighter pixel peak of the nonwoven. Threshold the image at the minimum gray level value to generate a binary image. In the processed image, the apertures appear as black and nonwoven as white.

Select the analyze particles function. Set the minimum aperture area exclusion limit to 0.3 mm² and for the analysis to exclude the edge apertures. Set the software to calculate: effective aperture area, perimeter, feret (length of the aperture) and minimum feret (width of the aperture). Record the average effective aperture area to the nearest 0.01 mm², and the average perimeter to the nearest 0.01 mm. Again select the analyze particles function, but his time set the analysis to include the edge holes as it calculates the effective aperture areas. Sum the effective aperture areas (includes whole and partial apertures) and divide by the total area included in the image (2500 mm²). Record as the % effective open area to the nearest 0.01%.

In like fashion analyze the remaining four specimen images. Calculate and report the average effective aperture area to the nearest 0.01 mm², the average aperture perimeter to the nearest 0.01mm, feret and minimum feret to the nearest 0.01 mm, and the % effective open area to the nearest 0.01% for the five replicates.

### Flow Control Material Outward Extension Method/ Flow Control Material Penetration Depth Method

### Experimental settings

The values indicated herein are measured according to the methods indicated herein below, unless specified otherwise. All measurements are performed at 21°C ± 2°C and 50% ± 20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### Equipment

- Razor blade: VWR Single Edge Industrial, 0.009" thick surgical carbon steel or equivalent.
- SEM (Hitachi S3500N or equivalent)

### Procedure

An area 1.5 cm to 3 cm in length and 1.3 cm in width is cut from an absorbent article in an area where the liquid permeable topsheet comprises a flow control material. This subsample is sectioned along the length, which includes flow control material, using a new razor blade (VWR Single Edge Industrial, 0.009" thick surgical carbon steel or equivalent.) If the topsheet contains protruded or inverted elements, the cross-sections should be made across the middle of these features, in an area which includes flow control material. The sectioned subsample is adhered to an SEM mount using double-sided conductive tape, with the topsheet's wearer-facing surface of the section up and sectioned edge at the mount edge, so that the cross-section is revealed when the SEM stage is tilted backward 90°. The mounted sample is sputter Au coated and viewed in an SEM (Hitachi S3500N or equivalent). It can be advantageous to use OsO4 or I vapor staining to help visualize some flow control materials. If these are used, sputter Au coating to increase the sample's conductivity may not be necessary.

The following measurements should be made with the manual line tool in PCI v4.2 image analysis software (or equivalent) from cross-sectional images:
(1) **Flow Control Material Outward Extension Method** is determined by measuring the z-directional distance between the lowest point the flow control material is observed in a cross-sectional image (in the z-direction away from the wearer) and the nearest point on the topsheet's wearer-facing surface where no flow control material has been applied. Three areas should be measured if the topsheet system is planar, and line lengths averaged. If the topsheet contains protrusions and/or inversions, three areas should be measured for the protrusions (if there are any), three for the inversions (if there are any), and three from land area between these features (if there is any). Line lengths measured at protrusions are averaged; line lengths measured at inversions are averaged, and line lengths measured at land area are averaged. Flow Control Material Penetration Depth is designated as the higher of these averaged measurements and reported in micrometers to the nearest 0.1 micrometer.
(2) **Flow Control Material Penetration Depth Method** is determined by measuring the z-directional distance between the lowest point the flow control material is observed in a cross-sectional image (in the z-direction away from the wearer) and the nearest point on the topsheet's wearer-facing surface where no flow control material has been applied. Three areas should be measured if the topsheet system is planar, and line lengths averaged. If the topsheet contains protrusions and/or inversions, three areas should be measured for the protrusions (if there are any), three for the inversions (if there are any), and three from land area between these features (if there is any). Line lengths measured at protrusions are averaged; line lengths measured at inversions are averaged, and line lengths measured at land area are averaged. Flow Control Material Penetration Depth is designated as the higher of these averaged measurements and reported in micrometers to the nearest 0.1 micrometer.

### In-Bag Stack Height Test

The in-bag stack height of a package of absorbent articles is determined as follows:

### Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

### Test Procedure

Absorbent article packages are equilibrated at 23 ± 2 °C and 50 ± 5 % relative humidity prior to measurement.

The horizontal sliding plate is raised and an absorbent article package is placed centrally under the horizontal sliding plate in such a way that the absorbent articles within the package are in a horizontal orientation (see Fig. 69). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

## Claims

1. An absorbent article comprising:
a liquid permeable topsheet;
a liquid impermeable backsheet;
an absorbent core positioned at least partially intermediate the liquid permeable topsheet and the liquid impermeable backsheet; and
a lateral axis defining a front region and a back region;
wherein the liquid permeable topsheet comprises a first zone at least partially in the front region, wherein the first zone comprises a first morphological treatment configured for urine handling, wherein the liquid permeable topsheet comprises a second zone at least partially in the back region, wherein the second zone comprises a second morphological treatment configured for BM handling, wherein a pattern of the first morphological treatment in the first zone is nonsymmetrical to a pattern of the second morphological treatment in the second zone about the lateral axis; and
wherein the first morphological treatment or the second morphological treatment comprises areas where portions of a liquid management system, positioned at least partially intermediate the liquid permeable topsheet and the absorbent core, extend into or through the liquid permeable topsheet or comprises areas where portions of the liquid permeable topsheet extend into or through the liquid management system.

2. The absorbent article according to Claim 1, wherein the liquid permeable topsheet has apertures defined therein.

3. The absorbent article according to Claim 1 or 2, wherein a portion of the first zone and a portion of the second zone overlap a longitudinal axis of the absorbent article.

4. The absorbent article according to any one of the preceding claims, comprising a substantially laterally-extending separation element, wherein the first zone is positioned on a first side of the separation element, and wherein the second zone is positioned on a second side of the separation element.

5. The absorbent article according to any one of the preceding claims, wherein the first zone or the second zone comprises a chemical treatment.

6. The absorbent article according to any one of the preceding claims, wherein the first zone or the second zone comprises a third morphological treatment.

7. The absorbent article according to any one of Claims 1-5, wherein the liquid permeable topsheet comprises a third morphological treatment.

8. The absorbent article according to any one of the preceding claims, wherein the liquid permeable topsheet comprises a third zone comprising a third morphological treatment.

9. The absorbent article according to any one of the preceding claims, wherein an area of the first zone is smaller than an area of the second zone.

10. The absorbent article according to any one of the preceding claims, wherein the first zone has a dimension of at least 40mm measured in a direction parallel to the lateral axis

11. The absorbent article according to any one of the preceding claims, wherein the second zone has a dimension of at least 40mm measured in the direction parallel to the lateral axis.

12. The absorbent article according to any one of the preceding claims, comprising barrier leg cuffs.

13. The absorbent article according to any one of the preceding claims, comprising a waist edge.

14. The absorbent article according to any one of the preceding claims, wherein the absorbent article is a diaper, an adult incontinence product, or a sanitary napkin.

15. A package comprising a plurality of the absorbent articles of any one of the preceding claims, wherein the package has an in-bag stack height of less than about 80 mm, according to the In-Bag Stack Height Test herein.

## Patentansprüche

1. Absorptionsartikel, umfassend:
eine flüssigkeitsdurchlässige Oberschicht;
eine flüssigkeitsundurchlässige Unterschicht;
einen Absorptionskern, der mindestens teilweise zwischen der flüssigkeitsdurchlässigen Oberschicht und der flüssigkeitsundurchlässigen Unterschicht angeordnet ist; und
eine Querachse, die einen Vorderbereich und einen Rückenbereich definiert;
wobei die flüssigkeitsdurchlässige Oberschicht eine erste Zone umfasst, die mindestens teilweise im Vorderbereich angeordnet ist, wobei die erste Zone eine erste morphologische Behandlung umfasst, die zum Umgang mit Urin konfiguriert ist, wobei die flüssigkeitsdurchlässige Oberschicht eine zweite Zone umfasst, die mindestens teilweise im Rückenbereich angeordnet ist, wobei die zweite Zone eine zweite morphologische Behandlung umfasst, die zum Umgang mit Stuhl konfiguriert ist, wobei ein Muster der ersten morphologischen Behandlung in der ersten Zone nicht-symmetrisch zu einem Muster der zweiten morphologischen Behandlung in der zweiten Zone um die Querachse ist; und
wobei die erste morphologische Behandlung oder die zweite morphologische Behandlung Flächen umfasst, wo Abschnitte eines Flüssigkeitshandhabungssystem, die mindestens teilweise zwischen der flüssigkeitsdurchlässigen Oberschicht und dem Absorptionskern angeordnet sind, in oder durch die flüssigkeitsdurchlässige Oberschicht verlaufen, oder Flächen umfasst, wo Abschnitte der flüssigkeitsdurchlässigen Oberschicht in oder durch das Flüssigkeitshandhabungssystem verlaufen.

2. Absorptionsartikel nach Anspruch 1, wobei die flüssigkeitsdurchlässige Oberschicht darin definierte Öffnungen aufweist.

3. Absorptionsartikel nach Anspruch 1 oder 2, wobei ein Abschnitt der ersten Zone und ein Abschnitt der zweiten Zone eine Längsachse des Absorptionsartikels überlappen.

4. Absorptionsartikel nach einem der vorstehenden Ansprüche, umfassend ein wesentlich quer verlaufendes Trennelement, wobei die erste Zone auf einer ersten Seite des Trennelements angeordnet ist, und wobei die zweite Zone auf einer zweiten Seite des Trennelements angeordnet ist.

5. Absorptionsartikel nach einem der vorstehenden Ansprüche, wobei die erste Zone oder die zweite Zone eine chemische Behandlung umfasst.

6. Absorptionsartikel nach einem der vorstehenden Ansprüche, wobei die erste Zone oder die zweite Zone eine dritte morphologische Behandlung umfasst.

7. Absorptionsartikel nach einem der Ansprüche 1 bis 5, wobei die flüssigkeitsdurchlässige Oberschicht eine dritte morphologische Behandlung umfasst.

8. Absorptionsartikel nach einem der vorstehenden Ansprüche, wobei die flüssigkeitsdurchlässige Oberschicht eine dritte Zone umfasst, die eine dritte morphologische Behandlung umfasst.

9. Absorptionsartikel nach einem der vorstehenden Ansprüche, wobei eine Fläche der ersten Zone kleiner als eine Fläche der zweiten Zone ist.

10. Absorptionsartikel nach einem der vorstehenden Ansprüche, wobei die erste Zone eine Abmessung von mindestens 40 mm aufweist, gemessen in einer Richtung parallel zur Querachse.

11. Absorptionsartikel nach einem der vorstehenden Ansprüche, wobei die zweite Zone eine Abmessung von mindestens 40 mm aufweist, gemessen in der Richtung parallel zur Querachse.

12. Absorptionsartikel nach einem der vorstehenden Ansprüche, der Sperrbeinbündchen umfasst.

13. Absorptionsartikel nach einem der vorstehenden Ansprüche, der einen Taillenrand umfasst.

14. Absorptionsartikel nach einem der vorstehenden Ansprüche, wobei der Absorptionsartikel eine Windel, ein Erwachseneninkontinenzprodukt oder eine Damenbinde ist.

15. Packung, umfassend eine Vielzahl von Absorptionsartikeln nach einem der vorstehenden Ansprüche, wobei die Packung eine Stapelhöhe im Beutel von weniger als ungefähr 80 mm, gemäß dem Test der Stapelhöhe im Beutel hierin, aufweist.

## Revendications

1. Article absorbant comprenant :
une feuille de dessus perméable aux liquides ;
une feuille de fond imperméable aux liquides ;
une âme absorbante positionnée au moins partiellement entre la feuille de dessus perméable aux liquides et la feuille de fond imperméable aux liquides ; et
un axe latéral définissant une région antérieure et une région postérieure ;
dans lequel la feuille de dessus perméable aux liquides comprend une première zone au moins partiellement dans la région antérieure, dans lequel la première zone comprend un premier traitement morphologique configuré pour la prise en charge de l'urine, dans lequel la feuille de dessus perméable aux liquides comprend une deuxième zone au moins partiellement dans la région postérieure, dans lequel la deuxième zone comprend un deuxième traitement morphologique configuré pour la prise en charge des selles, dans lequel un motif du premier traitement morphologique dans la première zone est non symétrique par rapport à un motif du deuxième traitement morphologique dans la deuxième zone autour de l'axe latéral ; et
dans lequel le premier traitement morphologique ou le deuxième traitement morphologique comprend des régions où des parties d'un système de prise en charge de liquide, positionnées au moins partiellement entre la feuille de dessus perméable aux liquides et l'âme absorbante, s'étendent dans ou à travers la feuille de dessus perméable aux liquides ou comprennent des régions où des parties de la feuille de dessus perméable aux liquides s'étendent dans ou à travers le système de prise en charge de liquide.

2. Article absorbant selon la revendication 1, dans lequel la feuille de dessus perméable aux liquides a des ouvertures définies en son sein.

3. Article absorbant selon la revendication 1 ou 2, dans lequel une partie de la première zone et une partie de la deuxième zone chevauchent un axe longitudinal de l'article absorbant.

4. Article absorbant selon l'une quelconque des revendications précédentes, comprenant un élément de séparation s'étendant de façon essentiellement latérale, dans lequel la première zone est positionnée sur un premier côté de l'élément de séparation, et dans lequel la deuxième zone est positionnée sur un deuxième côté de l'élément de séparation.

5. Article absorbant selon l'une quelconque des revendications précédentes, dans lequel la première zone ou la deuxième zone comprend un traitement chimique.

6. Article absorbant selon l'une quelconque des revendications précédentes, dans lequel la première zone ou la deuxième zone comprend un troisième traitement morphologique.

7. Article absorbant selon l'une quelconque des revendications 1 à 5, dans lequel la feuille de dessus perméable aux liquides comprend un troisième traitement morphologique.

8. Article absorbant selon l'une quelconque des revendications précédentes, dans lequel la feuille de dessus perméable aux liquides comprend une troisième zone comprenant un troisième traitement morphologique.

9. Article absorbant selon l'une quelconque des revendications précédentes, dans lequel une aire de la première zone est plus petite qu'une aire de la deuxième zone.

10. Article absorbant selon l'une quelconque des revendications précédentes, dans lequel la première zone a une dimension d'au moins 40 mm mesurée dans une direction parallèle à l'axe latéral.

11. Article absorbant selon l'une quelconque des revendications précédentes, dans lequel la deuxième zone a une dimension d'au moins 40 mm mesurée dans la direction parallèle à l'axe latéral.

12. Article absorbant selon l'une quelconque des revendications précédentes, comprenant des revers de jambe de barrière.

13. Article absorbant selon l'une quelconque des revendications précédentes, comprenant un bord de ceinture.

14. Article absorbant selon l'une quelconque des revendications précédentes, dans lequel l'article absorbant est une couche, un produit pour l'incontinence chez l'adulte ou une serviette hygiénique.

15. Conditionnement comprenant une pluralité des articles absorbants selon l'une quelconque des revendications précédentes, dans lequel le conditionnement a une hauteur de pile en sachet inférieure à environ 80 mm, selon le test de hauteur de pile en sachet ici.
